# EUROPÄISCHE PATENTSCHRIFT

(11) **EP 1 601 659 B1**
(45) Veröffentlichungstag und Bekanntmachung des Hinweises auf die Patenterteilung: **16.01.2008**
(21) Anmeldenummer: 04719974.0
(22) Anmeldetag: 12.03.2004
(51) Int. Cl.: C07D 239/56, C07D 239/54, C07D 239/36, C07D 239/52, C07D 405/14, C07D 409/14, C07D 403/14, C07D 401/12, A61K 31/505, A61P 25/28, A61P 25/24

(54) **PYRIMIDIN-2-ON-VERBINDUNGEN UND IHRE VERWENDUNG ALS DOPAMIN-D3-REZEPTORLIGANDEN**
PYRIMIDIN-2-ONE COMPOUNDS AND THEIR USE AS DOPAMINE D3 RECEPTOR LIGANDS
COMPOSES DE PYRIMIDIN-2-ONE ET LEUR UTILISATION EN TANT QUE LIGANDS DU RECEPTEUR DE LA DOPAMINE D3

(30) Priorität: 13.03.2003 DE 10311065
(43) Veröffentlichungstag der Anmeldung: 07.12.2005
(73) Patentinhaber: Abbott GmbH & Co. KG, 65205 Wiesbaden (DE)
(72) Erfinder: GENESTE, Hervé, 67141 Neuhofen (DE); KLING, Andreas, 68239 Mannheim (DE); BRAJE, Wilfried, 31737 Rinteln (DE); HAUPT, Andreas, 68723 Schwetzingen (DE); UNGER, Liliane, 67056 Ludwigshafen (DE)
(74) Vertreter: Pohl, Michael Friedrich
(86) Internationale Anmeldenummer: PCT/EP2004/002609
(87) Internationale Veröffentlichungsnummer: WO 2004/080981

(56) Entgegenhaltungen:
- EP-A- 0 748 800
- WO-A-96/02519
- WO-A-03/002543
- MOKROSZ J L ET AL: "A search for new trazodone-like antidepressants: synthesis and preliminary receptor binding studies" ARCHIV DER PHARMAZIE, Bd. 328, 1995, Seiten 623-625, XP002016667
- DATABASE CHEMCATS CHEMICAL ABSTRACTS SERVICE, COLUMBUS, OHIO, US; XP002283265 gefunden im STN Database accession no. 2003:1379694 & "Ambinter Screening Library" 1. Januar 2004 (2004-01-01), AMBINTER , PARIS, FR
- LOPEZ F J ET AL: "Synthesis, pharmacology and pharmacokinetics of 3-(4-aryl-piperazin-1-ylalkyl)-uracils as uroselective alpha1-antagonists" BIOORGANIC AND MEDICINAL CHEMISTRY LETTERS, Bd. 13, Nr. 11, 2. Juni 2003 (2003-06-02), Seiten 1873-1878, XP002283264

## Beschreibung

Die vorliegende Erfindung betrifft neue Pyrimidin-2-on-Verbindungen. Diese Verbindungen besitzen wertvolle therapeutische Eigenschaften und sind insbesondere zur Behandlung von Erkrankungen geeignet, die auf die Modulation des Dopamin-D₃-Rezeptors ansprechen.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin. Es liegen gesicherte Erkenntnisse über die Anwesenheit von Dopamin und dessen physiologische Funktion als Neurotransmitter vor. Störungen im dopaminergen Transmittersystem resultieren in Erkrankungen des zentralen Nervensystems, zu denen z. B. Schizophrenie, Depression oder Parkinson-Krankheit zählen. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren. In jüngerer Zeit wurde ein dritter Subtyp gefunden, nämlich der D₃-Rezeptor, der einige Effekte der Antipsychotika und Anti-Parkinsonmittel zu vermitteln scheint (J.C. Schwartz et al., The Dopamine D3 Receptor as a Target for Antipsychotics, in Novel Antipsychotic Drugs, H.Y. Meltzer, Ed. Raven Press, New York 1992, Seiten 135-144; M. Dooley et al., Drugs and Aging 1998, 12, 495-514, J.N. Joyce, Pharmacology and Therapeutics 2001, 90, S. 231-59 "The Dopamine D3 Receptor as a Therapeutic Target for Antipsychotic and Antiparkinsonian Drugs").

Mittlerweile teilt man die Dopamin-Rezeptoren in zwei Familien ein. Einerseits die D₂-Gruppe bestehend aus D₂-, D₃- und D₄-Rezeptoren, andererseits die D₁-Gruppe bestehend aus D₁- und D₅-Rezeptoren. Während D₁- und D₂-Rezeptoren weit verbreitet sind, scheinen D₃-Rezeptoren hingegen regioselektiv exprimiert zu werden. So findet man diese Rezeptoren vorzugsweise im limbischen System, den Projektionsbereichen des mesolimbischen Dopaminsystems, vor allem im Nucleus accumbens, aber auch in anderen Bereichen, wie der Amygdala. Wegen dieser vergleichsweise regioselektiven Expression gelten D₃-Rezeptoren als nebenwirkungsarmes Target, und es wird angenommen, dass ein selektiver D₃-Ligand wohl die Eigenschaften bekannter Antipsychotika, nicht aber ihre Dopamin-D₂-Rezeptor-vermittelten neurologischen Nebenwirkungen haben sollte (P. Sokoloff et al., Localization and Function of the D3 Dopamine Receptor, Arzneim. Forsch./Drug Res. 42(1), 224 (1992); P. Sokoloff et al. Molecular Cloning and Characterization of a Novel Dopamine Receptor (D3) as a Target for Neuroleptics, Nature, 347, 146 (1990)). Pyrimidinverbindungen mit Dopamin-D₃-Rezeptoraffinität sind aus der DE 10131543 und der WO 96/02519 bekannt. Diese Verbindungen weisen teilweise hohe Affinitäten zum D₃-Rezeptor auf. Sie werden daher zur Behandlung von Erkrankungen des zentralen Nervensystems vorgeschlagen.

Die Verwendung von 1-(3-Chlorphenyl)-piperazinen als Antidepressiva wird von Mokrosz et al. in "A search for new trazodone-like antidepressants: synthesis and preliminary receptor binding studies" Archiv der Pharmazie, 328 (1995), 623-625 beschrieben.

Die EP 748 800 A2 beschreibt Pyrimidindion-, Pyrimidintrion-, Triazindion- und Tetrahydrochinazolindion-Derivate, die als α-Adrenorezeptor-Antagonisten wirken.

Verschiedentlich wurde darüber berichtet, dass Neuroleptika zu einer Hemmung der mitochondrialen Atmung führen können. Es wurde gezeigt, dass eine derartige Hemmung Ursache für die neurotoxische Wirkung von Neuroleptika und für die bei längerer Gabe von Neuroleptika beobachteten, irreversiblen extrapyramidalen Nebenwirkungen ist (siehe C. Burkhardt et al, Annals of Neurology, Vol 33 (1993) 512 -517; I. Maurer et al, Molecular and Cellular Biochemistry 174 (1997) 225-229; S. Balijepalli et al, Neurochemistry International 38 (2001) 425-435). Es ist daher wünschenswert, über selektive Dopamin-D₃-Rezeptor-Liganden zu verfügen, die zudem keine oder nur eine geringe Hemmung der mitochondrialen Atmung bewirken.

Weiterhin sollten die Verbindungen eine geringe Plasmaproteinbindung aufweisen. Eine geringe Plasmaproteinbindung hat den Vorteil, dass die Verbindungen eine bessere Verträglichkeit aufweisen, da der Plasmaspiegel gleichmäßiger ist und eine unkontrollierte Freisetzung des Wirkstoffs aus der Plasmaproteinbindung, beispielsweise als Folge einer erhöhten körperlichen Aktivität oder aufgrund von Wechselwirkungen mit anderen Arzneimitteln, vermieden wird.

Der Erfindung liegt daher die Aufgabe zu Grunde, Verbindungen zur Verfügung zu stellen, die als selektive Dopamin-D₃-Rezeptor-Liganden wirken. Diese Verbindungen sollten ausserdem zu keiner oder nur bei hohen Dosierungen zu einer Hemmung der mitochondrialen Atmung führen. Die Verbindungen sollten zudem eine geringe Plasmaproteinbindung aufweisen.

Diese Aufgabe wird gelöst durch Pyrimidin-2-onverbindungen der allgemeinen Formel 1 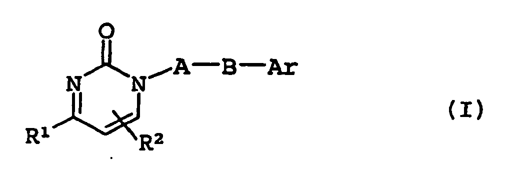 worin
- A: für (CH₂)ₙ, worin n für 4, 5 oder 6 steht, oder für *trans*-CH₂-CH=CH-CH₂-, *trans*-CH₂-C(CH₃)=CH-CH₂-, -CH₂-CH(CH₃)-CH₂-CH₂- oder -CH₂-CH₂-CH₂-CH(CH₃)- steht,
- B: für einen Rest der Formel: 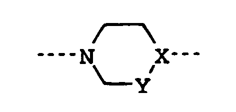 steht, worin X CH oder N bedeutet und Y für CH₂ oder CH₂CH₂ steht oder X-Y auch gemeinsam für C=CH, C=CH-CH₂ oder CH-CH=CH stehen können.
- R¹: ausgewählt ist unter OR^{3a}, NR⁴R⁵, SR⁶, C₃-C₆-Cycloalkyl, C₁-C₄-Alkyl, das gegebenenfalls durch OH, C₁-C₄-Alkoxy, Halogen oder Phenyl substituiert ist,
5- oder 6-gliedrigem aromatischem Heterocyclyl mit 1, 2 oder 3 Heteroatomen, ausgewählt unter O, S und N, das durch ein oder zwei Reste substituiert sein kann, die unabhängig voneinander ausgewählt sind unter C₁-C₄-Alkyl, C₁-C₄-Alkoxy, NR⁴R⁵, CN, OH, C₁-C₂-Fluoralkyl oder Halogen, und
Phenyl, das durch ein oder zwei Reste substituiert sein kann, die unabhängig voneinander ausgewählt sind unter C₁-C₄-Alkyl, C₁-C₄-Alkoxy, NR⁴R⁵, OH, CN, C₁-C₂-Fluoralkyl oder Halogen,
- R²: ausgewählt ist unter H, C₁-C₄-Alkyl, C₁-C₂-Fluoralkyl, Halogen und Cyano,
- Ar: für einen aromatischen Rest steht, der ausgewählt ist unter Phenyl, Pyridyl, Pyrimidinyl und Triazinyl, wobei der aromatische Rest 1, 2 oder 3 Substituenten aufweisen kann, die unabhängig voneinander ausgewählt sind unter C₁-C₆-Alkyl, das gegebenenfalls durch OH, C₁-C₄-Alkoxy, Halogen oder Phenyl substituiert ist, C₂-C₆-Alkenyl, C₂-C₆-Alkinyl, C₃-C₆-Cycloalkyl, C₅-C₁₀-Bicycloalkyl, C₆-C₁₀-Tricycloalkyl, wobei die drei letztgenannten Gruppen gegebenenfalls durch Halogen oder C₁-C₄-Alkyl substituiert sein können, Halogen, CN, OR^{3c}, NR⁴R⁵, NO₂, SR⁶, SO₂R⁶, SO₂NR⁴R⁵, COOR⁷, COR⁸, 5- oder 6-gliedriges Heterocyclyl mit 1, 2 oder 3 Heteroatomen, ausgewählt unter O, S und N, und Phenyl, wobei Phenyl und Heterocyclyl gegebenenfalls ein oder zwei Substituenten tragen, die unabhängig voneinander ausgewählt sind unter C₁-C₄-Alkyl, C₁-C₄-Alkoxy, NR⁴R⁵, CN, C₁-C₂-Fluoralkyl und Halogen, und wobei 2 an benachbarte C-Atome des aromatischen Rests gebundene Substituenten gemeinsam für C₃-oder C₄-Alkylen stehen können oder gemeinsam mit den C-Atomen, an die sie gebunden sind, für einen annellierten, ungesättigten 5 oder 6-gliedrigen Carbocyclus oder für einen 5- oder 6-gliedrigen Heterocyclus mit 1 oder 2 Stickstoffatomen als Ringglieder stehen können,
- R³, R^{3a}, R^{3b}, R^{3c}, R^{3d}, R⁴, R⁵, R⁶, R⁷ und R⁸: unabhängig voneinander für H, C₁-C₆-Alkyl, das gegebenenfalls durch OH, C₁-C₄-Alkoxy oder Phenyl substituiert ist, C₁-C₆-Halogenalkyl oder Phenyl, stehen, wobei R⁵ auch eine Gruppe COR⁹ bedeuten kann, wobei R⁹ für Wasserstoff, C₁-C₄-Alkyl oder Phenyl, das gegebenenfalls durch ein oder zwei Reste substituiert ist, die unabhängig voneinander ausgewählt sind unter C₁-C₄-Alkyl, C₁-C₄-Alkoxy, NR⁴R⁵, CN, C₁-C₂-Fluoralkyl oder Halogen, stehen, wobei
- R⁴ mit R⁵: auch gemeinsam mit dem Stickstoffatom, an das sie gebunden sind, einen 4-, 5- oder 6-gliedrigen gesättigten oder ungesättigten Heterocyclus bilden können, der gegebenenfalls ein weiteres Heteroatom, ausgewählt unter O, S und NR¹⁰ als Ringglied aufweisen kann, wobei R¹⁰ für Wasserstoff oder C₁-C₄-Alkyl steht, und wobei der Heterocyclus gegebenenfalls 1, 2, 3 oder 4 C₁-C₄-Alkylgruppen tragen kann,
sowie die Derivate und Tautomere der Formeln Ia oder Ib 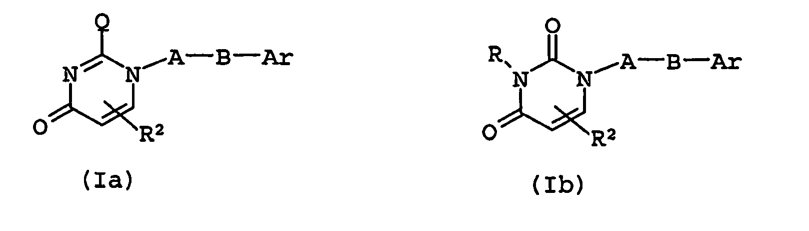 worin R für Wasserstoff oder C₁-C₄-Alkyl steht und Q Halogen oder eine Gruppe OR^{3d} bedeutet, und A, B, Ar und R² die zuvor genannten Bedeutungen aufweisen, und durch die physiologisch verträglichen Salze dieser Verbindungen.

Gegenstand der vorliegenden Erfindung sind daher Pyrimidinverbindungen der allgemeinen Formel I sowie die Derivate und Tautomere der Formeln Ia und Ib sowie die physiologisch akzeptablen Salze davon.

Gegenstand der vorliegenden Verbindung ist außerdem ein pharmazeutisches Mittel, enthaltend wenigstens eine Verbindung der Formeln I, Ia und/oder Ib und/oder deren physiologisch akzeptablen Säureadditionssalze und gegebenenfalls einen oder mehrere physiologisch akzeptable Träger.

Gegenstand der vorliegenden Erfindung ist auch die Verwendung einer Pyrimidinon-Verbindung der Formel I, von deren Derivaten und Tantomeren der Formeln Ia und Ib und von deren Salzen zur Herstellung eines pharmazeutischen Mittels zur Behandlung von Erkrankungen, die auf die Modulation durch Dopamin-D₃-Rezeptorliganden ansprechen.

Zu den Erkrankungen, die auf die Modulation durch Dopamin-D₃-Rezeptorliganden ansprechen, zählen beispielsweise Störungen und Erkrankungen des zentralen Nervensystems, insbesondere Schizophrenie und Depression, Parkinson und Epilepsie, weiterhin Suchterkrankungen sowie Nierenfunktionsstörungen.

Erfindungsgemäß verwendet man zur Behandlung der vorstehend genannten Indikationen wenigstens eine Verbindung der allgemeinen Formeln I, Ia und/oder Ib mit den eingangs genannten Bedeutungen. Sofern die Verbindungen der Formel I ein oder mehrere Asymmetriezentren aufweisen, können auch Enantiomerengemische, insbesondere Racemate, Diastereomerengemische, Tautomerengemische, vorzugsweise jedoch die jeweiligen im Wesentlichen reinen Enantiomere, Diastereomere und Tautomere eingesetzt werden.

Ebenfalls brauchbar sind physiologisch verträgliche Salze der Verbindungen der Formeln I, Ia und Ib, vor allem Säureadditionssalze mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Halogen steht hier und im folgenden für Fluor, Chlor, Brom oder lod.

Cₙ-Cₘ-Alkyl (auch in Resten wie Alkoxy, Alkylthio, Alkylamino etc.) bedeutet eine geradkettige oder verzweigte Alkylgruppe mit n bis m Kohlenstoffatomen, z.B. 1 bis 6 Kohlenstoffatomen und insbesondere 1 bis 4 Kohlenstoffatomen. Beispiele für eine Alkylgruppe sind Methyl, Ethyl, n-Propyl, iso-Propyl, n-Butyl, 2-Butyl, iso-Butyl, tert-Butyl, n-Pentyl, 2-Pentyl, Neopentyl, n-Hexyl und dergleichen.

Die Alkylgruppe kann einen oder mehrere Substituenten aufweisen, die unabhängig voneinander ausgewählt sind unter OH, C₁-C₄-Alkoxy, Halogen oder Phenyl. Im Falle eines Halogensubstituenten kann die Alkylgruppe insbesondere 1, 2, 3 oder 4 Halogenatome umfassen, die sich an einem oder mehreren C-Atomen befinden können, vorzugsweise in α- oder ω-Position. Derartige Gruppen werden im Folgenden auch als Halogenalkyl bezeichnet. Bevorzugtes Halogenalkyl ist C₁-C₂-Fluoralkyl oder C₁-C₂-Fluorchloralkyl insbesondere CF₃, CHF₂, CF₂Cl, CH₂F, CH₂CF₃.

Im Falle von Hydroxy substituiertem Alkyl weist die Alkylgruppe insbesondere eine Hydroxy-Gruppe auf wie z.B. Hydroxymethyl, 2-Hydroxyeth-1-yl, 2-Hydroxyprop-1-yl, 3-Hydroxy-prop-1-yl, 1-Hydroxyprop-2-yl, 2-Hydroxybut-1-yl, 3-Hydroxybut-1-yl, 4-Hydroxybut-1-yl, 1-Hydroxybut-2-yl, 1-Hydroxybut-3-yl, 2-Hydroxybut-3-yl, 1-Hydroxy-2-methylprop-3-yl, 2-Hydroxy-2-methylprop-3-yl oder 2-Hydroxymethylprop-2-yl, insbesondere 2-Hydroxyethyl.

Im Falle von Alkoxy-substituiertem Alkyl weist die Alkylgruppe insbesondere einen Alkoxysubstituenten auf. Diese Reste werden, abhängig von der Anzahl der Kohlenstoffatome auch als Cₙ-Cₘ-Alkoxy-Cₙ-Cₘ-alkyl bezeichnet und stehen z.B. für Methoxymethyl, Ethoxymethyl, 2-Methoxyethyl, 1-Methoxyethyl, 2-Ethoxyethyl, 1-Ethoxyethyl, n-Propoxymethyl, Isopropoxymethyl, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)-propyl, 3-(Ethoxy)propyl, 3-(n-Propoxy)propyl, 3-(1-Methylethoxy)propyl, 3-(n-Butoxy)/propyl, 3-(1-Methylpropoxy)propyl, 3-(2-Methylpropoxy)propyl, 3-(1,1-Dimethylethoxy)-propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, vorzugsweise Methoxymethyl, Ethoxymethyl, 2-Methoxyethyl, 2-Ethoxyethyl, 2-(Methoxy)propyl, 2-(Ethoxy)propyl oder 3-(Methoxy)propyl, 3-(Ethoxy)propyl.

Cycloalkyl steht insbesondere für C₃-C₆-Cycloalkyl, wie Cyclopropyl, Cyclobutyl, Cyclopentyl und Cyclohexyl.

Der Begriff "Alkylen" umfasst grundsätzlich geradkettige oder verzweigte Reste mit vorzugsweise mit 3 bis 10 und besonders bevorzugt 3 bis 8 Kohlenstoffatomen wie Prop-1,2-ylen, Prop-1,3-ylen, But-1,2-ylen, But-1,3-ylen, But-1,4-ylen, 2-Methylprop-1,3-ylen, Pent-1,2-ylen, Pent-1,3-ylen, Pent-1,4-ylen, Pent-1,5-ylen, Pent-2,3-ylen, Pent-2,4-ylen, 1-Methylbut-1,4-ylen, 2-Methylbut-1,4-ylen, Hex-1,3-ylen, Hex-2,4-ylen, Hex-1,4-ylen, Hex-1,5-ylen, Hex-1,6-ylen und dergleichen. C₀-Alkylen steht für eine Einfachbindung, C₁-Alkylen für Methylen und C₂-Alkylen für 1,1-Ethylen oder 1,2-Ethylen.

4, 5- oder 6-gliedriges Heterocyclyl umfasst sowohl aromatisches Heterocyclyl (Hetaryl bzw. Heteroaryl) als auch vollständig gesättigte oder teilweise ungesättigte heterocyclische Reste. Heterocyclyl weist 1, 2 oder 3 Heteroatome, ausgewählt unter O, S und N auf, z. B. 1, 2 oder 3 Stickstoffatome, 1 oder 2 Sauerstoffatome, oder 1 Sauerstoffatom und 1 oder 2 Stickstoffatome oder 1 Schwefelatom und 1 oder 2 Stickstoffatome. Heterocyclyl kann unsubstituiert sein oder 1 oder 2 Substituenten ausgewählt unter C₁-C₄-Alkyl, C₁-C₄-Alkoxy, OH, CN, NR⁴R⁵, C₁-C₂-Fluoralkyl und Halogen aufweisen. Außerdem kann Heterocyclyl einen anellierten (kondensierten) 5- oder 6-gliedrigen Carbocyclus, z. B. ein Benzol-, Cyclopentan- oder Cyclohexen-Ring oder einen anellierten Heterocyclus aufweisen, z. B. einen anellierten Pyrroly-Furan-, Thiophen-, Thiazol-, Pyridin-, Pyrimidin- oder Pyridazin-Ring.

Beispiele für gesättigtes Heterocyclyl sind Azetidinyl, Pyrrolidinyl, Piperidinyl, Piperazinyl, Morpholinyl, Oxolanyl, 1,3-Dioxolanyl, 1,3- und 1,4-Dioxanyl, 1,3-Oxothiolanyl, O-xazolidinyl und dergleichen.

Beispiele für "5- oder 6-gliedrige aromatische heterocyclische Reste", die 1, 2 oder 3 Heteroatome aufweisen, die ausgewählt sind unter O, S und N, sind vor allem Pyridinyl, Pyrimidinyl, Pyrazinyl, Triazinyl, Imidazolyl, Pyrrolyl, Pyrazolyl, Thienyl, Furanyl, Oxazolyl, Thiazolyl, Isoxazolyl, Tetrazolyl, Thiadiazolyl und Triazolyl. Diese können an den Stickstoffatomen sowie an den Kohlenstoffatomen 1 oder 2 der vorgenannten Substituenten aufweisen. Sofern einer der Substituenten Hydroxy ist, können die Reste auch in einer tautomeren Form mit Carbonylgruppe vorliegen. Beispiele für 5- oder 6-gliedrige heterocyclische Reste, die einen anellierten Carbocyclus aufweisen, bzw. mit diesem kondensiert sind, umfassen Benzofuranyl, Benzthienyl, Indolyl, Benzothiazolyl, Benzoxazolyl, Benzimidazolyl. Benzopyrazolyl, Chinolinyl, Isochinolinyl, Chinoxalinyl, Chinazolinyl, Cinnolinyl, sowie entsprechende teilhydrierte Gruppen.

Beispiele für einen annellierten 5 oder 6-gliedrigen Carbocyclus sind Cyclopenten, Cyclopentadien, Cyclohexen, Cyclohexadien und Benzol. Beispiele für einen annelierten 5-oder 6-gliedrigen Heterocyclus mit 1 oder 2 Stickstoffatomen als Ringglieder sind Pyridin, 1,2.3,4-und 1,2,5,6-Tetrahydropyridin, 1,2- und 1,4-Dihydrophyridin, Pyrimidin, Pyrazin und Pyridazin.

Im Hinblick auf die Verwendung der erfindungsgemäßen Verbindungen als Dopamin-D₃-Rezeptor-Liganden weisen die Variablen A, B, R¹, R² und Ar unabhängig voneinander vorzugsweise die nachstehend angegebenen Bedeutungen auf:
- A: steht für -(CH₂)ₙ, worin n für 4, 5 oder 6 und insbesondere für 4 steht oder A steht für *trans*-CH₂-CH=CH-CH₂-, *trans*-CH₂-C(CH₃)=CH-CH₂-, -CH₂-CH(CH₃)-CH₂-CH₂- oder -CH₂-CH₂-CH₂-CH(CH₃)-. Besonders bevorzugt steht A für -(CH₂)₄-;
- B: steht für einen bivalenten Rest der allgemeinen Formeln: 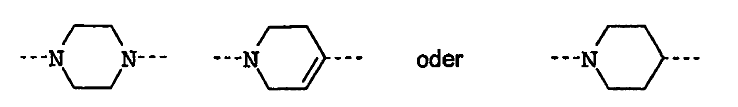 Hierin ist das Stickstoffatom mit der Gruppe A verknüpft. Insbesondere steht B für Piperazin-1,4-diyl.
- R¹: steht für eine Gruppe OR^{3a}, NR⁴R⁵, SR⁶, C₃-C₆-Cycloalkyl, C₁-C₄-Alkyl, das gegebenenfalls durch OH, C₁-C₄-Alkoxy, Halogen oder Phenyl substituiert ist, für 5- oder 6-gliedriges aromatisches Heterocyclyl mit 1, 2 oder 3 Heteroatomen, ausgewählt unter O, S und N, das durch ein oder zwei Reste substituiert sein kann, die unabhängig voneinander ausgewählt sind unter C₁-C₄-Alkyl, C₁-C₄-Alkoxy, NR⁴R⁵, CN, OH, C₁-C₂-Fluoralkyl oder Halogen, oder für Phenyl, das durch ein oder zwei Reste substituiert sein kann, die unabhängig voneinander ausgewählt sind unter C₁-C₄-Alkyl, C₁-C₄-Alkoxy, NR⁴R⁵, OH, CN, C₁-C₂-Fluoralkyl oder Halogen.

Insbesondere steht R¹ für C₁-C₄-Alkyl, Halogen, gegebenenfalls substituiertes Phenyl, C₁-C₂-Fluoralkyl, eine Gruppe OR^{3a}, eine Gruppe SR⁶ oder einen Rest NR⁴R⁵. Hierbei steht R^{3a} insbesondere für Wasserstoff, C₁-C₄-Alky, Phenyl oder Benzyl und speziell für Wasserstoff. R⁴ steht vorzugsweise für Wasserstoff oder Alkyl. R⁵ steht vorzugsweise für Wasserstoff, C₁-C₄-Alkyl, Phenyl oder Benzyl oder bildet zusammen mit dem Stickstoffatom und dem Rest R⁴ einen 4-, 5- oder 6-gliedrigen gesättigten Heterocyclus wie Azetidinyl, Pyrrolidinyl, Piperidinyl, Morpholinyl oder Piperazinyl. R⁶ steht hierbei vorzugsweise für Wasserstoff, C₁-C₄-Alkyl, Phenyl oder Benzyl und insbesondere für Wasserstoff. Substituiertes Phenyl bedeutet hier, dass der Phenylrest durch ein oder zwei Reste substituiert sein kann, z.B. durch C₁-C₄-Alkyl, C₁-C₄-Alkoxy, NR⁴R⁵, OH, CN, C₁-C₂-Fluoralkyl und/oder Halogen.

In einer besonders bevorzugten Ausführungsform der Erfindung steht R¹ für C₁-C₄-Alkyl, insbesondere Methyl, Trifluormethyl oder für einen Rest OR^{3a}. Hierin hat R^{3a} die zuvor genannten Bedeutungen und steht insbesondere für H, C₁-C₄-Alkyl, Phenyl oder Benzyl und speziell für H. Hierbei kann der Phenylring in Phenyl sowie in Benzyl durch ein oder zwei Reste substituiert sein, die unabhängig voneinander ausgewählt sind unter C₁-C₄-Alkyl, C₁-C₄-Alkoxy, NR⁴R⁵, OH, CN, C₁-C₂-Fluoralkyl oder Halogen.
- R²: ist vorzugsweise in der 5-Position des Pyrimidin-2-on-rings angeordnet. R² ist insbesondere ausgewählt unter H, C₁-C₄-Alkyl, C₁-C₂-Fluoralkyl, Halogen und Cyano, speziell unter H, Methyl, CN, Fluor und Trifluormethyl. In einer besonders bevorzugten Ausführungsform steht R² für C₁-C₄-Alkyl, insbesondere für Methyl. In einer anderen besonders bevorzugten Ausführungsform steht R² für Wasserstoff.

Eine ganz besonderes bevorzugte Ausführungsform der Erfindung betrifft Verbindungen der Formel I, worin R¹ für OR^{3a} und insbesondere für OH steht und R² insbesondere ausgewählt ist unter H, Fluor, Chlor, CN und C₁-C₄-Alkyl und speziell Wasserstoff oder Methyl.

Eine weitere ganz besonders bevorzugte Ausführungsform der Erfindung betrifft Verbindungen der Formel I, worin R¹ für Methyl steht und R² insbesondere ausgewählt ist unter H, Fluor, Chlor, CN und C₁-C₄-Alkyl und speziell für Wasserstoff steht.

Ar steht vorzugsweise für einen Rest der allgemeinen Formel: 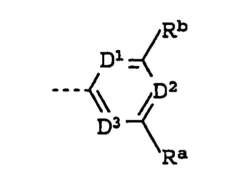 worin wenigstens einer der Variablen D¹ bis D³ für N und die übrigen Variablen D¹ bis D³ für CH stehen und R^{a} und R^{b} die zuvor als Substituenten an Ar angegebenen Bedeutungen aufweisen oder für Wasserstoff stehen. Hierunter sind Verbindungen bevorzugt, worin D¹ und D² jeweils Stickstoff bedeuten und D³ für CH steht. Vorzugsweise stehen R^{a} und R^{b} unabhängig voneinander für die folgenden Gruppen: Wasserstoff, OR^{3b}, NR⁴R⁵, CN, C₁-C₆-Alkyl, das gegebenenfalls durch OH, C₁-C₄-Alkoxy, Halogen oder Phenyl substituiert ist, C₂-C₆-Alkenyl, C₂-C₆-Alkinyl, C₅-C₆-Cycloalkyl, C₅-C₁₀-Bicycloalkyl, C₆-C₁₀-Tricycloalkyl, wobei die drei letztgenannten Gruppen gegebenenfalls durch Halogen oder C₁-C₄-Alkyl substituiert sein können, Halogen, CN, OR^{3c}, 5-oder 6-gliedriges Heterocyclyl mit 1, 2 oder 3 Heteroatomen, ausgewählt unter O, S und N, und Phenyl, wobei Phenyl und Heterocyclyl gegebenenfalls ein oder zwei Substituenten tragen, die unabhängig voneinander ausgewählt sind unter C₁-C₄-Alkyl, C₁-C₄-Alkoxy, NR⁴R⁵, CN, C₁-C₂-Fluoralkyl und Halogen. Vorzugsweise ist wenigstens einer der Reste R^{a}, R^{b} und insbesondere beide Reste R^{a}, R^{b} von Wasserstoff verschieden. Insbesondere steht R^{b} für C₁-C₆-Alkyl, besonders bevorzugt für verzweigtes C₃-C₆-Alkyl und speziell für tert.-Butyl. R^{a} ist vorzugsweise ausgewählt unter C₁-C₄-Alkyl, C₃-C₆-Cycloalkyl und C₁-C₂-Fluoralkyl und ganz besonders bevorzugt unter CF₃, Cyclopentyl und n-Propyl. Besonders bevorzugt weisen R^{a} und R^{b} gemeinsam die als bevorzugt angegebenen Bedeutungen auf.

Sofern Ar einen annellierten Ring aufweist steht Ar vorzugsweise für einen Rest der Formel: 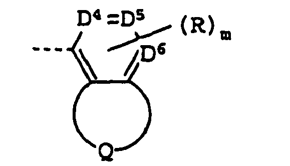 worin D⁴, D⁵ und D⁶ unabhängig voneinander für CH oder N stehen, Q für lineares C₃-C₄-Alkylen, C₃-C₄-Alkenylen oder für eine Gruppe CH=CH-N=CH oder N=CH-CH=CH steht, m 0, 1 oder 2 bedeutet und R für einen von Wasserstoff verschiedenen Substituenten wie C₁-C₄-Alkyl, C₁-C₄-Alkoxy, CN, OH, Halogen oder NR⁴R⁶ steht.

Unter den erfindungsgemäßen Pyrimidin-2-onverbindungen sind die Pyrimidinonverbindungen der allgemeinen Formel 1.1 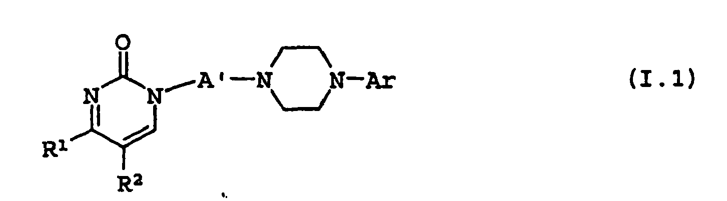 bevorzugt, worin Ar, R¹ und R² die zuvor genannten Bedeutungen und insbesondere die als bevorzugt angegebenen Bedeutungen aufweisen und A' eine Gruppe -(CH₂)ₙ bedeutet, worin n für 4, 5 oder 6 und Insbesondere für 4 steht, oder A' eine der folgenden Gruppen: *trans*-CH₂-CH=CH-CH₂-, *trans*-CH₂-C(CH₃)=CH-CH₂-, -CH₂-CH(CH₃)-CH₂-CH₂- oder -CH₂-CH₂-CH₂-CH(CH₃)- bedeutet.

Ebenfalls bevorzugt sind die Derivate und Tautomere der Formeln Ia.1 oder Ib.1 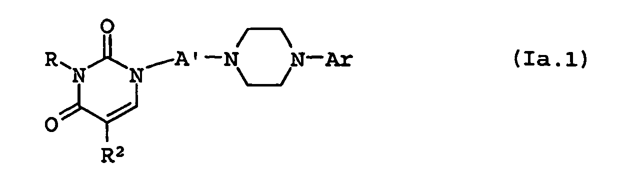 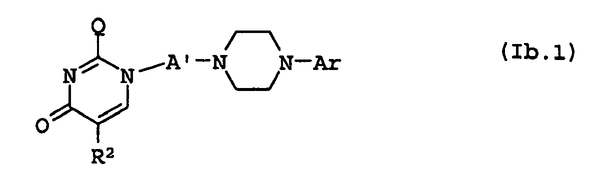 worin R, A', Q, Ar und R² die zuvor genannten genannten Bedeutungen und insbesondere die als bevorzugt angegebenen Bedeutungen aufweisen.

Im übrigen weisen die Gruppen R³, R^{3b}, R^{3c}, R^{3d}, R⁴, R⁵, R⁶, R⁷ und R⁸ vorzugsweise die nachfolgend angegebenen Bedeutungen auf:
R³ steht vorzugsweise für H, C₁-C₄-Alkyl, phenylsubstituiertes C₁-C₄-Alkyl oder COR¹¹. Hierin weist R¹¹ die für R⁸ angegebenen Bedeutungen auf und steht insbesondere für C₁-C₄-Alkyl. In Gruppen NR³ steht R³ vorzugsweise für H, C₁-C₄-Alkyl, phenylsubstituiertes C₁-C₄-Alkyl oder COR¹¹. Insbesondere bevorzugt steht NR³ für NH, NCH₃, NCOCH₃ oder NCH₂-Phenyl. In den Gruppen C(O)NR³ und NR³C(O) steht R³ vorzugsweise für H, C₁-C₄-Alkyl, phenylsubstituiertes C₁-C₄-Alkyl oder COR¹¹. Insbesondere bevorzugt steht C(O)NR³ für CONH, CONCH₃ oder CONCH₂-Phenyl. Insbesondere bevorzugt steht NR³C(O) für NHCO, NCH₃CO oder N(CH₂-Phenyl)CO.
R^{3b}, R^{3c} und R^{3d} stehen unabhängig voneinander vorzugsweise für H, C₁-C₄-Alkyl, CF₃, CHF₂ oder Phenyl. Insbesondere bevorzugt stehen OR^{3b}, OR^{3c} und OR^{3d} für Methoxy, Trifluormethoxy oder Phenoxy.
R⁴ steht vorzugsweise für Wasserstoff oder Alkyl. R⁵ steht vorzugsweise für Wasserstoff, C₁-C₄-Alkyl, Phenyl, Benzyl oder eine Gruppe COR¹¹. In Substituenten CONR⁴R⁵ steht R⁴ vorzugsweise für H oder C₁-C₄-Alkyl und R⁵ vorzugsweise für H, C₁-C₄-Alkyl oder COR¹¹. Insbesondere bevorzugt steht CONR⁴R⁵ für CONH₂, CONHCH₃, CON(CH₃)₂ oder C(O)NHC(O)CH₃. In Substituenten NR⁴R⁵ steht R⁴ vorzugsweise für H, C₁-C₄-Alkyl oder phenylsubstituiertes C₁-C₄-Alkyl und R⁵ für H, C₁-C₄-Alkyl oder COR¹¹. Insbesondere bevorzugt steht NR⁴R⁵ für NH₂, NHCH₃, N(CH₃)₂, NH-Benzyl oder NHCOCH₃. In Substituenten SO₂NR⁴R⁵ steht R⁴ vorzugsweise für H oder C₁-C₄-Alkyl und R⁵ vorzugsweise für H, C₁-C₄-Alkyl oder COR¹¹. Insbesondere bevorzugt steht SO₂NR⁴R⁵ für Sulfamoyl. In den vorgenannten Gruppen können R⁴ und R⁵ auch gemeinsam mit dem Stickstoffatom, an das sie gebunden sind, einen gesättigten oder ungesättigten 4-, 5- oder 6-gliedrigen, vorzugsweise gesättigten Stickstoffheterocyclus bilden, der ein weiteres Heteroatom wie N, S oder O aufweisen kann und der durch 1, 2, 3 oder 4 Alkylgruppen substituiert sein kann. Beispiele für derartige Heterocyclen sind Piperidinyl, Morpholinyl, Pyrrolidinyl, 4-Methylpiperazinyl und 4-Methylpiperidinyl.
R⁶ steht vorzugsweise für H, C₁-C₄-Alkyl, Phenyl oder Benzyl. In Substituenten SR⁶ steht R⁶ vorzugsweise für H, C₁-C₄-Alkyl, Phenyl oder Benzyl. In Substituenten SOR⁶ steht R⁶ vorzugsweise Phenyl oder C₁-C₄-Alkyl. In Substituenten SO₂R⁶ steht R⁶ vorzugsweise für H oder C₁-C₄-Alkyl. Insbesondere bevorzugt steht SO₂R⁶ für Methylsulfonyl.

In Substituenten COOR⁷ steht R⁷ für H oder C₁-C₄-Alkyl. Insbesondere bevorzugt steht COOR⁷ für C₁-C₄-Alkoxycarbonyl, wie Methoxycarbonyl, Ethoxycarbonyl, n-Propoxycarbonyl, i-Propoxycarbonyl, n-Butoxycarbonyl oder t-Butoxycarbonyl. In den Substituenten COR⁸ und OC(O)R⁸ steht R⁸ vorzugsweise für H, C₁-C₄-Alkyl oder Phenyl. Insbesondere bevorzugt steht COR⁸ für Formyl, Acetyl oder Benzoyl.

Unter den Verbindungen der allgemeinen Formel I.1 sind insbesondere die Verbindungen der allgemeinen Formel I.1a bevorzugt, 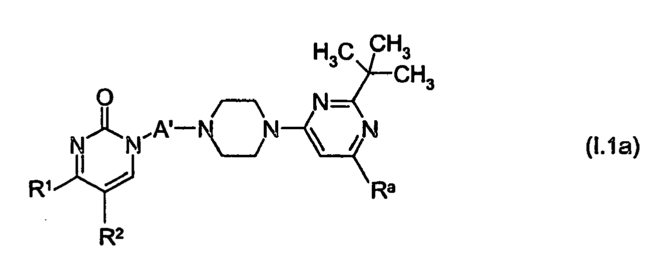 worin A', R¹, R² und R^{a} die zuvor angegebenen Bedeutungen, insbesondere die als bevorzugt angegebenen Bedeutungen aufweisen Beispiele für derartige Verbindungen sind die in der nachfolgenden Tabelle 1 aufgeführten Verbindungen 1.1 a.1 bis I.1a.871, wobei die Variablen A', R¹, R² und R^{a} jeweils gemeinsam die in einer Zeile der Tabelle 1 angegebene Bedeutung aufweisen.

**Tabelle 1**

| | R¹ | R² | A' | R^{a} |
|---|---|---|---|---|
| 1. | OH | H | -(CH₂)₄- | CF₃ |
| 2. | OH | CH₃ | -(CH₂)₄- | CF₃ |
| 3. | CH₃ | H | -(CH₂)₄- | CF₃ |
| 4. | C(CH₃)₃ | H | -(CH₂)₄- | CF₃ |
| 5. | C₆H₅ | H | -(CH₂)₄- | CF₃ |
| 6. | C₆H₅ | CH₃ | -(CH₂)₄- | CF₃ |
| 7. | CF₃ | H | -(CH₂)₄- | CF₃ |
| 8. | OH | F | -(CH₂)₄- | CF₃ |
| 9. | OH | CN | -(CH₂)₄- | CF₃ |
| 10. | N(CH₃)₂ | H | -(CH₂)₄- | CF₃ |
| 11. | N(CH₃)₂ | CH₃ | -(CH₂)₄- | CF₃ |
| 12. | OH | H | *trans*-CH₂-CH=CH-CH₂- | CF₃ |
| 13. | OH | CH₃ | *trans*-CH₂-CH=CH-CH₂- | CF₃ |
| 14. | CH₃ | H | *trans*-CH₂-CH=CH-CH₂- | CF₃ |
| 15. | C(CH₃)₃ | H | *trans*-CH₂-CH=CH-CH₂- | CF₃ |
| 16. | C₆H₅ | H | *trans* -CH₂-CH=CH-CH₂- | CF₃ |
| 17. | C₆H₅ | CH₃ | *trans*-CH₂-CH=CH-CH₂- | CF₃ |
| 18. | CF₃ | H | *trans* -CH₂-CH=CH-CH₂- | CF₃ |
| 19. | OH | F | *trans* -CH₂-CH=CH-CH₂- | CF₃ |
| 20. | OH | CN | *trans* -CH₂-CH=CH-CH₂- | CF₃ |
| 21. | N(CH₃)₂ | H | *trans*-CH₂-CH=CH-CH₂- | CF₃ |
| 22. | N(CH₃)₂ | CH₃ | *trans*-CH₂-CH=CH-CH₂- | CF₃ |
| 23. | OH | H | *trans*-CH₂-C(CH₃)=CH-CH₂- | CF₃ |
| 24. | OH | CH₃ | *trans*-CH₂-C(CH₃)=CH-CH₂- | CF₃ |
| 25. | CH₃ | H | *trans* -CH₂-C(CH₃)=CH-CH₂- | CF₃ |
| 26. | C(CH₃)₃ | H | *tran*s-CH₂-C(CH₃)=CH-CH₂- | CF₃ |
| 27. | C₆H₅ | H | *trans*-CH₂-C(CH₃)=CH-CH₂- | CF₃ |
| 28. | C₆H₅ | CH₃ | *trans*-CH₂C(CH₃)=CH-CH₂- | CF₃ |
| 29. | CF₃ | H | *trans*-CH₂-C(CH₃)=CH-CH₂- | CF₃ |
| 30. | OH | F | *trans*-CH₂-C(CH₃)=CH-CH₂- | CF₃ |
| 31. | OH | CN | *trans*-CH₂-C(CH₃)=CH-CH₂- | CF₃ |
| 32. | N(CH₃)₂ | H | *trans*-CH₂-C(CH₃)=CH-CH₂- | CF₃ |
| 33. | N(CH₃)₂ | CH₃ | *trans*-CH₂-C(CH₃)=CH-CH₂- | CF₃ |
| 34. | OH | H | -CH₂-CH(CH₃)-CH₂-CH₂- | CF₃ |
| 35. | OH | CH₃ | -CH₂-CH(CH₃)-CH₂-CH₂- | CF₃ |
| 36. | CH₃ | H | -CH₂-CH(CH₃)-CH₂-CH₂- | CF₃ |
| 37. | C(CH₃)₃ | H | -CH₂-CH(CH₃)-CH₂-CH₂- | CF₃ |
| 38. | C₆H₅ | H | -CH₂-CH(CH₃)-CH₂-CH₂- | CF₃ |
| 39. | C₆H₅ | CH₃ | -CH₂-CH(CH₃)-CH₂-CH₂- | CF₃ |
| 40. | CF₃ | H | -CH₂-CH(CH₃)-CH₂-CH₂- | CF₃ |
| 41. | OH | F | -CH₂-CH(CH₃)-CH₂-CH₂- | CF₃ |
| 42. | OH | CN | -CH₂-CH(CH₃)-CH₂-CH₂- | CF₃ |
| 43. | N(CH₃)₂ | H | -CH₂-CH(CH₃)-CH₂-CH₂- | CF₃ |
| 44. | N(CH₃)₂ | CH₃ | -CH₂-CH(CH₃)-CH₂-CH₂- | CF₃ |
| 45. | OH | H | -CH₂-CH₂-CH₂-CH(CH₃)- | CF₃ |
| 46. | OH | CH₃ | -CH₂-CH₂-CH₂-CH(CH₃)- | CF₃ |
| 47. | CH₃ | H | -CH₂-CH₂-CH₂-CH(CH₃)- | CF₃ |
| 48. | C(CH₃)₃ | H | -CH₂-CH₂CH₂-CH(CH₃)- | CF₃ |
| 49. | C₆H₅ | H | -CH₂-CH₂-CH₂-CH(CH₃)- | CF₃ |
| 50. | C₆H₅ | CH₃ | -CH₂-CH₂-CH₂-CH(CH₃)- | CF₃ |
| 51. | CF₃ | H | -CH₂-CH₂CH₂-CH(CH₃)- | CF₃ |
| 52. | OH | F | -CH₂-CH₂-CH₂-CH(CH₃)- | CF₃ |
| 53. | OH | CN | -CH₂-CH₂-CH₂-CH(CH₃)- | CF₃ |
| 54. | N(CH₃)₂ | H | -CH₂-CH₂-CH₂-CH(CH₃)- | CF₃ |
| 55. | N(CH₃)₂ | CH₃ | -CH₂-CH₂-CH₂-CH(CH₃)- | CF₃ |
| 56. | OH | H | -(CH₂)₄- | CHF₂ |
| 57. | OH | CH₃ | -(CH₂)₄- | CHF₂ |
| 58. | CH₃ | H | -(CH₂)₄- | CHF₂ |
| 59. | C(CH₃)₃ | H | -(CH₂)₄- | CHF₂ |
| 60. | C₆H₅ | H | -(CH₂)₄- | CHF₂ |
| 61. | C₆H₅ | CH₃ | -(CH₂)₄- | CHF₂ |
| 62. | CF₃ | H | -(CH₂)₄- | CHF₂ |
| 63. | OH | F | -(CH₂)₄- | CHF₂ |
| 64. | OH | CN | -(CH₂)₄- | CHF₂ |
| 65. | N(CH₃)₂ | H | -(CH₂)₄- | CHF₂ |
| 66. | N(CH₃)₂ | CH₃ | -(CH₂)₄- | CHF₂ |
| 67. | OH | H | *trans*-CH₂-CH=CH-CH₂- | CHF₂ |
| 68. | OH | CH₃ | *trans* -CH₂-CH=CH-CH₂- | CHF₂ |
| 69. | CH₃ | H | *trans* -CH₂-CH=CH-CH₂- | CHF₂ |
| 70. | C(CH₃)₃ | H | *trans* -CH₂-CH=CH-CH₂- | CHF₂ |
| 71. | C₆H₅ | H | *trans*-CH₂-CH=CH-CH₂- | CHF₂ |
| 72. | C₆H₅ | CH₃ | *trans*-CH₂-CH=CH-CH₂- | CHF₂ |
| 73. | CF₃ | H | *trans*-CH₂-CH=CH-CH₂- | CHF₂ |
| 74. | OH | F | *trans* -CH₂-CH=CH-CH₂- | CHF₂ |
| 75. | OH | CN | *trans* -CH₂-CH=CH-CH₂- | CHF₂ |
| 76. | N(CH₃)₂ | H | *trans* -CH₂-CH=CH-CH₂- | CHF₂ |
| 77. | N(CH₃)₂ | CH₃ | *trans* -CH₂-CH=CH-CH₂- | CHF₂ |
| 78. | OH | H | *trans* -CH₂-C(CH₃)=CH-CH₂- | CHF₂ |
| 79. | OH | CH₃ | *trans*-CH₂-C(CH₃)=CH-CH₂- | CHF₂ |
| 80. | CH₃ | H | *trans*-CH₂-C(CH₃)=CH-CH₂- | CHF₂ |
| 81. | C(CH₃)₃ | H | *trans* -CH₂-C(CH₃)=CH-CH₂- | CHF₂ |
| 82. | C₆H₅ | H | *trans* -CH₂-C(CH₃)=CH-CH₂- | CHF₂ |
| 83. | C₆H₅ | CH₃ | *trans* -CH₂-C(CH₃)=CH-CH₂- | CHF₂ |
| 84. | CF₃ | H | *trans* -CH₂-C(CH₃)=CH-CH₂- | CHF₂ |
| 85. | OH | F | *trans* -CH₂-C(CH₃)=CH-CH₂- | CHF₂ |
| 86. | OH | CN | *trans* -CH₂-C(CH₃)=CH-CH₂- | CHF₂ |
| 87. | N(CH₃)₂ | H | *trans* -CH₂-C(CH₃)=CH-CH₂-, | CHF₂ |
| 88. | N(CH₃)₂ | CH₃ | *trans*-CH₂-C(CH₃)=CH-CH₂- | CHF₂ |
| 89. | OH | H | -CH₂-CH(CH₃)-CH₂-CH₂- | CHF₂ |
| 90. | OH | CH₃ | -CH₂-CH(CH₃)-CH₂-CH₂- | CHF₂ |
| 91. | CH₃ | H | -CH₂-CH(CH₃)-CH₂-CH₂- | CHF₂ |
| 92. | C(CH₃)₃ | H | -CH₂-CH(CH₃)-CH₂-CH₂- | CHF₂ |
| 93. | C₆H₅ | H | -CH₂-CH(CH₃)-CH₂-CH₂- | CHF₂ |
| 94. | C₆H₅ | CH₃ | -CH₂-CH(CH₃)-CH₂-CH₂- | CHF₂ |
| 95. | CF₃ | H | -CH₂-CH(CH₃)-CH₂-CH₂- | CHF₂ |
| 96. | OH | F | -CH₂-CH(CH₃)-CH₂-CH₂- | CHF₂ |
| 97. | OH | CN | -CH₂-CH(CH₃)-CH₂-CH₂- | CHF₂ |
| 98. | N(CH₃)₂ | H | -CH₂-CH(CH₃)-CH₂-CH₂- | CHF₂ |
| 99. | N(CH₃)₂ | CH₃ | -CH₂-CH(CH₃)-CH₂-CH₂- | CHF₂ |
| 100. | OH | H | -CH₂-CH₂-CH₇CH(CH₃)- | CHF₂ |
| 101. | OH | CH₃ | -CH₂-CH₂-CH₂-CH(CH₃)- | CHF₂ |
| 102. | CH₃ | H | -CH₂-CH₂-CH₂-CH(CH₃)- | CHF₂ |
| 103. | C(CH₃)₃ | H | -CH₂-CH₂-CH₂-CH(CH₃)- | CHF₂ |
| 104. | C₆H₅ | H | -CH₂-CH₂-CH₂-CH(CH₃)- | CHF₂ |
| 105. | C₆H₅ | CH₃ | -CH₂-CH₂-CH₂-CH(CH₃)- | CHF₂ |
| 106. | CF₃ | H | -CH₂-CH₂-CH₂-CH(CH₃)- | CHF₂ |
| 107. | OH | F | -CH₂-CH₂-CH₂-CH(CH₃)- | CHF₂ |
| 108. | OH | CN | -CH₂-CH₂-CH₂-CH(CH₃)- | CHF₂ |
| 109. | N(CH₃)₂ | H | -CH₂-CH₂-CH₂-CH(CH₃)- | CHF₂ |
| 110. | N(CH₃)₂ | CH₃ | -CH₂-CH₂-CH₂-CH(CH₃)- | CHF₂ |
| 111. | OH | H | -(CH₂)₄- | C₆H₅ |
| 112. | OH | CH₃ | -(CH₂)₄- | C₆H₅ |
| 113. | CH₃ | H | -(CH₂)₄- | C₆H₅ |
| 114. | C(CH₃)₃ | H | -(CH₂)₄- | C₆H₅ |
| 115. | C₆H₅ | H | -(CH₂)₄- | C₆H₅ |
| 116. | C₆H₅ | CH₃ | -(CH₂)₄- | C₆H₅ |
| 117. | CF₃ | H | -(CH₂)₄- | C₆H₅ |
| 118. | OH | F | -(CH₂)₄- | C₆H₅ |
| 119. | OH | CN | -(CH₂)₄- | C₆H₅ |
| 120. | N(CH₃)₂ | H | -(CH₂)₄- | C₆H₅ |
| 121. | N(CH₃)₂ | CH₃ | -(CH₂)₄- | C₆H₅ |
| 122. | OH | H | *trans* -CH₂-CH=CH-CH₂- | C₆H₅ |
| 123. | OH | CH₃ | *trans* -CH₂-CH=CH-CH₂- | C₆H₅ |
| 124. | CH₃ | H | *trans* -CH₂-CH=CH-CH₂- | C₆H₅ |
| 125. | C(CH₃)₃ | H | *trans* -CH₂-CH=CH-CH₂- | C₆H₅ |
| 126. | C₆H₅ | H | *trans* -CH₂CH=CH-CH₂- | C₆H₅ |
| 127. | C₆H₅ | CH₃ | *trans* -CH₂-CH=CH-CH₂- | C₆H₅ |
| 128. | CF₃ | H | *trans* -CH₂-CH=CH-CH₂- | C₆H₅ |
| 129. | OH | F | *trans* -CH₂-CH=CH-CH₂- | C₆H₅ |
| 130. | OH | CN | *trans* -CH₂-CH=CH-CH₂- | C₆H₅ |
| 131. | N(CH₃)₂ | H | *trans*-CH₂-CH=CH-CH₂- | C₆H₅ |
| 132. | N(CH₃)₂ | CH₃ | *trans* -CH₂-CH=CH-CH₂- | C₆H₅ |
| 133. | OH | H | *trans* -CH₂-C(CH₃)=CH-CH₂- | C₆H₅ |
| 134. | OH | CH₃ | *trans* -CH₂-C(CH₃)=CH-CH₂- | C₆H₅ |
| 135. | CH₃ | H | *trans* -CH₂-C(CH₃)=CH-CH₂- | C₆H₅ |
| 136. | C(CH₃)₃ | H | *trans* -CH₂-C(CH₃)=CH-CH₂- | C₆H₅ |
| 137. | C₆H₅ | H | *trans* -CH₂-C(CH₃)=CH-CH₂- | C₆H₅ |
| 138. | C₆H₅ | CH₃ | *trans* -CH₂-C(CH₃)=CH-CH₂- | C₆H₅ |
| 139. | CF₃ | H | *trans* -CH₂-C(CH₃)=CH-CH₂- | C₆H₅ |
| 140. | OH | F | *trans* -CH₂-C(CH₃)=CH-CH₂- | C₆H₅ |
| 141. | OH | CN | *trans* -CH₂-C(CH₃)=CH-CH₂- | C₆H₅ |
| 142. | N(CH₃)₂ | H | *trans* -CH₂-C(CH₃)=CH-CH₂- | C₆H₅ |
| 143. | N(CH₃)₂ | CH₃ | *trans* -CH₂-C(CH₃)=CH-CH₂- | C₆H₅ |
| 144. | OH | H | -CH₂-CH(CH₃)-CH₂-CH₂- | C₆H₅ |
| 145. | OH | CH₃ | -CH₂-CH(CH₃)-CH₂-CH₂- | C₆H₅ |
| 146. | CH₃ | H | -CH₂-CH(CH₃)-CH₂-CH₂- | C₆H₅ |
| 147. | C(CH₃)₃ | H | -CH₂-CH(CH₃)-CH₂-CH₂- | C₆H₅ |
| 148. | C₆H₅ | H | -CH₂-CH(CH₃)-CH₂-CH₂- | C₆H₅ |
| 149. | C₆H₅ | CH₃ | -CH₂-CH(CH₃)-CH₂-CH₂- | C₆H₅ |
| 150. | CF₃ | H | -CH₂-CH(CH₃)-CH₂-CH₂- | C₆H₅ |
| 151. | OH | F | -CH₂-CH(CH₃)-CH₂-CH₂- | C₆H₅ |
| 152. | OH | CN | -CH₂-CH(CH₃)-CH₂-CH₂- | C₆H₅ |
| 153. | N(CH₃)₂ | H | -CH₂-CH(CH₃)-CH₂-CH₂- | C₆H₅ |
| 154. | N(CH₃)₂ | CH₃ | -CH₂-CH(CH₃)-CH₂-CH₂- | C₆H₅ |
| 155. | OH | H | -CH₂-CH₂-CH₂-CH(CH₃)- | C₆H₅ |
| 156. | OH | CH₃ | -CH₂-CH₂-CH₂-CH(CH₃)- | C₆H₅ |
| 157. | CH₃ | H | -CH₂-CH₂-CH₂-CH(CH₃)- | C₆H₅ |
| 158. | C(CH₃)₃ | H | -CH₂-CH₂-CH₂-CH(CH₃)- | C₆H₅ |
| 159. | C₆H₅ | H | -CH₂-CH₂-CH₂-CH(CH₃)- | C₆H₅ |
| 160. | C₆H₅ | CH₃ | -CH₂-CH₂-CH₂-CH(CH₃)- | C₆H₅ |
| 161. | CF₃ | H | -CH₂-CH₂-CH₂-CH(CH₃)- | C₆H₅ |
| 162. | OH | F | -CH₂-CH₂-CH₂-CH(CH₃)- | C₆H₅ |
| 163. | OH | CN | -CH₂-CH₂-CH₂-CH(CH₃)- | C₆H₅ |
| 164. | N(CH₃)₂ | H | -CH₂-CH₂-CH₂-CH(CH₃)- | C₆H₅ |
| 165. | N(CH₃)₂ | CH₃ | -CH₂-CH₂-CH₂-CH(CH₃)- | C₆H₅ |
| 166. | OH | H | -(CH₂)₄- | C(CH₃)₃ |
| 167. | OH | CH₃ | -(CH₂)₄- | C(CH₃)₃ |
| 168. | CH₃ | H | -(CH₂)₄- | C(CH₃)₃ |
| 169. | C(CH₃)₃ | H | -(CH₂)₄- | C(CH₃)₃ |
| 170. | C₆H₅ | H | -(CH₂)₄- | C(CH₃)₃ |
| 171. | C₆H₅ | CH₃ | -(CH₂)₄- | C(CH₃)₃ |
| 172. | CF₃ | H | -(CH₂)₄- | C(CH₃)₃ |
| 173. | OH | F | -(CH₂)₄- | C(CH₃)₃ |
| 174. | OH | CN | -(CH₂)₄- | C(CH₃)₃ |
| 175. | N(CH₃)₂ | H | -(CH₂)₄- | C(CH₃)₃ |
| 176. | N(CH₃)₂ | CH₃ | -(CH₂)₄- | C(CH₃)₃ |
| 177. | OH | H | *trans* -CH₂-CH=CH-CH₂- | C(CH₃)₃ |
| 178. | OH | CH₃ | *trans*-CH₂-CH=CH-CH₂- | C(CH₃)₃ |
| 179. | CH₃ | H | *trans* -CH₂-CH=CH-CH₂- | C(CH₃)₃ |
| 180. | C(CH₃)₃ | H | *trans* -CH₂-CH=CH-CH₂- | C(CH₃)₃ |
| 181. | C₆H₅ | H | *trans* -CH₂-CH=CH-CH₂- | C(CH₃)₃ |
| 182. | C₆H₅ | CH₃ | *trans* -CH₂-CH=CH-CH₂- | C(CH₃)₃ |
| 183. | CF₃ | H | *trans* -CH₂-CH=CH-CH₂- | C(CH₃)₃ |
| 184. | OH | F | *trans* -CH₂-CH=CH-CH₂- | C(CH₃)₃ |
| 185. | OH | CN | *trans* -CH₂-CH=CH-CH₂- | C(CH₃)₃ |
| 186. | N(CH₃)₂ | H | *trans* -CH₂-CH=CH-CH₂- | C(CH₃)₃ |
| 187. | N(CH₃)₂ | CH₃ | *trans* -CH₂-CH=CH-CH₂- | C(CH₃)₃ |
| 188. | OH | H | *trans* -CH₂-C(CH₃)=CH-CH₂- | C(CH₃)₃ |
| 189. | OH | CH₃ | *trans* -CH₂-C(CH₃)=CH-CH₂- | C(CH₃)₃ |
| 190. | CH₃ | H | *trans* -CH₂-C(CH₃)=CH-CH₂- | C(CH₃)₃ |
| 191. | C(CH₃)₃ | H | *trans* -CH₂-C(CH₃)=CH-CH₂- | C(CH₃)₃ |
| 192. | C₆H₅ | H | *trans* -CH₂-C(CH₃)=CH-CH₂- | C(CH₃)₃ |
| 193. | C₆H₅ | CH₃ | trans -CH₂-C(CH₃)=CH-CH₂- | C(CH₃)₃ |
| 194. | CF₃ | H | *trans* -CH₂-C(CH₃)=CH-CH₂- | C(CH₃)₃ |
| 195. | OH | F | *trans* -CH₂-C(CH₃)=CH-CH₂- | C(CH₃)₃ |
| 196. | OH | CN | *trans* -CH₂-C(CH₃)=CH-CH₂- | C(CH₃)₃ |
| 197. | N(CH₃)₂ | H | *trans*-CH₂-C(CH₃)=CH-CH₂- | C(CH₃)₃ |
| 198. | N(CH₃)₂ | CH₃ | *trans*-CH₂-C(CH₃)=CH-CH₂- | C(CH₃)₃ |
| 199. | OH | H | -CH₂-CH(CH₃)-CH₂-CH₂- | C(CH₃)₃ |
| 200. | OH | CH₃ | -CH₂-CH(CH₃)-CH₂-CH₂- | C(CH₃)₃ |
| 201. | CH₃ | H | -CH₂-CH(CH₃)-CH₂-CH₂- | C(CH₃)₃ |
| 202. | C(CH₃)₃ | H | -CH₂-CH(CH₃)-CH₂-CH₂- | C(CH₃)₃ |
| 203. | C₆H₅ | H | -CH₂-CH(CH₃)-CH₂-CH₂- | C(CH₃)₃ |
| 204. | C₆H₅ | CH₃ | -CH₂-CH(CH₃)-CH₂-CH₂- | C(CH₃)₃ |
| 205. | CF₃ | H | -CH₂-CH(CH₃)-CH₂-CH₂- | C(CH₃)₃ |
| 206. | OH | F | -CH₂-CH(CH₃)-CH₂-CH₂- | C(CH₃)₃ |
| 207. | OH | CN | -CH₂-CH(CH₃)-CH₂-CH₂- | C(CH₃)₃ |
| 208. | N(CH₃)₂ | H | -CH₂-CH(CH₃)-CH₂-CH₂- | C(CH₃)₃ |
| 209. | N(CH₃)₂ | CH₃ | -CH₂-CH(CH₃)-CH₂-CH₂- | C(CH₃)₃ |
| 210. | OH | H | -CH₂CH₂CH₂CH(CH₃)- | C(CH₃)₃ |
| 211. | OH | CH₃ | -CH₂-CH₂-CH₂-CH(CH₃)- | C(CH₃)₃ |
| 212. | CH₃ | H | -CH₂-CH₂-CH₂-CH(CH₃)- | C(CH₃)₃ |
| 213. | C(CH₃)₃ | H | -CH₂-CH₂-CH₂-CH(CH₃)- | C(CH₃)₃ |
| 214. | C₆H₅ | H | -CH₂-CH₂-CH₂-CH(CH₃)- | C(CH₃)₃ |
| 215. | C₆H₅ | CH₃ | -CH₂-CH₂-CH₂-CH(CH₃)- | C(CH₃)₃ |
| 216. | CF₃ | H | -CH₂-CH₂-CH₂-CH(CH₃)- | C(CH₃)₃ |
| 217. | OH | F | -CH₂-CH₂-CH₂-CH(CH₃)- | C(CH₃)₃ |
| 218. | OH | CN | -CH₂-CH₂-CH₂-CH(CH₃)- | C(CH₃)₃ |
| 219. | N(CH₃)₂ | H | -CH₂-CH2-CH₂-CH(CH₃)- | C(CH₃)₃ |
| 220. | N(CH₃)₂ | CH₃ | -CH₂-CH₂-CH₂-CH(CH₃)- | C(CH₃)₃ |
| 221. | OH | H | -(CH₂)₄- | *cyclo*-C₅H₉ |
| 222. | OH | CH₃ | -(CH₂)₄- | *cyclo*-C₅H₉ |
| 223. | CH₃ | H | -(CH₂)₄- | *cyclo*-C₅H₉ |
| 224. | C(CH₃)₃ | H | -(CH₂)₄- | *cyclo*-C₅H₉ |
| 225. | C₆H₅ | H | -(CH₂)₄- | *cyclo*-C₅H₉ |
| 226. | C₆H₅ | CH₃ | -(CH₂)₄- | *cyclo*-C₅H₉ |
| 227. | CF₃ | H | -(CH₂)₄- | *cyclo*-C₅H₉ |
| 228. | OH | F | -(CH₂)₄- | *cyclo-*C₅H₉ |
| 229. | OH | CN | -(CH₂)₄- | *cyclo*-C₅H₉ |
| 230. | N(CH₃)₂ | H | -(CH₂)₄- | *cyclo*-C₅H₉ |
| 231. | N(CH₃)₂ | CH₃ | -(CH₂)₄- | *cyclo*-C₅H₉ |
| 232. | OH | H | *trans*-CH₂-CH=CH-CH₂- | *cyclo*-C₅H₉ |
| 233. | OH | CH₃ | *trans*-CH₂-CH=CH-CH₂- | *cyclo*-C₂H₉ |
| 234. | CH₃ | H | *trans*-CH₂-CH=CH-CH₂- | *cyclo*-C₅H₉ |
| 235. | C(CH₃)₃ | H | *trans*-CH₂-CH=CH-CH₂- | *cyclo*-C₅H₉ |
| 236. | C₆H₅ | H | *trans* -CH₂-CH=CH-CH₂- | *cyclo*-C₅H₉ |
| 237. | C₆H₅ | CH₃ | *trans* -CH₂-CH=CH-CH₂- | *cyclo*-C₅H₉ |
| 238. | CF₃ | H | *trans*-CH₂-CH=CH-CH₂- | *cyclo*-C₅H₉ |
| 239. | OH | F | *trans* -CH₂-CH=CH-CH₂- | *cyclo*-C₅H₉ |
| 240. | OH | CN | *trans* -CH₂-CH=CH-CH₂- | *cyclo*-C₅H₉ |
| 241. | N(CH₃)₂ | H | *trans* -CH₂-CH=CH-CH₂- | *cyclo*-C₅H₉ |
| 242. | N(CH₃)₂ | CH₃ | *trans*-CH₂-CH=CH-CH₂- | *cyclo*-C₅H₉ |
| 243. | OH | H | *trans*-CH₂-C(CH₃)=CH-CH₂- | *cyclo*-C₅H₉ |
| 244. | OH | CH₃ | *trans* -CH₂-C(CH₃)=CH-CH₂- | *cyclo*-C₅H₉ |
| 245. | CH₃ | H | *trans* -CH₂-C(CH₃)=CH-CH₂- | *cyclo*-C₅H₉ |
| 246. | C(CH₃)₃ | H | *trans*-CH₂-C(CH₃)=CH-CH₂- | *cyclo*-C₅H₉ |
| 247. | C₆H₅ | H | *trans* -CH₂-C(CH₃)=CH-CH₂- | *cyclo-*C₅H₉ |
| 248. | C₆H₅ | CH₃ | *trans*-CH₂-C(CH₃)=CH-CH₂- | *cyclo*-C₅H₉ |
| 249. | CF₃ | H | *trans* -CH₂C(CH₃)=CH-CH₂- | *cyclo*-C₅H₉ |
| 250. | OH | F | *trans* -CH₂-C(CH₃)=CH-CH₂- | *cyclo*-C₅H₉ |
| 251. | OH | CN | *trans* -CH₂-C(CH₃)=CH-CH₂- | *cyclo*-C₅H₉ |
| 252. | N(CH₃)₂ | H | *trans*-CH₂-C(CH₃)=CH-CH₂- | *cyclo*-C₅H₉ |
| 253. | N(CH₃)₂ | CH₃ | *trans* -CH₂-C(CH₃)=CH-CH₂ | *cyclo*-C₅H₉ |
| 254. | OH | H | -CH₂-CH(CH₃)-CH₂-CH₂- | *cyclo*-C₅H₉ |
| 255. | OH | CH₃ | -CH₂-CH(CH₃)-CH₂-CH₂- | *cyclo-*C₅H₉ |
| 256. | CH₃ | H | -CH₂-CH(CH₃)-CH₂-CH₂- | *cyclo*-C₅H₉ |
| 257. | C(CH₃)₃ | H | -CH₂-CH(CH₃)-CH₂-CH₂- | *cyclo*-C₅H₉ |
| 258. | C₆H₅ | H | -CH₂-CH(CH₃)-CH₂-CH₂- | *cyclo*-C₅H₉ |
| 259. | C₆H₅ | CH₃ | -CH₂-CH(CH₃)-CH₂-CH₂- | *cyclo*-C₅H₉ |
| 260. | CF₃ | H | -CH₂-CH(CH₃)-CH₂-CH₂- | *cyclo*-C₅H₉ |
| 261. | OH | F | -CH₂-CH(CH₃)-CH₂-CH₂- | *cyclo*-C₅H₉ |
| 262. | OH | CN | -CH₂CH(CH₃)-CH₂-CH₂- | cyclo-C₅H₉ |
| 263. | N(CH₃)₂ | H | -CH₂-CH(CH₃)-CH₂-CH₂- | *cyclo*-C₅H₉ |
| 264. | N(CH₃)₂ | CH₃ | -CH₂-CH(CH₃)-CH₂-CH₂- | *cyclo*-C₅H₉ |
| 265. | OH | H | -CH₂-CH₂-CH₂-CH(CH₃)- | *cyclo*-C₅H₉ |
| 266. | OH | CH₃ | -CH₂-CH₂-CH₂-CH(CH₃)- | *cyclo*-C₅H₉ |
| 267. | CH₃ | H | -CH₂-CH₂-CH₂-CH(CH₃)- | *cyclo-*C₅H₉ |
| 268. | C(CH₃)₃ | H | -CH₂-CH₂-CH₂-CH(CH₃)- | *cyclo*-C₅H₉ |
| 269. | C₆H₅ | H | -CH₂-CH₂-CH₂-CH(CH₃)- | *cy*c*lo-*C₅H₉ |
| 270. | C₆H₅ | CH₃ | -CH₂-CH₂-CH₂-CH(CH₃)- | *cyclo*-C₅H₉ |
| 271. | CF₃ | H | -CH₂-CH₂-CH₂-CH(CH₃)- | *cyclo*-C₅H₉ |
| 272. | OH | F | -CH₂-CH₂-CH₂-CH(CH₃)- | *cyclo*-C₅H₉ |
| 273. | OH | CN | -CH₂-CH₂-CH₂-CH(CH₃)- | *cyclo*-C₅H₉ |
| 274. | N(CH₃)₂ | H | -CH₂-CH₂-CH₂-CH(CH₃)- | *cyclo*-C₅H₉ |
| 275. | N(CH₃)₂ | CH₃ | -CH₂-CH₂-CH₂-CH(CH₃)- | *cyclo*-C₅H₉ |
| 276. | OH | H | -(CH₂)₄- | CH₃ |
| 277. | OH | CH₃ | -(CH₂)₄- | CH₃ |
| 278. | CH₃ | H | -(CH₂)₄- | CH₃ |
| 279. | C(CH₃)₃ | H | -(CH₂)₄- | CH₃ |
| 280. | C₆H₅ | H | -(CH₂)₄- | CH₃ |
| 281. | C₆H₅ | CH₃ | -(CH₂)₄- | CH₃ |
| 282. | CF₃ | H | -(CH₂)₄- | CH₃ |
| 283. | OH | F | -(CH₂)₄- | CH₃ |
| 284. | OH | CN | -(CH₂)₄- | CH₃ |
| 285. | N(CH₃)₂ | H | -(CH₂)₄- | CH₃ |
| 286. | N(CH₃)₂ | CH₃ | -(CH₂)₄- | CH₃ |
| 287. | OH | H | *trans* -CH₂-CH=CH-CH₂ | CH₃ |
| 288. | OH | CH₃ | *trans* -CH₂CH=CH-CH₂- | CH₃ |
| 289. | CH₃ | H | *trans* -CH₂CH=CH-CH₂- | CH₃ |
| 290. | C(CH₃)₃ | H | *trans* -CH₂-CH=CH-CH₂- | CH₃ |
| 291. | C₆H₅ | H | *trans*-CH₂-CH=CH-CH₂- | CH₃ |
| 292. | C₆H₅ | CH₃ | *trans* -CH₂-CH=CH-CH₂- | CH₃ |
| 293. | CF₃ | H | *trans* -CH₂-CH=CH-CH₂- | CH₃ |
| 294. | OH | F | *trans* -CH₂-CH=CH-CH₂- | CH₃ |
| 295. | OH | CN | *trans* -CH₂-CH=CH-CH₂- | CH₃ |
| 296. | N(CH₃)₂ | H | *trans* -CH₂-CH=CH-CH₂- | CH₃ |
| 297. | N(CH₃)₂ | CH₃ | *trans* -CH₂-CH=CH-CH₂- | CH₃ |
| 298. | OH | H | *trans* -CH₂-C(CH₃)=CH-CH₂- | CH₃ |
| 299. | OH | CH₃ | *trans* -CH₂-C(CH₃)=CH-CH₂- | CH₃ |
| 300. | CH₃ | H | *trans* -CH₂-C(CH₃)=CH-CH₂- | CH₃ |
| 301. | C(CH₃)₃ | H | *trans* -CH₂-C(CH₃)=CH-CH₂- | CH₃ |
| 302. | C₆H₅ | H | *trans* -CH₂-C(CH₃)=CH-CH₂- | CH₃ |
| 303. | C₆H₅ | CH₃ | *trans* -CH₂-C(CH₃)=CH-CH₂- | CH₃ |
| 304. | CF₃ | H | *trans* -CH₂-C(CH₃)=CH-CH₂- | CH₃ |
| 305. | OH | F | *trans* -CH₂-C(CH₃)=CH-CH₂- | CH₃ |
| 306. | OH | CN | *trans* -CH₂-C(CH₃)=CH-CH₂- | CH₃ |
| 307. | N(CH₃)₂ | H | *trans* -CH₂-C(CH₃)=CH-CH₂- | CH₃ |
| 308. | N(CH₃)₂ | CH₃ | *trans* -CH₂-C(CH₃)=CH-CH₂- | CH₃ |
| 309. | OH | H | -CH₂-CH(CH₃)-CH₂-CH₂- | CH₃ |
| 310. | OH | CH₃ | -CH₂-CH(CH₃)-CH₂-CH₂- | CH₃ |
| 311. | CH₃ | H | -CH₂-CH(CH₃)-CH₂-CH₂- | CH₃ |
| 312. | C(CH₃)₃ | H | -CH₂-CH(CH₃)-CH₂-CH₂- | CH₃ |
| 313. | C₆H₅ | H | -CH₂-CH(CH₃)-CH₂-CH₂- | CH₃ |
| 314. | C₆H₅ | CH₃ | -CH₂-CH(CH₃)-CH₂-CH₂- | CH₃ |
| 315. | CF₃ | H | -CH₂-CH(CH₃)-CH₂-CH₂- | CH₃ |
| 316. | OH | F | -CH₂CH(CH₃)-CH₂-CH₂- | CH₃ |
| 317. | OH | CN | -CH₂-CH(CH₃)-CH₂-CH₂- | CH₃ |
| 318. | N(CH₃)₂ | H | -CH₂-CH(CH₃)-CH₂-CH₂- | CH₃ |
| 319. | N(CH₃)₂ | CH₃ | -CH₂-CH(CH₃)-CH₂-CH₂- | CH₃ |
| 320. | OH | H | -CH₂-CH₂-CH₂-CH(CH₃)- | CH₃ |
| 321. | OH | CH₃ | -CH₂-CH₂-CH₂-CH(CH₃)- | CH₃ |
| 322. | CH₃ | H | -CH₂-CH₂-CH₂-CH(CH₃)- | CH₃ |
| 323. | C(CH₃)₃ | H | -CH₂-CH₂-CH₂-CH(CH₃)- | CH₃ |
| 324. | C₆H₅ | H | -CH₂-CH₂-CH₂-CH(CH₃)- | CH₃ |
| 325. | C₆H₅ | CH₃ | -CH₂-CH₂-CH₂-CH(CH₃)- | CH₃ |
| 326. | CF₃ | H | -CH₂-CH₂-CH₂-CH(CH₃)- | CH₃ |
| 327. | OH | F | -CH₂-CH₂-CH₂-CH(CH₃)- | CH₃ |
| 328. | OH | CN | -CH₂-CH₂-CH₂-CH(CH₃)- | CH₃ |
| 329. | N(CH₃)₂ | H | -CH₂-CH₂-CH₂-CH(CH₃)- | CH₃ |
| 330. | N(CH₃)₂ | CH₃ | -CH₂-CH₂-CH₂-CH(CH₃)- | CH₃ |
| 331. | OH | H | -(CH₂)₄- | CH(CH₃)₂ |
| 332. | OH | CH₃ | -(CH₂)₄- | CH(CH₃)₂ |
| 333. | CH₃ | H | -(CH₂)₄- | CH(CH₃)₂ |
| 334. | C(CH₃)₃ | H | -(CH₂)₄- | CH(CH₃)₂ |
| 335. | C₆H₅ | H | -(CH₂)₄- | CH(CH₃)₂ |
| 336. | C₆H₅ | CH₃ | -(CH₂)₄- | CH(CH₃)₂ |
| 337. | CF₃ | H | -(CH₂)₄- | CH(CH₃)₂ |
| 338. | OH | F | -(CH₂)₄- | CH(CH₃)₂ |
| 339. | OH | CN | -(CH₂)₄- | CH(CH₃)₂ |
| 340. | N(CH₃)₂ | H | -(CH₂)₄- | CH(CH₃)₂ |
| 341: | N(CH₃)₂ | CH₃ | -(CH₂)₄- | CH(CH₃)₂ |
| 342. | OH | H | *trans* -CH₂-CH=CH-CH₂- | CH(CH₃)₂ |
| 343. | OH | CH₃ | *trans* -CH₂-CH=CH-CH₂- | CH(CH₃)₂ |
| 344. | CH₃ | H | *trans* -CH₂-CH=CH-CH₂- | CH(CH₃)₂ |
| 345. | C(CH₃)₃ | H | *trans* -CH₂-CH=CH-CH₂- | CH(CH₃)₂ |
| 346. | C₆H₅ | H | *trans* -CH₂-CH=CH-CH₂- | CH(CH₃)₂ |
| 347. | C₆H₅ | CH₃ | *trans* -CH₂-CH=CH-CH₂- | CH(CH₃)₂ |
| 348. | CF₃ | H | *trans* -CH₂-CH=CH-CH₂- | CH(CH₃)₂ |
| 349. | OH | F | *trans* -CH₂-CH=CH-CH₂- | CH(CH₃)₂ |
| 350. | OH | CN | *trans* -CH₂-CH=CH-CH₂- | CH(CH₃)₂ |
| 351. | N(CH₃)₂ | H | *trans* -CH₂-CH=CH-CH₂- | CH(CH₃)₂ |
| 352. | N(CH₃)₂ | CH₃ | *trans* -CH₂-CH=CH-CH₂- | CH(CH₃)₂ |
| 353. | OH | H | *trans* -CH₂-C(CH₃)=CH-CH₂- | CH(CH₃)₂ |
| 354. | OH | CH₃ | *trans* -CH₂-C(CH₃)=CH-CH₂- | CH(CH₃)₂ |
| 355. | CH₃ | H | *trans* -CH₂C(CH₃)=CH-CH₂- | CH(CH₃)₂ |
| 356. | C(CH₃)₃ | H | *trans* -CH₂-C(CH₃)=CH-CH₂- | CH(CH₃)₂ |
| 357. | C₆H₅ | H | *trans* -CH₂-C(CH₃)=CH-CH₂- | CH(CH₃)₂- |
| 358. | C₆H₅ | CH₃ | *trans* -CH₂-C(CH₃)=CH-CH₂- | CH(CH₃)₂ |
| 359. | CF₃ | H | *trans* -CH₂-C(CH₃)=CH-CH₂- | CH(CH₃)₂ |
| 360. | OH | F | *trans* -CH₂-C(CH₃)=CH-CH₂- | CH(CH₃)₂ |
| 361. | OH | CN | *trans* -CH₂-C(CH₃)=CH-CH₂- | CH(CH₃)₂ |
| 362. | N(CH₃)₂ | H | *trans* -CH₂-C(CH₃)=CH-CH₂- | CH(CH₃)₂ |
| 363. | N(CH₃)₂ | CH₃ | *trans* -CH₂-C(CH₃)=CH-CH₂- | CH(CH₃)₂ |
| 364. | OH | H | -CH₂-CH(CH₃)-CH₂-CH₂- | CH(CH₃)₂ |
| 365. | OH | CH₃ | -CH₂-CH(CH₃)-CH₂-CH₂- | CH(CH₃)₂ |
| 366. | CH₃ | H | -CH₂-CH(CH₃)-CH₂-CH₂- | CH(CH₃)₂ |
| 367. | C(CH₃)₃ | H | -CH₂-CH(CH₃)-CH₂-CH₂- | CH(CH₃)₂ |
| 368. | C₆H₅ | H | -CH₂-CH(CH₃)-CH₂-CH₂- | CH(CH₃)₂ |
| 369. | C₆H₅ | CH₃ | -CH₂-CH(CH₃)-CH₂-CH₂- | CH(CH₃)₂ |
| 370. | CF₃ | H | -CH₂-CH(CH₃)-CH₂-CH₂- | CH(CH₃)₂ |
| 371. | OH | F | -CH₂-CH(CH₃)-CH₂-CH₂- | CH(CH₃)₂ |
| 372. | OH | CN | -CH₂-CH(CH₃)-CH₂-CH₂- | CH(CH₃)₂ |
| 373. | N(CH₃)₂ | H | -CH₂-CH(CH₃)-CH₂-CH₂- | CH(CH₃)₂ |
| 374. | N(CH₃)₂ | CH₃ | -CH₂-CH(CH₃)-CH₂-CH₂- | CH(CH₃)₂ |
| 375. | OH | H | -CH₂-CH₂-CH₂-CH(CH₃)- | CH(CH₃)₂ |
| 376. | OH | CH₃ | -CH₂CH₂-CH₂-CH(CH)₃- | CH(CH₃)₂ |
| 377. | CH₃ | H | -CH₂-CH₂-CH₂-CH(CH₃)- | CH(CH₃)₂ |
| 378. | C(CH₃)₃ | H | -CH₂-CH₂-CH₂-CH(CH₃)- | CH(CH₃)₂ |
| 379. | C₆H₅ | H | -CH₂CH₂-CH₂-CH(CH₃)- | CH(CH₃)₂ |
| 380. | C₆H₅ | CH₃ | -CH₂-CH₂-CH₂-CH(CH₃)- | CH(CH₃)₂ |
| 381. | CF₃ | H | -CH₂-CH₂-CH₂-CH(CH₃)- | CH(CH₃)₂ |
| 382. | OH | F | -CH₂-CH₂-CH₂-CH(CH₃)- | CH(CH₃)₂ |
| 383. | OH | CN | -CH₂-CH₂-CH₂-CH(CH₃)- | CH(CH₃)₂ |
| 384. | N(CH₃)₂ | H | -CH₂CH₂-CH₂-CH(CH₃)- | CH(CH₃)₂ |
| 385. | N(CH₃)₂ | CH₃ | -CH₂-CH₂-CH₂-CH(CH₃)- | CH(CH₃)₂ |
| 386. | OH | H | -(CH₂)₄- | CH₂CH₃ |
| 387. | OH | CH₃ | -(CH₂)₄- | CH₂CH₃ |
| 388. | CH₃ | H | -(CH₂)₄- | CH₂CH₃ |
| 389. | C(CH₃)₃ | H | -(CH₂)₄- | CH₂CH₃ |
| 390. | C₆H₅ | H | -(CH₂)₄- | CH₂CH₃ |
| 391. | C₆H₅ | CH₃ | -(CH₂)₄- | CH₂CH₃ |
| 392. | CF₃ | H | -(CH₂)₄- | CH₂CH₃ |
| 393. | OH | F | -(CH₂)₄- | CH₂CH₃ |
| 394. | OH | CN | -(CH₂)₄- | CH₂CH₃ |
| 395. | N(CH₃)₂ | H | -(CH₂)₄- | CH₂CH₃ |
| 396. | N(CH₃)₂ | CH₃ | -(CH₂)₄- | CH₂CH₃ |
| 397. | OH | H | *trans* -CH₂-CH=CH-CH₂- | CH₂CH₃ |
| 398. | OH | CH₃ | *trans* -CH₂-CH=CH-CH₂- | CH₂CH₃ |
| 399. | CH₃ | H | *trans* -CH₂-CH=CH-CH₂- | CH₂CH₃ |
| 400. | C(CH₃)₃ | H | *trans* -CH₂-CH=CH-CH₂- | CH₂CH₃ |
| 401. | C₆H₅ | H | *trans* -CH₂-CH=CH-CH₂- | CH₂CH₃ |
| 402. | C₆H₅ | CH₃ | *trans* -CH₂-CH=CH-CH₂- | CH₂CH₃ |
| 403. | CF₃ | H | *trans* -CH₂-CH=CH-CH₂- | CH₂CH₃ |
| 404. | OH | F | *trans* -CH₂-CH=CH-CH₂- | CH₂CH₃ |
| 405. | OH | CN | *trans* -CH₂-CH=CH-CH₂- | CH₂CH₃ |
| 406. | N(CH₃)₂ | H | *trans* -CH₂-CH=CH-CH₂- | CH₂CH₃ |
| 407. | N(CH₃)₂ | CH₃ | *trans*-CH₂-CH=CH-CH₂- | CH₂CH₃ |
| 408. | OH | H | *trans-*CH₂-C(CH₃)=CH-CH₂- | CH₂CH₃ |
| 409. | OH | CH₃ | *trans-*CH₂-C(CH₃)=CH-CH₂- | CH₂CH₃ |
| 410. | CH₃ | H | *trans*-CH₂-C(CH₃)=CH-CH₂- | CH₂CH₃ |
| 411. | C(CH₃)₃ | H | *trans* -CH₂-C(CH₃)=CH-CH₂- | CH₂CH₃ |
| 412. | C₆H₅ | H | *trans*-CH₂-C(CH₃)=CH-CH₂- | CH₂CH₃ |
| 413. | C₆H₅ | CH₃ | *trans* -CH₂-C(CH₃)=CH-CH₂- | CH₂CH₃ |
| 414. | CF₃ | H | *trans*-CH₂-C(CH₃)=CH-CH₂- | CH₂CH₃ |
| 415. | OH | F | *trans*-CH₂-C(CH₃)=CH-CH₂- | CH₂CH₃ |
| 416. | OH | CN | *trans*-CH₂-C(CH₃)=CH-CH₂- | CH₂CH₃ |
| 417. | N(CH₃)₂ | H | *trans*-CH₂-C(CH₃)=CH-CH₂- | CH₂CH₃ |
| 418. | N(CH₃)₂ | CH₃ | *trans* -CH₂-C(CH₃)=CH-CH₂- | CH₂CH₃ |
| 419. | OH | H | -CH₂-CH(CH₃)-CH₂-CH₂- | CH₂CH₃ |
| 420. | OH | CH₃ | -CH₂-CH(CH₃)-CH₂-CH₂- | CH₂CH₃ |
| 421. | CH₃ | H | -CH₂-CH(CH₃)-CH₂-CH₂- | CH₂CH₃ |
| 422. | C(CH₃)₃ | H | -CH₂-CH(CH₃)-CH₂-CH₂- | CH₂CH₃ |
| 423. | C₆H₅ | H | -CH₂CH(CH₃)-CH₂-CH₂- | CH₂CH₃ |
| 424. | C₆H₅ | CH₃ | -CH₂-CH(CH₃)-CH₂-CH₂- | CH₂CH₃ |
| 425. | CF₃ | H | -CH₂-CH(CH₃)-CH₂-CH₂- | CH₂CH₃ |
| 426. | OH | F | -CH₂-CH(CH₃)-CH₂-CH₂- | CH₂CH₃ |
| 427. | OH | CN | -CH₂-CH(CH₃)-CH₂-CH₂- | CH₂CH₃ |
| 428. | N(CH₃)₂ | H | -CH₂-CH(CH₃)-CH₂-CH₂- | CH₂CH₃ |
| 429. | N(CH₃)₂ | CH₃ | -CH₂-CH(CH₃)-CH₂-CH₂- | CH₂CH₃ |
| 430. | OH | H | -CH₂-CH₂-CH₂-CH(CH₃)- | CH₂CH₃ |
| 431. | OH | CH₃ | -CH₂-CH₂-CH₂-CH(CH₃)- | CH₂CH₃ |
| 432. | CH₃ | H | -CH₂-CH₂-CH₂-CH(CH₃)- | CH₂CH₃ |
| 433. | C(CH₃)₃ | H | -CH₂-CH₂-CH₂-CH(CH₃)- | CH₂CH₃ |
| 434. | C₆H₅ | H | -CH₂-CH₂-CH₂-CH(CH₃)- | CH₂CH₃ |
| 435. | C₆H₅ | CH₃ | -CH₂-CH₂-CH₂-CH(CH₃)- | CH₂CH₃ |
| 436. | CF₃ | H | -CH₂-CH₂-CH₂-CH(CH₃)- | CH₂CH₃ |
| 437. | OH | F | -CH₂-CH₂-CH₂-CH(CH₃)- | CH₂CH₃ |
| 438. | OH | CN | -CH₂-CH₂-CH₂-CH(CH₃)- | CH₂CH₃ |
| 439. | N(CH₃)₂ | H | -CH₂-CH₂-CH₂-CH(CH₃)- | CH₂CH₃ |
| 440. | N(CH₃)₂ | CH₃ | -CH₂-CH₂-CH₂-CH(CH₃)- | CH₂CH₃ |
| 441. | OH | H | -(CH₂)₄- | CH₂CH₂CH₃ |
| 442. | OH | CH₃ | -(CH₂)₄- | CH₂CH₂CH₃ |
| 443. | CH₃ | H | -(CH₂)₄- | CH₂CH₂CH₃ |
| 444. | C(CH₃)₃ | H | -(CH₂)₄- | CH₂CH₂CH₃ |
| 445. | C₆H₅ | H | -(CH₂)₄- | CH₂CH₂CH₃ |
| 446. | C₆H₅ | CH₃ | -(CH₂)₄- | CH₂CH₂CH₃ |
| 447. | CF₃ | H | -(CH₂)₄- | CH₂CH₂CH₃ |
| 448. | OH | F | -(CH₂)₄- | CH₂CH₂CH₃ |
| 449. | OH | CN | -(CH₂)₄- | CH₂CH₂CH₃ |
| 450. | N(CH₃)₂ | H | -(CH₂)₄- | CH₂CH₂CH₃ |
| 451. | N(CH₃)₂ | CH₃ | -(CH₂)₄- | CH₂CH₂CH₃ |
| 452. | OH | H | *trans* -CH₂-CH=CH-CH₂- | CH₂CH₂CH₃ |
| 453. | OH | CH₃ | *trans* -CH₂-CH=CH-CH₂ | CH₂CH₂CH₃ |
| 454. | CH₃ | H | *trans* -CH₂-CH=CH-CH₂- | CH₂CH₂CH₃ |
| 455. | C(CH₃)₃ | H | *trans* -CH₂-CH=CH-CH₂- | CH₂CH₂CH₃ |
| 456. | C₈H₅ | H | *trans* -CH₂-CH=CH-CH₂- | CH₂CH₂CH₃ |
| 457. | C₆H₅ | CH₃ | *trans* -CH₂-CH=CH-CH₂- | CH₂CH₂CH₃ |
| 458. | CF₃ | H | *trans* -CH₂-CH=CH-CH₂- | CH₂CH₂CH₃ |
| 459. | OH | F | *trans*-CH₂-CH=CH-CH₂- | CH₂CH₂CH₃ |
| 460. | OH | CN | *trans* -CH₂-CH=CH-CH₂ | CH₂CH₂CH₃ |
| 461. | N(CH₃)₂ | H | *trans* -CH₂-CH=CH-CH₂- | CH₂CH₂CH₃ |
| 462. | N(CH₃)₂ | CH₃ | *trans* -CH₂-CH=CH-CH₂- | CH₂CH₂CH₃ |
| 463. | OH | H | *trans* -CH₂-C(CH₃)=CH-CH₂- | CH₂CH₂CH₃ |
| 464. | OH | CH₃ | *trans* -CH₂-C(CH₃)=CH-CH₂- | CH₂CH₂CH₃ |
| 465. | CH₃ | H | *trans* -CH₂-C(CH₃)=CH-CH₂- | CH₂CH₂CH₃ |
| 466. | C(CH₃)₃ | H | *trans* -CH₂-C(CH₃)=CH-CH₂- | CH₂CH₂CH₃ |
| 467. | C₆H₅ | H | *trans* -CH₂C(CH₃)=CH-CH₂- | CH₂CH₂CH₃ |
| 468. | C₆H₅ | CH₃ | *trans* -CH₂-C(CH₃)=CH-CH₂- | CH₂CH₂CH₃ |
| 469. | CF₃ | H | *trans* -CH₂-C(CH₃)=CH-CH₂- | CH₂CH₂CH₃ |
| 470. | OH | F | *trans* -CH₂-C(CH₃)=CH-CH₂- | CH₂CH₂CH₃ |
| 471. | OH | CN | *trans* -CH₂-C(CH₃)=CH-CH₂- | CH₂CH₂CH₃ |
| 472. | N(CH₃)₂ | H | *trans* -CH₂-C(CH₃)=CH-CH₂ | CH₂CH₂CH₃ |
| 473. | N(CH₃)₂ | CH₃ | *trans* -CH₂-C(CH₃)=CH-CH₂ | CH₂CH₂CH₃ |
| 474. | OH | H | -CH₂-CH(CH₃)-CH₂-CH₂- | CH₂CH₂CH₃ |
| 475. | OH | CH₃ | -CH₂-CH(CH₃)-CH₂-CH₂- | CH₂CH₂CH₃ |
| 476. | CH₃ | H | -CH₂-CH(CH₃)-CH₂-CH₂- | CH₂CH₂CH₃ |
| 477. | C(CH₃)₃ | H | -CH₂-CH(CH₃)-CH₂-CH₂- | CH₂CH₂CH₃ |
| 478. | C₆H₅ | H | -CH₂CH(CH₃)-CH₂-CH₂ | CH₂CH₂CH₃ |
| 479. | C₆H₅ | CH₃ | -CH₂-CH(CH₃)-CH₂-CH₂- | CH₂CH₂CH₃ |
| 480. | CF₃ | H | -CH₂-CH(CH₃)-CH₂-CH₂- | CH₂CH₂CH₃ |
| 481. | OH | F | -CH₂-CH(CH₃)-CH₂-CH₂- | CH₂CH₂CH₃ |
| 482. | OH | CN | -CH₂-CH(CH₃)-CH₂-CH₂ | CH₂CH₂CH₃ |
| 483. | N(CH₃)₂ | H | -CH₂-CH(CH₃)-CH₂-CH₂- | CH₂CH₂CH₃ |
| 484. | N(CH₃)₂ | CH₃ | -CH₂-CH(CH₃)-CH₂-CH₂- | CH₂CH₂CH₃ |
| 485. | OH | H | -CH₂-CH₂-CH₂-CH(CH₃)- | CH₂CH₂CH₃ |
| 486. | OH | CH₃ | -CH₂-CH₂-CH₂-CH(CH₃)- | CH₂CH₂CH₃ |
| 487. | CH₃ | H | -CH₂-CH₂-CH₂-CH(CH₃)- | CH₂CH₂CH₃ |
| 488. | C(CH₃)₃ | H | -CH₂CH₂-CH₂-CH(CH₃)- | CH₂CH₂CH₃ |
| 489. | C₆H₅ | H | -CH₂-CH₂-CH₂-CH(CH₃)- | CH₂CH₂CH₃ |
| 490. | C₆H₅ | CH₃ | -CH₂-CH₂-CH₂-CH(CH₃)- | CH₂CH₂CH₃ |
| 491. | CF₃ | H | -CH₂-CH₂-CH₂-CH(CH₃)- | CH₂CH₂CH₃ |
| 492. | OH | F | -CH₂-CH₂-CH₂-CH(CH₃)- | CH₂CH₂CH₃ |
| 493. | OH | CN | -CH₂-CH₂-CH₂-CH(CH₃)- | CH₂CH₂CH₃ |
| 494. | N(CH₃)₂ | H | -CH₂-CH₂-CH₂-CH(CH₃)- | CH₂CH₂CH₃ |
| 495. | N(CH₃)₂ | CH₃ | -CH₂-CH₂-CH₂-CH(CH₃)- | CH₂CH₂CH₃ |
| 496. | 1-Methylpyrrol-2-yl | H | -(CH₂)₄- | CF₃ |
| 497. | 3-Pyridyl | H | -(CH₂)₄- | CF₃ |
| 498. | 3-Thienyl | H | -(CH₂)₄- | CF₃ |
| 499. | 4-Fluorphenyl | H | -(CH₂)₄- | CF₃ |
| 500. | 4-Pyridyl | H | -(CH₂)₄- | CF₃ |
| 501. | 3-Furyl | H | -(CH₂)₄- | CF₃ |
| 502. | 2-Furyl | H | -(CH₂)₄- | CF₃ |
| 503. | 2-Pyrrolyl | H | -(CH₂)₄- | CF₃ |
| 504. | 2-Thienyt | H | -(CH₂)₄- | CF₃ |
| 505. | Pyridazin-2-yl | H | -(CH₂)₄- | CF₃ |
| 506. | 4-Methylthiazol-5-yl | H | -(CH₂)₄- | CF₃ |
| 507. | 2-Methyloxazol-4-yl | H | -(CH₂)₄- | CF₃ |
| 508. | Cyclopropyl | H | -(CH₂)₄- | CF₃ |
| 509. | Cyclobutyl | H | -(CH₂)₄- | CF₃ |
| 510. | Cyclopentyl | H | -(CH₂)₄- | CF₃ |
| 511. | Cyclohexyl | H | -(CH₂)₄- | CF₃ |
| 512. | H₃C-O-CH₂ | H | -(CH₂)₄- | CF₃ |
| 513. | Oxan-4-yl | H | -(CH₂)₄- | CF₃ |
| 514. | 1-Methylpiperidin-4-yl | H | -(CH₂)₄- | CF₃ |
| 515. | 1-Methylpyrrol-2-yl | H | *trans* -CH₂-CH=CH-CH₂- | CF₃ |
| 516. | 3-Pyridyl | H | *trans* -CH₂-CH=CH-CH₂- | CF₃ |
| 517. | 3-Thienyl | H | *trans* -CH₂-CH=CH-CH₂- | CF₃ |
| 518. | 4-Fluorphenyl | H | *trans* -CH₂-CH=CH-CH₂- | CF₃ |
| 519. | 4-Pyridyl | H | *trans* -CH₂-CH=CH-CH₂- | CF₃ |
| 520. | 3-Furyl | H | *trans* -CH₂-CH=CH-CH₂- | CF₃ |
| 521. | 2-Furyl | H | *trans* -CH₂-CH=CH-CH₂- | CF₃ |
| 522. | 2-Pyrrolyl | H | *trans* -CH₂-CH=CH-CH₂- | CF₃ |
| 523. | 2-Thienyl | H | *trans* -CH₂-CH=CH-CH₂- | CF₃ |
| 524. | Pyridazin-2-yl | H | *trans*-CH₂-CH=CH-CH₂- | CF₃ |
| 525. | 4-Methylthiazol-5-yl | H | *trans*-CH₂-CH=CH-CH₂- | CF₃ |
| 526. | 2-Methyloxazol-4-yl | H | *trans*-CH₂-CH=CH-CH₂- | CF₃ |
| 527. | Cyclopropyl | H | *trans*-CH₂-CH=CH-CH₂- | CF₃ |
| 528. | Cyclobutyl | H | *trans* -CH₂-CH=CH-CH₂- | CF₃ |
| 529. | Cyclopentyl | H | *trans* -CH₂-CH=CH-CH₂- | CF₃ |
| 530. | Cyclohexyl | H | *trans* -CH₂-CH=CH-CH₂- | CF₃ |
| 531. | H₃C-O-CH₂ | H | *trans*-CH₂-CH=CH-CH₂- | CF₃ |
| 532. | Oxan-4-yl | H | *trans*-CH₂-CH=CH-CH₂- | CF₃ |
| 533. | 1-Methylpiperidin-4-yl | H | *trans* -CH₂-CH=CH-CH₂- | CF₃ |
| 534. | 1-Methylpyrrol-2-yl | H | -(CH₂)₄- | CH₂CH₂CH₃ |
| 535. | 3-Pyridyl | H | -(CH₂)₄- | CH₂CH₂CH₃ |
| 536. | 3-Thienyl | H | -(CH₂)₄- | CH₂CH₂CH₃ |
| 537. | 4-Fluorphenyl | H | -(CH₂)₄- | CH₂CH₂CH₃ |
| 538. | 4-Pyridyl | H | -(CH₂)₄- | CH₂CH₂CH₃ |
| 539. | 3-Furyl | H | -(CH₂)₄- | CH₂CH₂CH₃ |
| 540. | 2-Furyl | H | -(CH₂)₄- | CH₂CH₂CH₃ |
| 541. | 2-Pyrrolyl | H | -(CH₂)₄- | CH₂CH₂CH₃ |
| 542. | 2-Thienyl | H | -(CH₂)₄- | CH₂CH₂CH₃ |
| 543. | Pyridazin-2-yl | H | -(CH₂)₄- | CH₂CH₂CH₃ |
| 544. | 4-Methylthiazol-5-yl | H | -(CH₂)₄- | CH₂CH₂CH₃ |
| 545. | 2-Methyloxazol-4-yl | H | -(CH₂)₄- | CH₂CH₂CH₃ |
| 546. | Cyclopropyl | H | -(CH₂)₄- | CH₂CH₂CH₃ |
| 547. | Cyclobutyl | H | -(CH₂)₄- | CH₂CH₂CH₃ |
| 548. | Cyclopentyl | H | -(CH₂)₄- | CH₂CH₂CH₃ |
| 549. | Cyclohexyl | H | -(CH₂)₄- | CH₂CH₂CH₃ |
| 550. | H₃C-O-CH₂ | H | -(CH₂)₄- | CH₂CH₂CH₃ |
| 551. | Oxan-4-yl | H | -(CH₂)₄- | CH₂CH₂CH₃ |
| 552. | 1-Methylpiperidin-4-yl | H | -(CH₂)₄- | CH₂CH₂CH₃ |
| 553. | 1-Methylpyrrol-2-yl | H | *trans* -CH₂-CH=CH-CH₂- | CH₂CH₂CH₃ |
| 554. | 3-Pyddyl | H | *trans* -CH₂-CH=CH-CH₂- | CH₂CH₂CH₃ |
| 555. | 3-Thienyl | H | *trans* -CH₂-CH=CH-CH₂- | CH₂CH₂CH₃ |
| 556. | 4-Fluorphenyl | H | *trans* -CH₂-CH=CH-CH₂- | CH₂CH₂CH₃ |
| 557. | 4-Pyridyl | H | *trans* -CH₂-CH=CH-CH₂- | CH₂CH₂CH₃ |
| 558. | 3-Furyl | H | *trans* -CH₂-CH=CH-CH₂- | CH₂CH₂CH₃ |
| 559. | 2-Furyl | H | *trans* -CH₂-CH=CH-CH₂- | CH₂CH₂CH₃ |
| 560. | 2-Pyrrolyl | H | *trans* -CH₂-CH=CH-CH₂- | CH₂CH₂CH₃ |
| 561. | 2-Thienyl | H | *trans* -CH₂-CH=CH-CH₂- | CH₂CH₂CH₃ |
| 562. | Pyridazin-2-yl | H | *trans* -CH₂-CH=CH-CH₂- | CH₂CH₂CH₃ |
| 563. | 4-Methylthiazol-5-yl | H | *trans* -CH₂-CH=CH-CH₂- | CH₂CH₂CH₃ |
| 564. | 2-Methyloxazol-4-yl | H | *trans* -CH₂CH=CH-CH₂- | CH₂CH₂CH₃ |
| 565. | Cyclopropyl | H | *trans* -CH₂-CH=CH-CH₂- | CH₂CH₂CH₃ |
| 566. | Cyclobutyl | H | *trans* -CH₂-CH=CH-CH₂- | CH₂CH₂CH₃ |
| 567. | Cyclopentyl | H | *trans* -CH₂-CH=CH-CH₂- | CH₂CH₂CH₃ |
| 568. | Cyclohexyl | H | *trans* -CH₂-CH=CH-CH₂- | CH₂CH₂CH₃ |
| 569. | H₃C-O-CH₂ | H | *trans* -CH₂-CH=CH-CH₂- | CH₂CH₂CH₃ |
| 570. | Oxan-4-yl | H | *trans* -CH₂-CH=CH-CH₂- | CH₂CH₂CH₃ |
| 571. | 1-Methylpiperidin-4-yl | H | *trans*-CH₂-CH=CH-CH₂- | CH₂CH₂CH₃ |
| 572. | 1-Methylpyrrol-2-yl | H | -(CH₂)₄- | CH₃ |
| 573. | 3-Pyridyl | H | -(CH₂)₄- | CH₃ |
| 574. | 3-Thienyl | H | -(CH₂)₄- | CH₃ |
| 575. | 4-Fluorphenyl | H | -(CH₂)₄- | CH₃ |
| 576. | 4-Pyridyl | H | -(CH₂)₄- | CH₃ |
| 577. | 3-Furyl | H | -(CH₂)₄- | CH₃ |
| 578. | 2-Furyl | H | -(CH₂)₄- | CH₃ |
| 579. | 2-Pyrrolyl | H | -(CH₂)₄- | CH₃ |
| 580. | 2-Thienyl | H | -(CH₂)₄- | CH₃ |
| 581. | Pyridazin-2-yl | H | -(CH₂)₄- | CH₃ |
| 582. | 4-Methylthiazol-5-yl | H | -(CH₂)₄- | CH₃ |
| 583. | 2-Methyloxazol-4-yl | H | -(CH₂)₄- | CH₃ |
| 584. | Cyclopropyl | H | -(CH₂)₄- | CH₃ |
| 585. | Cyclobutyl | H | -(CH₂)₄- | CH₃ |
| 586. | Cyclopentyl | H | -(CH₂)₄- | CH₃ |
| 587. | Cyclohexyl | H | -(CH₂)₄- | CH₃ |
| 588. | H₃C-O-CH₂ | H | -(CH₂)₄- | CH₃ |
| 589. | Oxan-4-yl | H | -(CH₂)₄- | CH₃ |
| 590. | 1-Methylpiperidin-4-yl | H | -(CH₂)₄- | CH₃ |
| 591. | 1-Methylpyrrol-2-yl | H | *trans* -CH₂CH=CH-CH₂- | CH₃ |
| 592. | 3-Pyridyl | H | *trans* -CH₂-CH=CH-CH₂- | CH₃ |
| 593. | 3-Thienyl | H | *trans* -CH₂-CH=CH-CH₂- | CH₃ |
| 594. | 4-Fluorphenyl | H | *trans* -CH₂-CH=CH-CH₂- | CH₃ |
| 595. | 4-Pyridyl | H | *trans* -CH₂-CH=CH-CH₂- | CH₃ |
| 596. | 3-Furyl | H | *trans* -CH₂-CH=CH-CH₂- | CH₃ |
| 597. | 2-Furyl | H | *trans* -CH₂-CH=CH-CH₂- | CH₃ |
| 598. | 2-Pyrrolyl | H | *trans* -CH₂-CH=CH-CH₂- | CH₃ |
| 599. | 2-Thienyl | H | *trans* -CH₂-CH=CH-CH₂- | CH₃ |
| 600. | Pyridazin-2-yl | H | *trans* -CH₂-CH=CH-CH₂- | CH₃ |
| 601. | 4-Methylthiazol-5-yl | H | *trans* -CH₂-CH=CH-CH₂- | CH₃ |
| 602. | 2-Methyloxazol-4-yl | H | *trans* -CH₂-CH=CH-CH₂- | CH₃ |
| 603. | Cyclopropyl | H | *trans* -CH₂-CH=CH-CH₂- | CH₃ |
| 604. | Cyclobutyl | H | *trans*-CH₂-CH=CH-CH₂- | CH₃ |
| 605. | Cyclopentyl | H | *trans*-CH₂-CH=CH-CH₂- | CH₃ |
| 606. | Cyclohexyl | H | *trans*-CH₂-CH=CH-CH₂- | CH₃ |
| 607. | H₃C-O-CH₂ | H | *trans* -CH₂-CH=CH-CH₂- | CH₃ |
| 608. | Oxan-4-yl | H | *trans* -CH₂-CH=CH-CH₂- | CH₃ |
| 609. | 1-Methylpiperidin-4-yl | H | *trans* -CH-₂CH=CH-CH₂- | CH₃ |
| 610. | 1-Methylpyrrol-2-yl | H | -(CH₂)₄- | C(CH₃)₃ |
| 611. | 3-Pyridyl | H | -(CH₂)₄- | C(CH₃)₃ |
| 612. | 3-Thienyl | H | -(CH₂)₄- | C(CH₃)₃ |
| 613. | 4-Fluorphenyl | H | -(CH₂)₄- | C(CH₃)₃ |
| 614. | 4-Pyridyl | H | -(CH₂)₄- | C(CH₃)₃ |
| 615. | 3-Furyl | H | -(CH₂)₄- | C(CH₃)₃ |
| 616. | 2-Furyl | H | -(CH₂)₄- | C(CH₃)₃ |
| 617. | 2-Pyrrolyl | H | -(CH₂)₄- | C(CH₃)₃ |
| 618. | 2-Thienyl | H | -(CH₂)₄- | C(CH₃)₃ |
| 619. | Pyridazin-2-yl | H | -(CH₂)₄- | C(CH₃)₃ |
| 620. | 4-Methylthiazol-5-yl | H | -(CH₂)₄- | C(CH₃)₃ |
| 621. | 2-Methyloxazol-4-yl | H | -(CH₂)₄- | C(CH₃)₃ |
| 622. | Cyclopropyl | H | -(CH₂)₄- | C(CH₃)₃ |
| 623. | Cyclobutyl | H | -(CH₂)₄- | C(CH₃)₃ |
| 624. | Cyclopentyl | H | -(CH₂)₄- | C(CH₃)₃ |
| 625. | Cyclohexyl | H | -(CH₂)₄- | C(CH₃)₃ |
| 626. | H₃C-O-CH₂ | H | -(CH₂)₄- | C(CH₃)₃ |
| 627. | Oxan-4-yl | H | -(CH₂)₄- | C(CH₃)₃ |
| 628. | 1-Methylpiperidin-4-yl | H | -(CH₂)₄- | C(CH₃)₃ |
| 629. | 1-Methylpyrrol-2-yl | H | *trans* -CH₂-CH=CH-CH₂- | C(CH₃)₃ |
| 630. | 3-Pyridyl | H | *trans* -CH₂-CH=CH-CH₂- | C(CH₃)₃ |
| 631. | 3-Thienyl | H | *trans* -CH₂-CH=CH-CH₂ | C(CH₃)₃ |
| 632. | 4-Fluorphenyl | H | *trans* -CH₂-CH=CH-CH₂- | C(CH₃)₃ |
| 633. | 4-Pyridyl | H | *trans* -CH₂-CH=CH-CH₂- | C(CH₃)₃ |
| 634. | 3-Furyl | H | *trans* -CH₂-CH=CH-CH₂- | C(CH₃)₃ |
| 635. | 2-Furyl | H | *trans* -CH₂-CH=CH-CH₂- | C(CH₃)₃ |
| 636. | 2-Pyrrolyl | H | *trans*-CH₂-CH=CH-CH₂- | C(CH₃)₃ |
| 637. | 2-Thienyl | H | *trans* -CH₂-CH=CH-CH₂- | C(CH₃)₃ |
| 638. | Pyridazin-2-yl | H | *trans* -CH₂-CH=CH-CH₂- | C(CH₃)₃ |
| 639. | 4-Methylthiazol-5-yl | H | *trans* -CH₂-CH=CH-CH₂- | C(CH₃)₃ |
| 640. | 2-Methyloxazol-4-yl | H | *trans* -CH₂-CH=CH-CH₂- | C(CH₃)₃ |
| 641. | Cyclopropyl | H | *trans* -CH₂-CH=CH-CH₂- | C(CH₃)₃ |
| 642. | Cyclobutyl | H | *trans* -CH₂-CH=CH-CH₂- | C(CH₃)₃ |
| 643. | Cyclopentyl | H | *trans* -CH₂CH=CH-CH₂- | C(CH₃)₃ |
| 644. | Cyclohexyl | H | *trans* -CH₂-CH=CH-CH₂- | C(CH₃)₃ |
| 645. | H₃C-O-CH₂ | H | *trans* -CH₂-CH=CH-CH₂- | C(CH₃)₃ |
| 646. | Oxan-4-yl | H | *trans* -CH₂-CH=CH-CH₂- | C(CH₃)₃ |
| 647. | 1-Methylpiperidin-4-yl | H | *trans* -CH₂-CH=CH-CH₂- | C(CH₃)₃ |
| 648. | 1-Methylpyrrol-2-yl | H | -(CH₂)₄- | *cyclo*-C₅H₉ |
| 649. | 3-Pyridyl | H | -(CH₂)₄- | *cyclo*-C₅H₉ |
| 650. | 3-Thienyl | H | -(CH₂)₄- | *cyclo*-C₅H₉ |
| 651. | 4-Fluorphenyl | H | -(CH₂)₄- | *cyclo*-C₅H₉ |
| 652. | 4-Pyridyl | H | -(CH₂)₄- | *cyclo*-C₅H₉ |
| 653. | 3-Furyl | H | -(CH₂)₄- | *cyclo*-C₅H₉ |
| 654. | 2-Furyl | H | -(CH₂)₄- | *cyclo*-C₅H₉ |
| 655. | 2-Pyrrolyl | H | -(CH₂)₄- | *cyclo*-C₅H₉ |
| 656. | 2-Thienyl | H | -(CH₂)₄- | *cyclo*-C₅H₉ |
| 657. | Pyridazin-2-yl | H | -(CH₂)₄- | *cyclo*-C₅H₉ |
| 658. | 4-Methylthiazol-5-yl | H | -(CH₂)₄- | *cyclo*-C₅H₉ |
| 659. | 2-Methyloxazol-4-yl | H | -(CH₂)₄- | *cyclo*-C₅H₉ |
| 660. | Cyclopropyl | H | -(CH₂)₄- | *cyclo*-C₅H₉ |
| 661. | Cyclobutyl | H | -(CH₂)₄- | *cyclo*-C₅H₉ |
| 662. | Cyclopentyl | H | -(CH₂)₄- | *cyclo-*C₅H₉ |
| 663. | Cyclohexyl | H | -(CH₂)₄- | *cyclo*-C₅H₉ |
| 664. | H₃C-O-CH₂ | H | -(CH₂)₄- | *cyclo*-C₅H₉ |
| 665. | Oxan-4-yl | H | -(CH₂)₄- | *cyclo*-C₅H₉ |
| 666. | 1-Methylpiperidin-4-yl | H | -(CH₂)₄- | *cyclo*-C₅H₉ |
| 667. | 1-Methylpyrrol-2-yl | H | *trans*-CH₂-CH=CH-CH₂- | *cyclo*-C₅H₉ |
| 668. | 3-Pyridyl | H | *trans* -CH₂-CH=CH-CH₂- | *cyclo*-C₅H₉ |
| 669. | 3-Thienyl | H | *trans* -CH₂-CH=CH-CH₂- | *cyclo*-C₅H₉ |
| 670. | 4-Fluorphenyl | H | *trans* -CH₂CH=CH-CH₂ | *cyclo*-C₅H₉ |
| 671. | 4-Pyridyl | H | *trans* -CH₂-CH=CH-CH₂- | *cyclo*-C₅H₉ |
| 672. | 3-Furyl | H | *trans*-CH₂-CH=CH-CH₂- | *cyclo*-C₅H₉ |
| 673. | 2-Furyl | H | *trans* -CH₂-CH=CH-CH₂- | *cyclo*-C₅H₉ |
| 674. | 2-Pyrrolyl | H | *trans* -CH₂-CH=CH-CH₂- | *cyclo*-C₅H₉ |
| 675. | 2-Thienyl | H | *trans* -CH₂-CH=CH-CH₂- | *cyclo-*C₅H₉ |
| 676. | Pyridazin-2-yl | H | *trans* -CH₂-CH=CH-CH₂- | *cyclo*-C₅H₉ |
| 677. | 4-Methylthiazol-5-yl | H | *trans* -CH₂-CH=CH-CH₂- | *cyclo*-C₅H₉ |
| 678. | 2-Methyloxazol-4-yl | H | *trans* -CH₂-CH=CH-CH₂- | *cyclo*-C₅H₉ |
| 679. | Cyclopropyl | H | *trans* -CH₂-CH=CH-CH₂- | *cyclo*-C₅H₉ |
| 680. | Cyclobutyl | H | *trans* -CH₂-CH=CH-CH₂- | *cyclo*-C₅H₉ |
| 681. | Cyclopentyl | H | *trans* -CH₂-CH=CH-CH₂- | *cyclo*-C₅H₉ |
| 682. | Cyclohexyl | H | *trans* -CH₂-CH=CH-CH₂- | *cyclo*-C₅H₉ |
| 683. | H₃C-O-CH₂ | H | *trans* -CH₂-CH=CH-CH₂- | *cyclo*-C₅H₉ |
| 684. | Oxan-4-yl | H | *trans* -CH₂-CH=CH-CH₂- | *cyclo-*C₅H₉ |
| 685. | 1-Methylpiperidin-4-yl | H | *trans* -CH₂-CH=CH-CH₂- | *cyclo*-C₅H₉ |
| 686. | OH | H | -(CH₂)₄- | *cyclo*-C₃H₅ |
| 687. | OH | CH₃ | -(CH₂)₄- | *cyclo*-C₃H₅ |
| 688. | CH₃ | H | -(CH₂)₄- | *cyclo*-C₃H₅ |
| 689. | C(CH₃)₃ | H | -(CH₂)₄- | *cyclo*-C₃H₅ |
| 690. | C₆H₅ | H | -(CH₂)₄- | *cyclo*-C₃H₅ |
| 691. | C₆H₅ | CH₃ | -(CH₂)₄- | *cyclo*-C₃H₅ |
| 692. | CF₃ | H | -(CH₂)₄- | *cyclo*-C₃H₅ |
| 693. | OH | F | -(CH₂)₄- | *cyclo*-C₃H₅ |
| 694. | OH | CN | -(CH₂)₄- | *cyclo*-C₃H₅ |
| 695. | N(CH₃)₂ | H | -(CH₂)₄- | *cyclo*-C₃H₅ |
| 696. | N(CH₃)₂ | CH₃ | -(CH₂)₄- | *cyclo*-C₃H₅ |
| 697. | OH | H | *trans* -CH₂-CH=CH-CH₂- | *cyclo*-C₃H₅ |
| 698. | OH | CH₃ | *trans* -CH₂-CH=CH-CH₂- | *cyclo*-C₃H₅ |
| 699. | CH₃ | H | *trans* -CH₂-CH=CH-CH₂- | *cyclo*-C₃H₅ |
| 700. | C(CH₃)₃ | H | *trans* -CH₂-CH=CH-CH₂- | *cyclo*-C₃H₅ |
| 701. | C₆H₅ | H | *trans* -CH₂-CH=CH-CH₂- | *cyclo*-C₃H₅ |
| 702. | C₆H₅ | CH₃ | *trans* -CH₂-CH=CH-CH₂- | *cyclo*-C₃H₅ |
| 703. | CF₃ | H | *trans*-CH₂-CH=CH-CH₂- | *cyclo*-C₃H₅ |
| 704. | OH | F | *trans* -CH₂-CH=CH-CH₂- | *cyclo*-C₃H₅ |
| 705. | OH | CN | *trans* -CH₂-CH=CH-CH₂- | *cyclo*-C₃H₅ |
| 706. | N(CH₃)₂ | H | *trans* -CH₂-CH=CH-CH₂- | *cyclo*-C₃H₅ |
| 707. | N(CH₃)₂ | CH₃ | *trans* -CH₂-CH=CH-CH₂- | *cyclo*-C₃H₅ |
| 708. | OH | H | *trans*-CH₂-C(CH₃)=CH-CH₂- | *cyclo*-C₃H₅ |
| 709. | OH | CH₃ | *trans*-CH₂-C(CH₃)=CH-CH₂- | *cyclo*-C₃H₅ |
| 710. | CH₃ | H | *trans* -CH₂-C(CH₃)=CH-CH₂- | *cyclo*-C₃H₅ |
| 711. | C(CH₃)₃ | H | *trans* -CH₂-C(CH₃)=CH-CH₂- | *cyclo*-C₃H₅ |
| 712. | C₆H₅ | H | *trans* -CH₂-C(CH₃)=CH-CH₂- | *cyclo*-C₃H₅ |
| 713. | C₆H₅ | CH₃ | *trans*-CH₂-C(CH₃)=CH-CH₂- | *cyclo*-C₃H₅ |
| 714. | CF₃ | H | *trans* -CH₂-C(CH₃)=CH-CH₂- | *cyclo*-C₃H₅ |
| 715. | OH | F | *trans*-CH₂-C(CH₃)=CH-CH₂- | *cyclo*-C₃H₅ |
| 716. | OH | CN | *trans*-CH₂-C(CH₃)=CH-CH₂- | *cyclo*-C₃H₅ |
| 717. | N(CH₃)₂ | H | *trans*-CH₂-C(CH₃)=CH-CH₂- | *cyclo*-C₃H₅ |
| 718. | N(CH₃)₂ | CH₃ | *trans*-CH₂-C(CH₃)=CH-CH₂- | *cyclo*-C₃H₅ |
| 719. | OH | H | -CH₂-CH(CH₃)-CH₂-CH₂- | *cyclo*-C₃H₅ |
| 720. | OH | CH₃ | -CH₂-CH(CH₃)-CH₂-CH₂- | *cyclo*-C₃H₅ |
| 721. | CH₃ | H | -CH₂-CH(CH₃)-CH₂-CH₂- | *cyclo*-C₃H₅ |
| 722. | C(CH₃)₃ | H | -CH₂-CH(CH₃)-CH₂-CH₂- | *cyclo*-C₃H₅ |
| 723. | C₆H₅ | H | -CH₂-CH(CH₃)-CH₂-CH₂- | *cyclo*-C₃H₅ |
| 724. | C₆H₅ | CH₃ | -CH₂-CH(CH₃)-CH₂-CH₂- | *cyclo*-C₃H₅ |
| 725. | CF₃ | H | -CH₂-CH(CH₃)-CH₂-CH₂- | *cyclo*-C₃H₅ |
| 726. | OH | F | -CH₂-CH(CH₃)-CH₂-CH₂- | *cyclo-*C₃H₅ |
| 727. | OH | CN | -CH₂-CH(CH₃)-CH₂-CH₂- | *cyclo*-C₃H₅ |
| 728. | N(CH₃)₂ | H | -CH₂-CH(CH₃)-CH₂-CH₂- | *cyclo*-C₃H₅ |
| 729. | N(CH₃)₂ | CH₃ | -CH₂-CH(CH₃)-CH₂-CH₂- | *cyclo*-C₃H₅ |
| 730. | OH | H | -CH₂-CH₂-CH₂-CH(CH₃)- | *cyclo*-C₃H₅ |
| 731. | OH | CH₃ | -CH₂-CH₂-CH₂-CH(CH₃)- | *cyclo-*C₃H₅ |
| 732. | CH₃ | H | -CH₂-CH₂-CH₂-CH(CH₃)- | *cyclo*-C₃H₅ |
| 733. | C(CH₃)₃ | H | -CH₂-CH₂-CH₂-CH(CH₃)- | *cyclo-*C₃H*₅* |
| 734. | C₆H₅ | H | -CH₂-CH₂-CH₂-CH(CH₃)- | *cyclo*-C₃H₅ |
| 735. | C₆H₅ | CH₃ | -CH₂-CH₂-CH₂-CH(CH₃)- | *cyclo-*C₃H₅ |
| 736. | CF₃ | H | -CH₂-CH₂-CH₂-CH(CH₃)- | *cyclo*-C₃H₅ |
| 737. | OH | F | -CH₂-CH₂-CH₂-CH(CH₃)- | *cyclo*-C₃H₅ |
| 738. | OH | CN | -CH₂-CH₂-CH₂-CH(CH₃)- | *cyclo*-C₃H₅ |
| 739. | N(CH₃)₂ | H | -CH₂-CH₂-CH₂-CH(CH₃)- | *cyclo*-C₃H₅ |
| 740. | N(CH₃)₂ | CH₃ | -CH₂-CH₂-CH₂-CH(CH₃)- | *cyclo*-C₃H₅ |
| 741. | 1-Methylpyrrol-2-yl | H | -(CH₂)₄- | *cyclo*-C₃H₅ |
| 742. | 3-Pyridyl | H | -(CH₂)₄- | *cyclo*-C₃H₅ |
| 743. | 3-Thienyl | H | -(CH₂)₄- | *cyclo*-C₃H₅ |
| 744. | 4-Fluorphenyl | H | -(CH₂)₄- | *cyclo*-C₃H₅ |
| 745. | 4-Pyridyl | H | -(CH₂)₄- | *cyclo*-C₃H₅ |
| 746. | 3-Furyl | H | -(CH₂)₄- | *cyclo*-C₃H₅ |
| 747. | 2-Furyl | H | -(CH₂)₄- | *cyclo*-C₃H₅ |
| 748. | 2-Pyrrolyl | H | -(CH₂)₄- | *cyclo*-C₃H₅ |
| 749. | 2-Thienyl | H | -(CH₂)₄- | *cyclo*-C₃H₅ |
| 750. | Pyridazin-2-yl | H | -(CH₂)₄- | *cyclo*-C₃H₅ |
| 751. | 4-Methylthiazol-5-yl | H | -(CH₂)₄- | *cyclo*-C₃H₅ |
| 752. | 2-Methytoxazol-4-yl | H | -(CH₂)₄- | *cyclo*-C₃H₅ |
| 753. | Cyclopropyl | H | -(CH₂)₄- | *cyclo-*C₃H₅ |
| 754. | Cyclobutyl | H | -(CH₂)₄- | *cyclo*-C₃H₅ |
| 755. | Cyclopentyl | H | -(CH₂)₄- | *cyclo*-C₃H₅ |
| 756. | Cyclohexyl | H | -(CH₂)₄- | *cyclo*-C₃H₅ |
| 757. | H₃C-O-CH₂ | H | -(CH₂)₄- | *cyclo*-C₃H₅ |
| 758. | Oxan-4-yl | H | -(CH₂)₄- | *cyclo*-C₃H₅ |
| 759. | 1-Methylpiperidin-4-yl | H | -(CH₂)₄- | *cyclo*-C₃H₅ |
| 760. | 1-Methylpyrrol-2-yl | H | *trans* -CH₂-CH=CH-CH₂- | *cyclo*-C₃H₅ |
| 761. | 3-Pyridyl | H | *trans*-CH₂-CH=CH-CH₂- | *cyclo*-C₃H₅ |
| 762. | 3-Thienyl | H | *trans* -CH₂-CH=CH-CH₂- | *cyclo*-C₃H₅ |
| 763. | 4-Fluorphenyl | H | *trans*-CH₂-CH=CH-CH₂- | *cyclo*-C₃H₅ |
| 764. | 4-Pyridyl | H | *trans* -CH₂-CH=CH-CH₂- | *cyclo*-C₃H₅ |
| 765. | 3-Furyl | H | *trans*-CH₂-CH=CH-CH₂- | *cyclo-*C₃H₅ |
| 766. | 2-Furyl | H | *trans* -CH₂-CH=CH-CH₂- | *cyclo*-C₃H₅ |
| 767. | 2-Pyrrolyl | H | *trans* -CH₂-CH=CH-CH₂- | *cyclo*-C₃H₅ |
| 768. | 2-Thienyl | H | *trans* -CH₂-CH=CH-CH₂- | *cyclo*-C₃H₅ |
| 769. | Pyridazin-2-yl | H | *trans-*CH₂-CH=CH-CH₂- | *cyclo*-C₃H₅ |
| 770. | 4-Methylthiazol-5-yl | H | *trans*-CH₂-CH=CH-CH₂- | *cyclo*-C₃H₅ |
| 771. | 2-Methyloxazol-4-yl | H | *trans*-CH₂-CH=CH-CH₂- | *cyclo*-C₃H₅ |
| 772. | Cyclopropyl | H | *trans* -CH₂-CH=CH-CH₂- | *cyclo*-C₃H₅ |
| 773. | Cyclobutyl | H | *trans* -CH₂-CH=CH-CH₂- | *cyclo*-C₃H₅ |
| 774. | Cyclopentyl | H | *trans* -CH₂-CH=CH-CH₂- | *cyclo*-C₃H₅ |
| 775. | Cyclohexyl | H | *trans* -CH₂-CH=CH-CH₂ | *cyclo*-C₃H₅ |
| 776. | H₃C-O-CH₂ | H | *trans* -CH₂-CH=CH-CH₂- | *cyclo*-C₃H₅ |
| 777. | Oxan-4-yl | H | *trans* -CH₂-CH=CH-CH₂- | *cyclo*-C₃H₅ |
| 778. | 1-Methylpiperidin-4-yl | H | *trans*-CH₂-CH=CH-CH₂- | *cyclo*-C₃H₅ |
| 779. | OH | H | -(CH₂)₄- | *cyclo-*C₄H₇ |
| 780. | OH | CH₃ | -(CH₂)₄- | *cyclo*-C₄H₇ |
| 781. | CH₃ | H | -(CH₂)₄- | *cyclo*-C₄H₇ |
| 782. | C(CH₃)₃ | H | -(CH₂)₄- | *cyclo*-C₄H₇ |
| 783. | C₆H₅ | H | -(CH₂)₄- | *cyclo*-C₄H₇ |
| 784. | C₆H₅ | CH₃ | -(CH₂)₄- | *cyclo-*C₄H₇ |
| 785. | CF₃ | H | -(CH₂)₄- | *cyclo*-C₄H₇ |
| 786. | OH | F | -(CH₂)₄- | *cyclo*-C₄H₇ |
| 787. | OH | CN | -(CH₂)₄- | *cyclo*-C₄H₇ |
| 788. | N(CH₃)₂ | H | -(CH₂)₄- | *cyclo*-C₄H₇ |
| 789. | N(CH₃)₂ | CH₃ | -(CH₂)₄- | *cyclo*-C₄H₇ |
| 790. | OH | H | *trans* -CH₂CH=CH-CH₂- | *cyclo*-C₄H₇ |
| 791. | OH | CH₃ | *trans* -CH₂-CH=CH-CH₂- | *cyclo*-C₄H₇ |
| 792. | CH₃ | H | *trans* -CH₂-CH=CH-CH₂- | *cyclo*-C₄H₇ |
| 793. | C(CH₃)₃ | H | *trans* -CH₂CH=CH-CH₂- | *cyclo*-C₄H₇ |
| 794. | C₆H₅ | H | trans -CH₂-CH=CH-CH₂- | *cyclo*-C₄H₇ |
| 795. | C₆H₅ | CH₃ | *trans*-CH₂-CH=CH-CH₂- | *cyclo*-C₄H₇ |
| 796. | CF₃ | H | *trans* -CH₂CH=CH-CH₂- | *cyclo*-C₄H₇ |
| 797. | OH | F | *trans* -CH₂-CH=CH-CH₂- | *cyclo-*C₄H₇ |
| 798. | OH | CN | *trans* -CH₂-CH=CH-CH₂- | *cyclo*-C₄H₇ |
| 799. | N(CH₃)₂ | H | *trans* -CH₂CH=CH-CH₂ | *cyclo*-C₄H₇ |
| 800. | N(CH₃)₂ | CH₃ | *trans* -CH₂-CH=CH-CH₂- | *cyclo*-C₄H₇ |
| 801. | OH | H | *trans* -CH₂C(CH₃)=CH-CH₂- | *cyclo*-C₄H₇ |
| 802. | OH | CH₃ | *trans* -CH₂-C(CH₃)=CH-CH₂- | *cyclo*-C₄H₇ |
| 803. | CH₃ | H | *trans* -CH₂-C(CH₃)=CH-CH₂- | *cyclo*-C₄H₇ |
| 804. | C(CH₃)₃ | H | *trans* -CH₂-C(CH₃)=CH-CH₂- | *cyclo*-C₄H₇ |
| 805. | C₆H₅ | H | *trans* -CH₂-C(CH₃)=CH-CH₂- | *cyclo*-C₄H₇ |
| 806. | C₆H₅ | CH₃ | *trans* -CH₂-C(CH₃)=CH-CH₂- | *cyclo*-C₄H₇ |
| 807. | CF₃ | H | *trans* -CH₂-C(CH₃)=CH-CH₂- | *cyclo*-C₄H₇ |
| 808. | OH | F | *trans* -CH₂-C(CH₃)=CH-CH₂- | *cyclo*-C₄H₇ |
| 809. | OH | CN | *trans* -CH₂-C(CH₃)=CH-CH₂- | *cyclo-*C₄H₇ |
| 810. | N(CH₃)₂ | H | *trans* -CH₂-C(CH₃)=CH-CH₂- | *cyclo*-C₄H₇ |
| 811. | N(CH₃)₂ | CH₃ | *trans* -CH₂-C(CH₃)=CH-CH₂- | *cyclo*-C₄H₇ |
| 812. | OH | H | -CH₂-CH(CH₃)-CH₂-CH₂- | *cyclo*-C₄H₇ |
| 813. | OH | CH₃ | -CH₂-CH(CH₃)-CH₂-CH₂- | *cyclo*-C₄H₇ |
| 814. | CH₃ | H | -CH₂-CH(CH₃)-CH₂-CH₂- | *cyclo*-C₄H₇ |
| 815. | C(CH₃)₃ | H | -CH₂-CH(CH₃)-CH₂-CH₂- | *cyclo*-C₄H₇ |
| 816. | C₆H₅ | H | -CH₂-CH(CH₃)-CH₂-CH₂- | *cyclo*-C₄H₇ |
| 817. | C₆H₅ | CH₃ | -CH₂-CH(CH₃)-CH₂-CH₂- | *cyclo*-C₄H₇ |
| 818. | CF₃ | H | -CH₂-CH(CH₃)-CH₂-CH₂- | *cyclo*-C₄H₇ |
| 819. | OH | F | -CH₂-CH(CH₃)-CH₂-CH₂- | *cyclo*-C₄H₇ |
| 820. | OH | CN | -CH₂-CH(CH₃)-CH₂-CH₂- | *cyclo*-C₄H₇ |
| 821. | N(CH₃)₂ | H | -CH₂-CH(CH₃)-CH₂-CH₂- | *cyclo*-C₄H₇ |
| 822. | N(CH₃)₂ | CH₃ | -CH₂-CH(CH₃)-CH₂-CH₂- | *cyclo*-C₄H₇ |
| 823. | OH | H | -CH₂-CH₂-CH₂-CH(CH₃)- | *cyclo*-C₄H₇ |
| 824. | OH | CH₃ | -CH₂-CH₂-CH₂-CH(CH₃)- | *cyclo*-C₄H₇ |
| 825. | CH₃ | H | -CH₂-CH₂-CH₂-CH(CH₃)- | *cyclo*-C₄H₇ |
| 826. | C(CH₃)₃ | H | -CH₂-CH₂-CH₂-CH(CH₃)- | *cyclo*-C₄H₇ |
| 827. | C₆H₅ | H | -CH₂-CH₂-CH₂-CH(CH₃)- | *cyclo*-C₄H₇ |
| 828. | C₆H₅ | CH₃ | -CH₂-CH_{z}-CH₂-CH(CH₃)- | *cyclo*-C₄H₇ |
| 829. | CF₃ | H | -CH₂-CH₂-CH₂-CH(CH₃)- | *cyclo*-C₄H₇ |
| 830. | OH | F | -CH₂-CH₂-CH₂-CH(CH₃)- | *cyclo*-C₄H₇ |
| 831. | OH | CN | -CH₂-CH₂-CH₂-CH(CH₃)- | *cyclo*-C₄H₇ |
| 832. | N(CH₃)₂ | H | -CH₂-CH₂-CH₂-CH(CH₃)- | *cyclo*-C₄H₇ |
| 833. | N(CH₃)₂ | CH₃ | -CH₂-CH₂-CH₂-CH(CH₃)- | *cyclo*-C₄H₇ |
| 834. | 1-Methylpyrrol-2-yl | H | -(CH₂)₄- | *cyclo*-C₄H₇ |
| 835. | 3-Pyridyl | H | -(CH₂)₄- | *cyclo*-C₄H₇ |
| 836. | 3-Thienyl | H | -(CH₂)₄- | *cyclo*-C₄H₇ |
| 837. | 4-Fluorphenyl | H | -(CH₂)₄- | *cyclo*-C₄H₇ |
| 838. | 4-Pyridyl | H | -(CH₂)₄- | *cyclo*-C₄H₇ |
| 839. | 3-Furyl | H | -(CH₂)₄- | *cyclo*-C₄H₇ |
| 840. | 2-Furyl | H | -(CH₂)₄- | *cyclo*-C₄H₇ |
| 841. | 2-Pyrrolyl | H | -(CH₂)₄- | *cyclo*-C₄H₇ |
| 842. | 2-Thienyl | H | -(CH₂)₄- | *cyclo*-C₄H₇ |
| 843. | Pyridazin-2-yl | H | -(CH₂)₄- | *cyclo*-C₄H₇ |
| 844. | 4-Methylthiazol-5-yl | H | -(CH₂)₄- | *cyclo*-C₄H₇ |
| 845. | 2-Methyloxazol-4-yl | H | -(CH₂)₄- | *cyclo*-C₄H₇ |
| 846. | Cyclopropyl | H | -(CH₂)₄- | *cyclo-*C₄H₇ |
| 847. | Cyclobutyl | H | -(CH₂)₄- | *cyclo*-C₄H₇ |
| 848. | Cyclopentyl | H | -(CH₂)₄- | *cyclo*-C₄H₇ |
| 849. | Cyclohexyl | H | -(CH₂)₄- | *cyclo*-C₄H₇ |
| 850. | H₃C-O-CH₂ | H | -(CH₂)₄- | *cyclo*-C₄H₇ |
| 851. | Oxan-4-yl | H | -(CH₂)₄- | *cyclo*-C₄H₇ |
| 852. | 1-Methylpiperidin-4-yl | H | -(CH₂)₄- | *cyclo*-C₄H₇ |
| 853. | 1-Methylpyrrol-2-yl | H | *trans* -CH₂-CH=CH-CH₂- | *cyclo*-C₄H₇ |
| 854. | 3-Pyridyl | H | *trans* -CH₂-CH=CH-CH₂- | *cyclo-*C₄H₇ |
| 855. | 3-Thienyl | H | *trans* -CH₂-CH=CH-CH₂- | *cyclo-*C₄H₇ |
| 856. | 4-Fluorphenyl | H | *trans* -CH₂-CH=CH-CH₂- | *cyclo-*C₄H₇ |
| 857. | 4-Pyridyl | H | *trans* -CH₂-CH=CH-CH₂- | *cyclo*-C₄H₇ |
| 858. | 3-Furyl | H | *trans* -CH₂-CH=CH-CH₂- | *cyclo*-C₄H₇ |
| 859. | 2-Furyl | H | *trans* -CH₂-CH=CH-CH₂- | *cyclo-*C₄H₇ |
| 860. | 2-Pyrrolyl | H | *trans*-CH₂-CH=CH-CH₂- | *cyclo-*C₄H₇ |
| 861. | 2-Thienyl | H | *trans*-CH₂-CH=CH-CH₂- | *cyclo*-C₄H₇ |
| 862. | Pyridazin-2-yl | H | *trans*-CH₂-CH=CH-CH₂- | *cyclo*-C₄H₇ |
| 863. | 4-Methylthiazol-5-yl | H | *trans*-CH₂-CH=CH-CH₂- | *cyclo-*C₄H₇ |
| 864. | 2-Methyloxazol-4-yl | H | *trans*-CH₂-CH=CH-CH₂- | *cyclo-*C₄H₇ |
| 865. | Cyclopropyl | H | *trans*-CH₂-CH=CH-CH₂- | *cyclo*-C₄H₇ |
| 866. | Cyclobutyl | H | *trans*-CH₂-CH=CH-CH₂- | *cyclo-*C₄H₇ |
| 867. | Cyclopentyl | H | *trans* -CH₂-CH=CH-CH₂- | *cyclo*-C₄H₇ |
| 868. | Cyclohexyl | H | *trans* -CH₂-CH=CH-CH₂- | *cyclo*-C₄H₇ |
| 869. | H₃C-O-CH₂ | H | *trans* -CH₂-CH=CH-CH₂- | *cyclo*-C₄H₇ |
| 870. | Oxan-4-yl | H | *trans*-CH₂-CH=CH-CH₂- | *cyclo*-C₄H₇ |
| 871. | 1-Methylpiperidin-4-yl | H | *trans* -CH₂-CH=CH-CH₂- | *cyclo-C₄H₇* |

Beispiele für weitere Verbindungen der allgemeinen Formel I.1 sind die Verbindungen der allgemeinen Formel I.1 b, 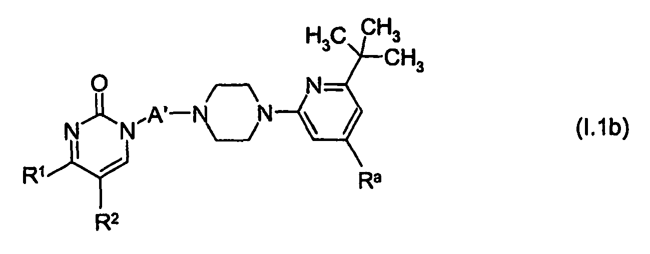 worin A', R^{a}, R¹ und R² die zuvor angegebenen Bedeutungen, insbesondere die als bevorzugt angegebenen Bedeutungen aufweisen. Beispiele für derartige Verbindungen sind die Verbindungen 1.1b.1 bis 1.1b.871, wobei die Variablen A', R^{a}, R¹ und R² jeweils gemeinsam die in einer der Zeilen 1 bis 647 der Tabelle 1 angegebene Bedeutung aufweisen.

Beispiele für weitere Verbindungen der allgemeinen Formel I.1 sind die Verbindungen der allgemeinen Formel I.1c, 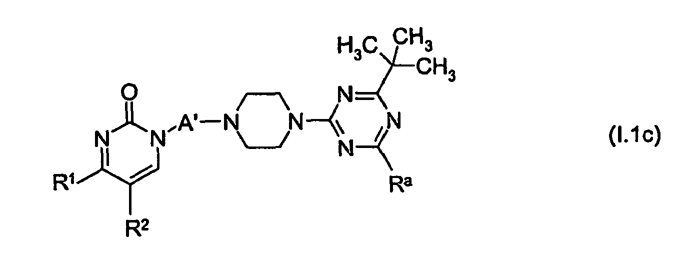 worin A', R^{a}, R¹ und R² die zuvor angegebenen Bedeutungen, insbesondere die als bevorzugt angegebenen Bedeutungen aufweisen. Beispiele für derartige Verbindungen sind die Verbindungen 1.1 c.1 bis 1.1 c.871, wobei die Variablen A', R^{a}, R¹ und R² jeweils gemeinsam die in einer der Zeilen 1 bis 871 der Tabelle 1 angegebene Bedeutung aufweisen.

Beispiele für weitere Verbindungen der allgemeinen Formel I.1 sind die Verbindungen der allgemeinen Formel I.1d, 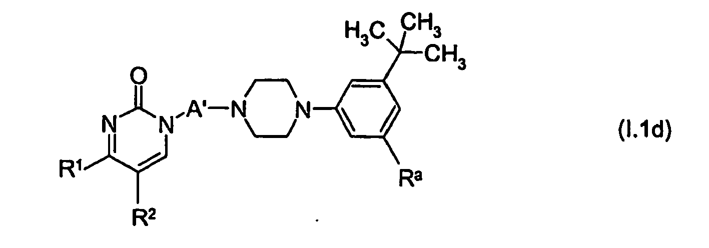 worin A', R^{a}, R¹ und R² die zuvor angegebenen Bedeutungen, insbesondere die als bevorzugt angegebenen Bedeutungen aufweisen. Beispiele für derartige Verbindungen sind die Verbindungen I.1d.1 bis I.1d.871, wobei die Variablen A', R^{a}, R¹ und R² jeweils gemeinsam die in einer der Zeilen 1 bis 871 der Tabelle 1 angegebene Bedeutung aufweisen.

Beispiele für weitere Verbindungen der allgemeinen Formel I.1 sind die Verbindungen der allgemeinen Formel I.1 e, 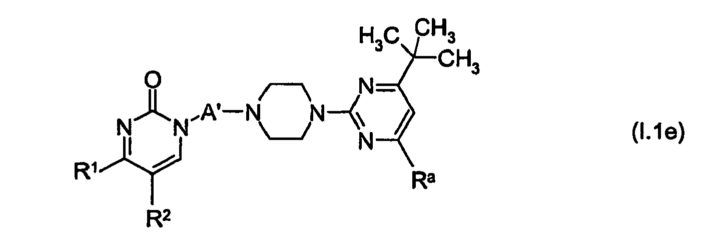 worin A', R^{a}, R¹ und R² die zuvor angegebenen Bedeutungen, insbesondere die als bevorzugt angegebenen Bedeutungen aufweisen. Beispiele für derartige Verbindungen sind die Verbindungen I.1e.1 bis I.1e.871, wobei die Variablen A', R^{a}, R¹ und R² jeweils gemeinsam die in einer der Zeilen 1 bis 871 der Tabelle 1 angegebene Bedeutung aufweisen.

Beispiele für weitere Verbindungen der allgemeinen Formel I.1 sind die Verbindungen der allgemeinen Formel I.1f, 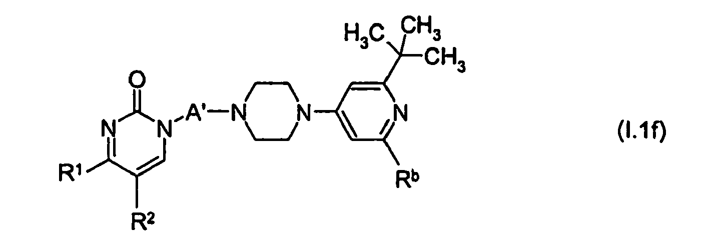 worin A', R^{a}, R¹ und R² die zuvor angegebenen Bedeutungen, insbesondere die als bevorzugt angegebenen Bedeutungen aufweisen. Beispiele für derartige Verbindungen sind die Verbindungen I.1f.1 bis I.1f.871, wobei die Variablen A', R^{a}, R¹ und R² jeweils gemeinsam die in einer der Zeilen 1 bis 871 der Tabelle 1 angegebene Bedeutung aufweisen.

Die Herstellung der erfindungsgemäßen Verbindungen I erfolgt analog zu literaturbekannten Methoden. Ein wichtiger Zugang zu den erfindungsgemäßen Verbindungen ist in Schema 1 dargestellt.

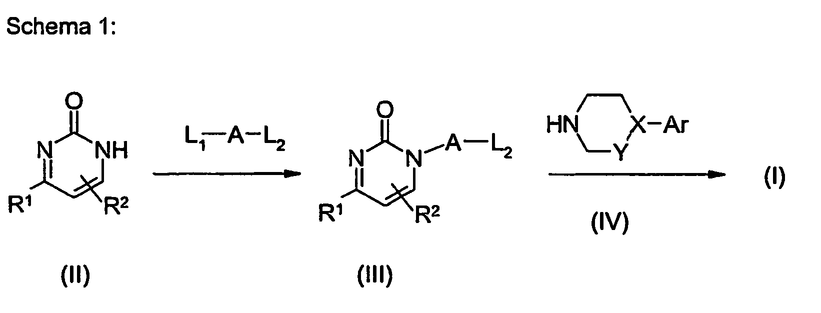

In Schema 1 haben R¹, R² , A, X, Y und Ar die zuvor genannten Bedeutungen. L₁ und L₂ stehen für nucleophil verdrängbare Abgangsgruppen. Beispiele für geeignete nucleophil verdrängbare Abgangsgruppen sind Halogen, insbesondere Chlor, Brom oder lod, Alkyl- und Arylsulfonat wie Mesylat, Tosylat. L₁ und L₂ sind vorzugsweise voneinander verschieden sind und weisen eine unterschiedliche Reaktivität auf. Beispielsweise steht L₁ für Brom oder lod und L₂ für Chlor. Die für die Umsetzung erforderlichen Reaktionsbedingungen entsprechen den für nucleophile Substitutionen üblichen Reaktionsbedingungen.

Verbindungen der allgemeinen Formel IV sind entweder literaturbekannt, z.B. aus WO 96/02519, WO 97/25324, WO 99/02503 oder der in diesen Schriften zitierten Literatur bekannt oder können nach den dort beschriebenen Verfahren hergestellt werden.

Die Pyrimidinon-Verbindungen der Formeln II sind bekannt und teilweise kommerziell erhältlich oder können nach bekannten Verfahren zur Pyrimidinonsynthese hergestellt werden, wie z. B. beschrieben in Austr. J. Chem. 1968, 221, S. 243-255; J. Med. Chem. 1978, 21, S. 623-628; Tetrahedron Lett. 1986, 27, S. 2611-2612; Chemiker Ztg. 1977, 6, S. 305. Die Verbindungen II können auch nach den in Schema 4 angegebenen Methoden hergestellt werden.

Außerdem können die Verbindungen der Formel II, sofern R¹ für gegebenenfalls substituiertes Alkenyl, gegebenenfalls substituiertes Phenyl oder gegebenenfalls substituiertes, C-gebundenes Heteroaryl steht, gemäß der in Schema 2 gezeigten Route via Suzuki-Kupplung hergestellt werden.

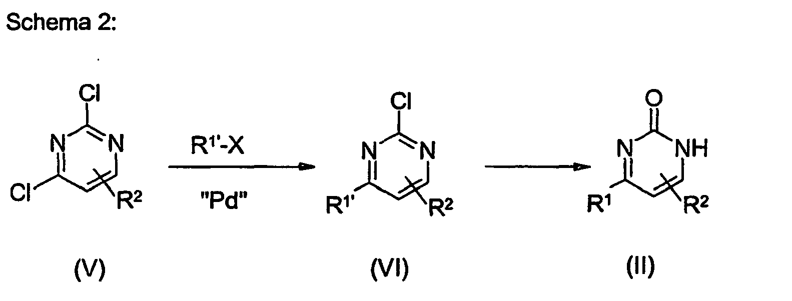

In Schema 2 steht R^{1'} für gegebenenfalls substituiertes Alkenyl, gegebenenfalls substituiertes Phenyl oder gegebenenfalls substituiertes, C-gebundenes Heteroaryl. X steht für eine Gruppe B(OH)₂, B(OR)₂ oder für den von dem entsprechenden Boronsäureanhydrid abgeleiteten Rest (BO)₃/3. "Pd" steht für einen Palladium(0)komplex, der vorzugsweise 4 Trialkylphosphin- oder Triarylphosphin-Liganden aufweist. R² hat die zuvor angegebenen Bedeutungen und steht insbesondere für Wasserstoff oder C₁-C₄-Alkyl.

Die Kupplung von V mit der Verbindung R^{1'}-X erfolgt unter den Bedingungen einer Suzuki-Kupplung (Übersicht siehe A. Suzuki et al. in Chem. Rev. 1995, 95, S. 2457-2483). Die für die Suzuki-Kupplung von 2,4-Dichlorpyrimidinen V mit R^{1'}-X erforderlichen Reaktionsbedingungen sind aus der Literatur bekannt, z.B. aus J. Org. Chem. 66(21) (2001), S. 7124-7128. Das dabei erhaltene 2-Chlorpyrimidin VI kann in an sich bekannter Weise, z.B. unter den in Acta Chem. Scand. B, 1984, 38, S. 505-508 angegebenen Bedingungen, in das entsprechende 2-Pyrimidinon II umgewandelt werden.

Ferner können die Verbindungen der Formel II, worin R¹ für gegebenenfalls substituiertes C₁-C₆-Alkyl oder C₃-C₆-Cycloalkyl, insbesondere für Ethyl, Isopropyl, Cyclopropyl, Cyclobutyl, Cyclohexyl, steht, und R² für H steht, beispielsweise nach dem im Schema 3 gezeigten Verfahren hergestellt werden.

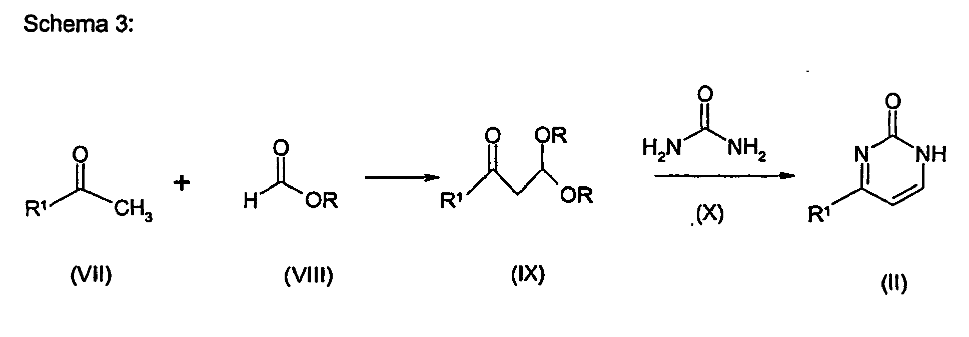

In Schema 3 steht R beispielsweise für C₁-C₄-Alkyl. In Schema 3 wird man zunächst ein Keton VII mit einem Ameisensäureester VIII, z. B. Ameisensäuremethylester, in an sich bekannter Weise in das Ketal IX überführen (siehe Helv. Chim. Acta 2002, 85, 2926-2929, Bsp. 6). Üblicherweise führt man die Umsetzung in Gegenwart einer Base wie einem Alkoholat in einem inerten Lösungsmittel wie einem Ether durch. Die Umsetzung des erhaltenen Ketals IX mit Harnstoff X unter Bildung des entsprechenden 2-Pyrimidinons II erfolgt unter literaturbekannten Bedingungen, z. B. gemäß Aust. J. Chem. 1968, 21, 243-55 (insbesondere S. 252).

Die Herstellung der 2-Pyrimidinone II, worin R¹ für Wasserstoff steht und R² für gegebenenfalls substituiertes Phenyl steht, kann beispielsweise nach dem im Schema 4 gezeigten Verfahren erfolgen.

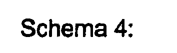

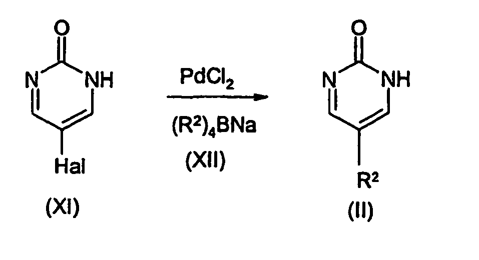

In Schema 4 steht Hal für Halogen, insbesondere für Brom oder Chlor. Die Kupplung des Halogenpyrimidinons XI mit dem Borat XII erfolgt unter Suzuki-Bedingungen (siehe Tetrahedron 1997, 53, 14437-50). Die modifizierte Suzuki-Kreuzkupplung zwischen dem Pyridinon XI und dem Borat XII erfolgt üblicherweise in wässrigen Lösungsmitteln in Gegenwart eines phosphinfreien Pd-Katalysators wie Palladium(II)-chlorid und in Gegenwart einer Base. Geeignete Basen sind beispielsweise Alkalimetallhydroxide wie Natriumhydroxid. Die Pyridinone XI und die Borate XII sind literaturbekannt.

Die Herstellung der erfindungsgemäßen Pyrimidinonverbindungen I, worin R¹ für SH steht, kann beispielsweise nach dem in Schema 5 gezeigten Verfahren erfolgen.

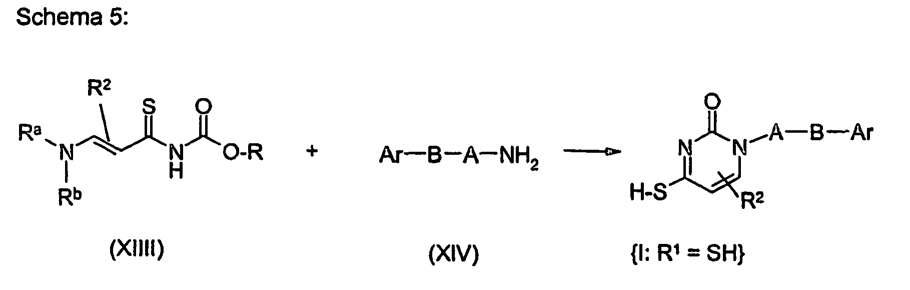

In Schema 5 stehen R^{a} und R^{b} beispielsweise beide für C₁-C₄-Alkyl oder bilden mit dem Stickstoffatom, an das sie gebunden sind einen gesättigten Ring, z.B. einen Piperidinyl-, Piperazinyl-, Pyrrolidinyl- oder Morpholinyl-Rest. R steht beispielsweise für C₁-C₄-Alkyl. Die in Schema 5 gezeigte Umsetzung ist prinzipiell bekannt, beispielsweise aus J. Hetercycl. Chem. 5 (1968) S. 837-844 oder aus WO 00/61579, und kann in analoger Weise zur Herstellung der erfindungsgemäßen Verbindungen I eingesetzt werden. Gleiches gilt für die Ausgangsverbindungen XIII. Die Verbindungen der Formel XIV sind beispielsweise aus dem Eingangs zitierten Stand der Technik bekannt oder können aus der entsprechenden Halogenverbindung Ar-B-A-Hal, worin Hal für Chlor, Brom oder lod steht, in an sich üblicher Weise hergestellt werden. Beispielsweise kann man das Amin XIV Ar-B-A-Hal durch Überführung in das entsprechende Azid Ar-B-AN₃ und anschließende Reduktion zum Amin herstellen. Die hierfür erforderlichen Bedingungen sind dem Fachmann bekannt, z.B. aus Chem. Rev. 1994, 94, S. 1, und können in analoger Weise auf die Herstellung von XIV angewendet werden. Die Halogenverbindungen Ar-B-A-Hal sind beispielsweise aus WO 96/02519, WO 97/25324, WO 99/09015, WO 99/02503 bekannt oder lassen sich in Analogie zu den dort beschriebenen Verfahren herstellen.

In den Verbindungen I mit R¹ = SH kann die Thiolgruppe nach Standardverfahren der organischen Chemie in andere Reste R¹ umgewandelt werden. Eine Übersicht gibt das Schema 6.

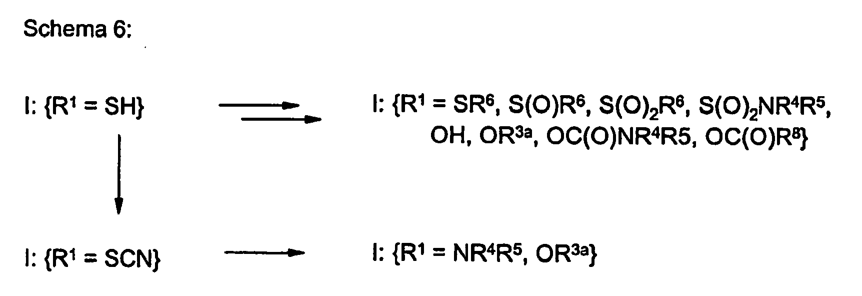

Verfahren hierzu sind dem Fachmann bekannt und umfassen die Umwandlung von SH in SR⁶ via Alkylierung, die Oxidation von SR⁶ zu den korrespondierenden Gruppen SOR⁶ und SO₂R⁶, den oxidativen Abbau von SH zu OH, mit gegebenenfalls sich anschließender Alkylierung oder Veresterung zu den Gruppen OR^{3a}, C)C(O)NR⁴R⁵ oder OC(O)R⁸.

Die Herstellung der erfindungsgemäßen Pyrimidinonverbindungen I.1, worin R¹ für NR⁴R⁵ steht, kann beispielsweise nach dem im Schema 7 gezeigten Verfahren erfolgen.

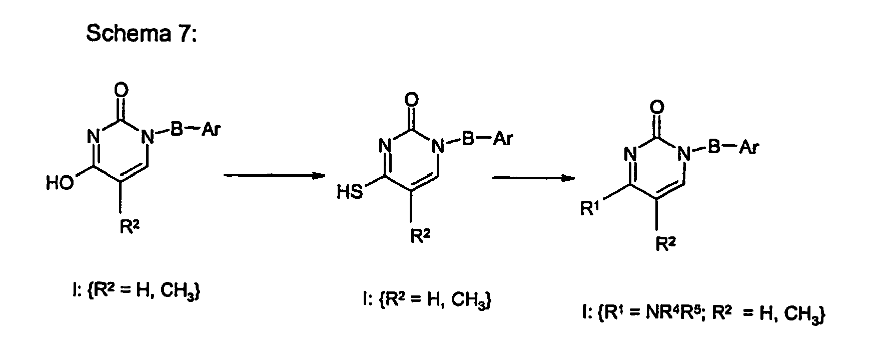

In Schema 7 haben B und Ar die zuvor genannten Bedeutungen. Gemäß Schema 7 wird man zunächst die Verbindung 1, worin R¹ für OH steht, in das entsprechende Thiol I mit R¹ = SH überführen. Beispiele für geeignete Schwefelungsmittel sind Organophosphorsulfide wie das Lawesson-Reagenz, Organozinnsulfide oder Phosphor(V)-sulfid. Ein bevorzugtes Schwefelungsmittel ist Phosphorpentasulfid (P₄S₁₀). Die für die Thionierung erforderlichen Bedingungen sind dem Fachmann bekannt, z. B. aus J. Med. Chem. 1984, 27, 1470-80 (insbesondere S. 1478, Beispiel 8b). Das hierbei erhaltene Thiol I mit R¹ = SH kann dann durch Umsetzung mit einer Verbindung der Formel HNR⁴R⁵, worin R⁴ und R⁶ die zuvor genannten Bedeutungen aufweisen, in andere Verbindungen I mit R¹ = NR⁴R⁵ überführt werden. Die Umsetzung erfolgt in der Regel in einem inerten Lösungsmittel. Die zur Umsetzung erforderliche Aktivierungsenergie kann mittels Mikrowellen in die Reaktionsmischung eingetragen werden (zur Umsetzung unter Einsatz von Mikrowellen, siehe Tetrahedron 2001, 57, S. 9199 ff, S. 9225 ff sowie allgemein "Microwaves in Organic Synthesis", André Loupy (Hrsg.), Wiley-VCH 2002).

Die Herstellung der Pyrimidinonverbindungen II, worin R¹ für NR⁴R⁵ steht, kann beispielsweise in Analogie zu obigem Schema 7 erfolgen. Die Herstellung ist in Schema 8 skizziert.

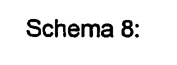

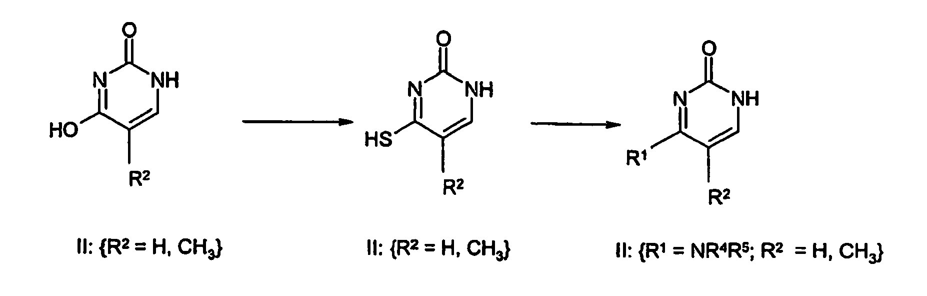

Die Herstellung der Tautomere la bzw. Ib kann in analoger Weise wie die hier beschriebene Herstellung der Verbindung I erfolgen. Beispielsweise kann man die Tautomere Ib nach dem in Schema 1 gezeigten Synteseweg herstellen. Außerdem kann man die Verbindung I in ihre Tautomere la mit Q = Halogen umwandeln, in dem man sie mit einem geeigneten Halogenierungsmittel wie PCl₃ oder POCl₃ behandelt.

Sofern nichts anderes angegeben wird, erfolgen die oben beschriebenen Umsetzungen im allgemeinen in einem Lösungsmittel bei Temperaturen zwischen Raumtemperatur und der Siedetemperatur des verwendeten Lösungsmittels. Brauchbare Lösungsmittel sind beispielsweise Ether, wie Diethylether, Diisopropylether, Methyl-tert.-butylether oder Tetrahydrofuran, Dimethylformamid, Dimethylsulfoxid, Dimethoxyethan, Toluol, Xylol, Acetonitril, Ketone, wie Aceton oder Methylethylketon, oder Alkohole, wie Methanol, Ethanol oder Butanol.

Gewünschtenfalls arbeitet man in Gegenwart einer Base zur Neutralisation von bei den Umsetzungen freiwerdenden Protonen. Geeignete Basen umfassen anorganische Basen, wie Natrium- oder Kaliumcarbonat, Natrium- oder Kaliumhydrogencarbonat, weiterhin Alkoholate wie Natriummethylat, Natriumethylat, Alkalimetallhydride wie Natriumhydrid, metallorganische Verbindungen, wie Butyllithium- oder Alkylmagnesium-Verbindungen, oder organische Stickstoffbasen, wie Triethylamin oder Pyridin. Letztere können gleichzeitig als Lösungsmittel dienen.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindungen kann in üblicher Weise erfolgen, beispielsweise durch Umkristallisieren aus einem Lösungsmittel, Chromatographie oder durch Überführen in eine Säureadditionssalz.

Die Säureadditionssalze werden in üblicher Weise durch Mischen der freien Base mit der entsprechenden Säure, gegebenenfalls in Lösung in einem organischen Lösungsmittel, beispielsweise einem niedermolekularen Alkohol, wie Methanol, Ethanol oder Propanol, einem Ether, wie Methyl-tert-butylether, Diisopropylether, einem Keton, wie Aceton oder Methylethylketon oder einem Ester, wie Essigsäureethylester, hergestellt. Bei den erfindungsgemäßen Verbindungen der Formel I handelt es sich um hochselektive Dopamin-D₃-Rezeptor-Liganden, die auf Grund ihrer geringen Affinität gegenüber anderen Rezeptoren wie D₁-Rezeptoren, D₄-Rezeptoren, α1- und/oder α2-adrenergen-Rezeptoren, muskarinergen Rezeptoren, histaminischen Rezeptoren, OpiatRezeptoren und insbesondere gegenüber Dopamin-D₂-Rezeptoren, nebenwirkungsärmer als die klassischen Neuroleptika sind, bei denen es sich um D₂-Rezeptor-Antagonisten handelt.

Die hohe Affinität der erfindungsgemäßen Verbindungen gegenüber D₃-Rezeptoren spiegelt sich in sehr geringen in vitro Kᵢ-Werte von in der Regel weniger als 100 nM (nmol/l) und vor allem von weniger als 50 nM wieder. Beispielsweise können Bindungsaffinitäten zu D₃-Rezeptoren in Rezeptorbindungsstudien über die Verdrängung von [¹²⁵I]-Iodosulprid bestimmt werden.

Erfindungsgemäß von besonderer Bedeutung sind Verbindungen deren Selektivität Kᵢ(D₂)/Kᵢ(D₃) vorzugsweise wenigstens 10, besser noch wenigstens 30 und besonders vorteilhaft wenigstens 50 beträgt. Rezeptorbindungsstudien an D₁-, D₂- und D₄-Rezeptoren können beispielsweise über die Verdrängung von [³H]SCH23390, [¹²⁵I]lodosulprid bzw. [¹²⁵I]Spiperon vorgenommen werden.

Die erfindungsgemäßen Verbindungen zeichnen sich außerdem durch eine geringere Hemmung der mitochondrialen Atmungskette aus, d.h. die mitochondriale Atmung wird durch die Verbindungen I erst bei vergleichsweise hohen Plasmaspiegeln gehemmt. Invitro beobachtet man für die erfindungsgemäßen Verbindungen eine Hemmung der mitochondrialen Atmung erst bei Konzentrationen > 50 µM, häufig > 100 µM und speziell bei Konzentrationen > 200 µM (IC₅₀-Werte).

Die erfindungsgemäßen Verbindungen zeichnen sich zudem durch eine vergleichsweise geringe Plasmaproteinbindung aus.

Die Verbindungen sind aufgrund ihres Bindungsprofils zur Behandlung von Erkrankungen brauchbar, die auf Dopamin-D₃-Liganden ansprechen, d. h. sie sind zur Behandlung von solchen Störungen bzw. Erkrankungen wirksam, bei denen eine Beeinflussung (Modulation) der Dopamin-D₃-Rezeptoren zur Verbesserung des Krankheitsbilds oder zum Ausheilen der Krankheit führt. Derartige Erkrankungen sind z. B. Störungen oder Erkrankungen des zentralen Nervensystems.

Unter Störungen bzw. Erkrankungen des zentralen Nervensystems versteht man Störungen, die Rückenmark und vor allem das Gehim betreffen. Der Begriff "Störung" im erfindungsgemäßen Sinne bezeichnet Anomalien, die in der Regel als krankhafte Zustände bzw. Funktionen angesehen werden und sich in Form bestimmter Anzeichen, Symptome und/oder Fehlfunktionen zu erkennen geben können. Die erfindungsgemäße Behandlung kann auf einzelne Störungen, d. h. Anomalien bzw. krankhafte Zustände gerichtet sein, es können aber auch mehrere, gegebenenfalls ursächlich miteinander verbundene Anomalien zu Mustern, d.h. Syndromen, zusammengefaßt sein, die erfindungsgemäß behandelt werden können.

Zu den erfindungsgemäß behandelbaren Störungen gehören vor allem psychiatrische und neurologische Störungen. Hierzu zählen insbesondere organische Störungen, symptomatische Störungen eingeschlossen, wie Psychosen vom akuten exogenen Reaktionstyp oder Begleit-Psychosen organischer bzw. exogener Ursache, z. B. bei Stoffwechselstörungen, Infektionen und Endokrinopathien; endogene Psychosen, wie Schizophrenie sowie schizotype und wahnhafte Störungen; affektive Störungen, wie Depressionen, Manie bzw. manisch-depressive Zustände; sowie Mischformen der zuvor geschilderten Störungen; neurotische und somatoforme Störungen sowie Störungen bei Belastung; dissoziative Störungen, z.B. Bewußtseinsausfälle, -eintrübungen und -spaltungen und Persönlichkeitsstörungen; Störungen von Aufmerksamkeit und

Wach/Schlafverhalten, wie Verhaltensstörungen und emotionale Störungen, deren Beginn in der Kindheit und Jugend liegt, z.B. Hyperaktivität bei Kindern, intellektuelle Defizite, insbesondere Aufmerksamkeitsstörungen (attention deficit disorders), Gedächtnis- und kognitive Störungen, z.B. Lern- und Gedächtnisschwäche (impaired cognitive function), Demenz, Narkolepsie und Schlafstörungen, z.B. restless legs syndrome; Entwicklungsstörungen; Angstzustände; Delirium; Störungen des Sexuallebens, z.B. Impotenz des Mannes; Eßstörungen, z.B. Anorexie oder Bulimie; Sucht; und weitere nicht näher bezeichnete psychiatrische Störungen.

Zu den erfindungsgemäß behandelbaren Störungen gehören auch Parkinson und Epilepsie und insbesondere die damit in Zusammenhang stehenden affektiven Störungen.

Zu Suchterkrankungen gehören die durch den Missbrauch von psychotropen Substanzen, wie Arzneimittel oder Drogen, verursachte psychische Störungen und Verhaltensstörungen sowie andere Suchterkrankungen, wie beispielsweise die Spielsucht (Impulse Control Disorders not elsewhere classified). Suchterzeugende Substanzen sind beispielsweise: Opioide (z. B. Morphin, Heroin, Codein); Kokain; Nikotin; Alkohol; Substanzen, die mit dem GABA-Chlorid-Kanal-Komplex interagieren, Sedativa, Hypnotika oder Tranquilizer, beispielsweise Benzodiazepine; LSD; Cannabinoide; psychomotorische Stimulanzien, wie 3,4-Methylendioxy-N-methylamphetamin (Ecstasy); Amphetamin und amphetaminartigen Substanzen wie Methylphenidat oder sonstige Stimulanzien einschließlich Koffein. Als suchterzeugende Substanzen kommen insbesondere Opioide, Kokain, Amphetamin oder amphetaminartige Substanzen, Nikotin und Alkohol in Betracht.

Im Hinblick auf die Behandlung von Suchterkrankungen sind unter den erfindungsgemäßen Verbindungen der Formel I solche besonders bevorzugt, die selbst keine psychotrope Wirkung besitzen. Dies kann im Test auch an Ratten beobachtet werden, die nach Verabreichung erfindungsgemäß brauchbarer Verbindungen die Selbstverabreichung von psychotropen Substanzen, beispielsweise Kokain, drosseln.

Gemäß einem weiteren Aspekt der vorliegenden Erfindung sind die erfindungsgemäßen Verbindungen zur Behandlung von Störungen geeignet, deren Ursachen zumindest zum Teil auf eine anomale Aktivität von Dopamin-D₃-Rezeptoren zurückzuführen sind.

Gemäß einem anderen Aspekt der vorliegenden Erfindung richtet sich die Behandlung vor allem auf diejenigen Störungen, die sich über eine Bindung von vorzugsweise exogen zugesetzten Bindungspartnem (Liganden) an Dopamin-D₃-Rezeptoren im Sinne einer zweckmäßigen medizinischen Behandlung beeinflussen lassen.

Die mit den erfindungsgemäßen Verbindungen behandelbaren Erkrankungen sind häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten.

Durch die erfindungsgemäßen Verbindungen lassen sich eine Vielzahl von Anzeichen, Symptomen und/oder Fehlfunktionen behandeln, die mit den Störungen des zentralen Nervensystems und insbesondere den vorstehend genannten Zuständen zusammenhängen. Hierzu gehören beispielsweise ein gestörter Realitätsbezug, mangelnde Einsicht und Fähigkeit, üblichen sozialen Normen bzw. Lebensanforderungen zu genügen, Wesensveränderungen, Veränderungen der Einzeltriebe, wie Hunger, Schlaf, Durst, etc., und der Stimmungslage, Störungen der Merk- und Kombinationsfähigkeit, Persönlichkeitsveränderungen, insbesondere Affektlabilität, Halluzinationen, Ich-Störungen, Zerfahrenheit, Ambivalenz, Autismus, Depersonalisation bzw. Sinnestäuschungen, Wahnideen, skandierende Sprache, fehlende Mitbewegung, kleinschrittiger Gang, Beugehaltung von Rumpf und Gliedern, Tremor, Mimikarmut, monotone Sprache, Depressionen, Apathie, erschwerte Spontanität und Entschlusskraft, verarmte Assoziationsfähigkeit, Angst, nervöse Unruhe, Stottern, soziale Phobie, Panikstörungen, Entzugssyndrome bei Abhängigkeit, maniforme Syndrome, Erregungs- und Verwirrtheitszustände, Dysphorie, dyskinetische Syndrome und Tic-Störungen, z.B. Chorea-Huntington, Gilles-de-la-Tourette-Syndrom, Schwindelsyndrome, z.B. peripherer Lage-, Dreh- und Schwankschwindel, Melancholie, Hysterie, Hypochondrie und ähnliches. Eine Behandlung im erfindungsgemäßen Sinne umfasst nicht nur die Behandlung akuter oder chronischer Anzeichen, Symptome und/oder Fehlfunktionen sondern auch eine vorbeugende Behandlung (Prophylaxe) insbesondere als Rezidiv- oder Phasen-Prophylaxe. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Vorzugsweise eignen sich die erfindungsgemäßen Verbindungen zur Behandlung von Erkrankungen des zentralen Nervensystems, insbesondere zur Behandlung von affektiven Störungen; neurotischen, Belastungs- und somatoformen Störungen und Psychosen und speziell zur Behandlung der Schizophrenie und der Depression. Aufgrund ihrer hohen Selektivität bezüglich des D₃-Rezeptors sind die erfindungsgemäßen Verbindungen I auch zur Behandlung von Nierenfunktionsstörungen, insbesondere von Nierenfunktionsstörungen, die durch Diabetes-Mellitus hervorgerufen wird (siehe WO 00/67847).

Die erfindungsgemäße Verwendung der beschriebenen Verbindungen beinhaltet im Rahmen der Behandlung ein Verfahren. Dabei wird dem zu behandelnden Individuum, vorzugsweise einem Säuger, insbesondere einem Menschen, Nutz- oder Haustier, eine wirksame Menge eines oder mehrerer Verbindungen, in der Regel der pharmazeutischen und tierarzneilichen Praxis entsprechend formuliert, verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken, bestimmte Anzeichen, Symptome und/oder Fehlfunktionen zu entwickeln, und weitere Faktoren miteinbezieht. Die Behandlung erfolgt in der Regel durch einmalige oder mehrmalige tägliche Verabfolgung, gegebenenfalls zusammen oder im Wechsel mit anderen Wirkstoffen oder wirkstoffhaltigen Präparaten, so dass einem zu behandelnden Individuum eine Tagesdosis von vorzugsweise etwa 0,1 bis 1000 mg/kg Körpergewicht bei oraler Gabe bzw. von etwa 0,1 bis 100 mg/kg Körpergewicht bei parenteraler Gabe zugeführt wird.

Die Erfindung betrifft auch die Herstellung pharmazeutischer Mittel zur Behandlung eines Individuums, vorzugsweise eines Säugers, insbesondere eines Menschen, Nutz-oder Haustieres. So werden die Liganden gewöhnlich in Form von pharmazeutischen Zusammensetzungen verabreicht, die einen pharmazeutisch verträglichen Exzipienten mit wenigstens einem erfindungsgemäßen Liganden und gegebenenfalls weiteren Wirkstoffen umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapsein, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions-und Infusionszubereitungen, Augen- und Ohrentropfen. Auch implantierte Abgabevorrichtungen können zur Verabreichung erfindungsgemäßer Inhibitoren verwendet werden. Ferner können auch Liposomen oder Mikrosphären zur Anwendung kommen.

Bei der Herstellung der Zusammensetzungen werden erfindungsgemäße Inhibitoren gewöhnlich mit einem Exzipienten vermischt oder verdünnt. Exzipienten können feste, halbfeste oder flüssige Materialien sein, die als Vehikel, Träger oder Medium für den Wirkstoff dienen.

Geeignete Exzipienten sind in den einschlägigen Arzneimonographien gelistet. Ferner können die Formulierungen pharmazeutisch akzeptable Träger oder übliche Hilfsstoffe, wie Gleitmittel; Netzmittel; emulgierende und suspendierende Mittel; konservierende Mittel; Antioxidantien; Antireizstoffe; Chelatbildner, Dragierhilfsmittel; Emulsionsstabilisatoren; Filmbildner; Gelbildner, Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und

Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Die nachfolgenden Beispiele dienen zur Erläuterung der Erfindung ohne sie zu begrenzen.

Die magnetischen Kernresonanzspektraleigenschaften (NMR) beziehen sich auf chemische Verschiebungen (δ), ausgedrückt in Teile pro Million (ppm). Die relative Fläche für die Verschiebungen in dem ¹H-NMR-Spektrum entspricht der Anzahl der Wasserstoffatome für einen bestimmten funktionalen Typ in dem Molekül. Die Art der Verschiebung hinsichtlich der Multiplizität ist angegeben als Singulett (s), breites Singulett (s. br.), Dublett (d), breites Dublett (d br.), Triplett (t), breites Triplett (t br.), Quartett (q), Quintett (quint.), Multiplett (m).

### Herstellungsbeispiele

### Beispiel 1:

### 1-(3-{4-[2-tert-Butyl-6-(trifluormethyl)pyrimidin-4-yl]piperazin-1-yl}propyl)-4-mercapto-5-methylpyrimidin-2(1H)-on

### 1.1 4-[4-(3-Azidopropyl)piperazin-1-yl]-2-tert-butyl-6-(trifluormethyl)pyrimidin

Zu 13,5 g (36,9 mmol) 2-*tert*-Butyl-4-[4-(3-chlorpropyl)piperazin-1-yl]-6-(trifluormethyl)pyrimidin (DE 197 35 410) in 60 ml N,N-Dimethylformamid (DMF) gab man 2,4 g (36,9 mmol) Natriumazid und rührte das Gemisch 3 Stunden bei 70 °C. Man ließ das Reaktionsgemisch auf Raumtemperatur abkühlen, goss das Gemisch in eine gesättigte Kochsalzlösung und extrahierte die wässrige Mischung mit Essigsäureethylester. Man wusch die organische Phase dreimal mit einer NaCl-Lösung, trocknete über Na₂SO₄, filtrierte das Trockenmittel ab und engte im Vakuum ein. Ausbeute 13,7 g.
ESI-MS: 373,1, [M+H⁺] = 372,1, 186,6;

### 1.2 3-{4-[2-tert-Butyl-6-(trifluormethyl)pyrimidin-4-yl]piperazin-1-yl}propan-1-amin Hydrochlorid

13,7 g (36,89 mmol) 4-[4-(3-Azidopropyl)piperazin-1-yl]-2-*tert*-butyl-6-(trifluormethyl)pyrimidin aus Beispiel 1.1 in 200 ml Methanol und 0,3 g Pd/Kohle (10%ig) wurden unter einer Wasserstoffatmosphäre 12 Stunden bei Raumtemperatur gerührt. Man filtrierte den Katalysator über Kieselgur ab, filtrierte das erhaltene Filtrat und engte die klare Lösung ein. Man löste den Rückstand in Diethylether und gab eine HCI-Lösung in Diethylether zu, wobei ein Niederschlag ausfiel. Man saugte das Hydrochlorid ab, wusch mit Diethylether und trocknete unter Stickstoff und anschließend im Vakuum bei 40 °C. Ausbeute 12,7 g.

¹H-NMR (400 MHz, DMSO-d₆) δ (ppm): 11,62 (1H, s br.), 8,23 (1H, s. br.), 7,23 (1H, s.), 4,67 (1H, s. br.), 3,55 (3H, d br.), 3,32-2,88 (2+2+1H, m br.), 2,10 (1H, quint.), 1,30 (9H, s.).

### 1.3 1-(3-{4-[2-tert-Butyl-6-(trifluormethyl)pyrimidin-4-yl]piperazin-1-yl}propyl)-4-mercapto-5-methylpyrimidin-2(1H)-on:

0,7 g (2,7 mmol) Ethyl-(2E)-2-methyl-3-piperidin-1-ylprop-2-enthioylcarbamat (WO 00/61579), 0,9 g (2,6 mmol) 3-{4-[2-*tert*-Butyl-6-(trifluormethyl)pyrimidin-4-yl]piperazin-1-yl}propan-1-amin Hydrochlorid aus Beispiel 1.2 und 0,6 g (5,5 mmol) N-Methylmorpholin in 20 ml Methanol wurden 12 Stunden unter einer Stickstoffatmosphäre bei Raumtemperatur gerührt. Man filtrierte vom Unlöslichen ab, wusch dreimal mit Methanol und Wasser und kristallisierte anschließend aus Essigsäureethylester um. Nach dem Trocknen bei 50 °C im Vakuum erhielt man 0,5 g der Titelverbindung.
ESI-MS: 472,1, [M+H⁺] = 471,1, 236,1;
¹H-NMR (360 MHz, DMSO-d₆) δ (ppm): 12,33 (1H, s.), 7,57 (1H, s.), 7,48 (1H, s.), 6,81 (1H, s.), 4,62 (2H, s br.), 3,98 (2H, t), 3,84 (2H, t br.), 3,65 (2H, d br.), 3,18 (2H, t br.), 2,36 (2H, quint.), 2,08 (3H, s), 1,33 (9H, s).

### Beispiel 2:

### 1-(4-{4-[2-tert-Butyl-6-(trifluormethyl)pyrimidin-4-yl]piperazin-1-yl}butyl)-4-mercapto-5-methylpyrimidin-2(1H)-on

Analog zu Beispiel 1 erhielt man aus 1,2 g (3,3 mmol) 4-{4-[2-*tert*-Butyl-6-(trifluormethyl)pyrimidin-4-yl]piperazin-1-yl}butan-1-amin 0,7 g der Titelverbindung.

ESI-MS: 486,4, [M+H⁺] = 485,4, 243,3;

¹H-NMR (360 MHz, CDCl₃) δ (ppm): 10,33 (1H, s. br.), 7,00 (1H, s.), 6,57 (1H, s.), 3,60-3,80 (6H, m), 2,52 (4H, t), 2,44 (2H, t), 2,10 (3H, s), 1,78 (2H, quint.), 1,58 (2H, quint.), 1,33 (9H, s).

### Beispiel 3:

### 1-{4-[4-(Benzylthio)-5-methyl-2-oxopyrimidin-1 (2H)-yl]butyl}-4-[2-tert-butyl-6-(trifluormethyl)pyrimidin-4-yl]piperazin-1-iumchlorid

Man rührte 145,4 mg (0,3 mmol) 1-(4-{4-[2-*tert*-Butyl-6-(trifluormethyl)pyrimidin-4-yl]piperazin-1-yl}butyl)-4-mercapto-5-methylpyrimidin-2(1*H*)-on aus Beispiel 2 und 78,8 mg (0,6 mmol) K₂CO₃ in 10 ml N,N-Dimethylformamid (DMF) 20 Minuten. Anschließend tropfte man 77,0 mg (0,5 mmol) Benzylbromid zu. Man rührte das Reaktionsgemisch 2 Tage bei Raumtemperatur, gab es danach auf Wasser und extrahierte mit Essigsäureethylester. Nach Trocknen der organischen Phase über Na₂SO₄, Abfiltrieren des Trockenmittels und Einengen im Vakuum verrührte man den erhaltenen Feststoff mit Diethylether und saugte ab. Nach dem Einengen der Mutterlauge im Vakuum nahm man den Rückstand in wenig CH₂Cl₂ auf und gab eine Lösung von HCl in Diethylether zu, wobei das Wertprodukt als Hydrochlorid ausfiel. Man saugte das Hydrochlorid ab, wusch mit Diethylether und trocknete die Titelverbindung im Vakuum bei 40 °C. Ausbeute: 25 mg.

ESI-MS: [M+Na⁺] = 597,3, 576,2, [M+H⁺] = 575,2, 288,1.

¹H-NMR (500 MHz, DMSO-d₆) δ (ppm): 10,72 (1H, s. br.), 7,85 (1H, s.), 7,40 (2H, d), 7,30 (2H, t), 7,24 (1H, t), 7,21 (1H, s.), 4,38 (2H, s.), 3,79 (2H, m), 3,55 (2H, d br.), 3,51-3,40 (2H, m br.), 3,15-3,00 (4H, m), 1,93 (3H, s.), 1,70 (4H, s. br.), 1,31 (9H, s.).

### Beispiel 4:

### 1-[2-tert-Butyl-6-(trifluormethyl)pyrimidin-4-yl]-4-{4-[5-methyl-4-(methylthio)-2-oxopyrimidin-1(2H)-yl]butyl}piperazin-4-iumchforid

Zu 145,4 mg (0,3 mmol) 1-(4-{4-[2-*tert*-Butyl-6-(trifluormethyl)pyrimidin-4-yl]piperazin-1-yl}butyl)-4-mercapto-5-methylpyrimidin-2(1*H*)-on aus Beispiel 2 in 12 ml Methanol gab man unter Stickstoff 96,9 mg (0,8 mmol) Ethyldiisopropylamin (DIPEA) und rührte das Reaktionsgemisch 20 Minuten. Danach tropfte man 183,1 mg (1,3 mmol) Methyliodid zu und rührte das Reaktionsgemisch 12 Stunden bei Raumtemperatur. Man versetzte das Reaktionsgemisch mit Wasser und extrahierte die wässrige Mischung zweimal mit Essigsäureethylester. Nach Trocknen der organischen Phase über Na₂SO₄, Abfiltrieren des Trockenmittels und Einengen des Lösungsmittels bis zur Trockne im Vakuum nahm man den Rückstand in Diethylether auf. Man gab eine Lösung von HCl in Diethylether zu, wobei das Wertprodukt als Hydrochlorid ausfiel. Man saugte das Hydrochlorid ab, wusch mit Diethylether und trocknete die Titelverbindung im Vakuum bei 40 °C. Ausbeute: 85,0 mg.

ESI-MS: [M+Na⁺] = 521,3, 500,3, [M+H⁺] = 499,2, 250,1;

¹H-NMR (500 MHz, DMSO-d₆) δ (ppm): 10,85 (1H, s. br.), 7,87 (1H, s.), 7,27 (1H, s.), 3,83 (3H, t), 3,60 (2H, d br.), 3,55-3,46 (2H, m), 3,18-3,04 (4H, m), 2,52 (3H, s), 2,00 (3H, s), 1,73 (4H, s. br.), 1,35 (9H, s.).

### Beispiel 5:

### 1-[2-tert-Butyl-6-(trifluormethyl)pyrimidin-4-yl]-4-[4-(4-hydroxy-5-methyl-2-oxopyrimidin-1(2H-yl)butyl]piperazin-4-iumchlorid

### Methode 1

Zu 50,0 mg (0,1 mmol) 1-[2-*tert*-Butyl-6-(trifluormethyl)pyrimidin-4-yl]-4-{4-[5-methyl-4-(methylthio)-2-oxopyrimidin-1 (2*H*)-yl]butyl}piperazin Hydrochlorid aus Beispiel 4 in 5 ml Eisessig tropfte man bei 10 °C 40,9 mg (1,2 mmol) H₂O₂ und rührte 3 Stunden bei Raumtemperatur nach. Anschließend tropfte man weitere 40,9 mg (1,2 mmol) H₂O₂ zu und rührte das Reaktionsgemisch weitere sechs Stunden bei Raumtemperatur nach. Man versetzte das Reaktionsgemisch mit Wasser versetzt und extrahierte anschließend zweimal das wässrige Gemisch mit Dichlormethan. Die organische Phase wurde mit einer wässrigen Natriumthiosulfat-Lösung peroxidfrei gewaschen und danach einmal mit verdünnter, wässriger Natriumcarbonat-Lösung und einmal mit gesättigter, wässriger Kochsalz-Lösung extrahiert. Nach Trocknen der organischen Phase über Na₂SO₄, Abfiltrieren des Trockenmittels und Einengen des Lösungsmittels bis zur Trockne im Vakuum nahm man den Rückstand in Diethylether auf. Man gab eine Lösung von HCl in Diethylether zu, wobei das Wertprodukt als Hydrochlorid ausfiel. Man saugte das Hydrochlorid ab, wusch mit Diethylether und trocknete die Titelverbindung im Vakuum bei 40 °C. Ausbeute: 25,0 mg.

### Methode 2

### 5.2.1 1-(4-Chlorbutyl)-4-hydroxy-5-methylpyrimidin-2(1H)-on

10,1 g (80,0 mmol) 4-Hydroxy-5-methylpyrimidin-2(1*H*)-on (Thymin) in 300 ml Dimethylsulfoxid (DMSO) und 11,1 g (80,0 mmol) K₂CO₃ wurden 1 Stunde bei Raumtemperatur gerührt. Danach tropfte man zu dem Gemisch 13,7 g (80,0 mmol) 1-Brom-4-chlorbutan und rührte das Reaktionsgemisch 5 Stunden bei Raumtemperatur nach. Man versetzte das Reaktionsgemisch mit Wasser und extrahierte anschließend mit Essigsäureethylester. Danach neutralisierte man die wässrige Phase und extrahierte mit Methylenchlorid. Nach Trocknen der organischen Phase, Abfiltrieren des Trockenmittels und Einengen des Lösungsmittels im Vakuum bis zur Trockne erhielt man 7,1 g 1-(4-Chlorbutyl)-4-hydroxy-5-methylpyrimidin-2(1*H*)-on.

ESI-MS: 219,1, [M+H⁺] = 217,1;
¹H-NMR (500 MHz, CDCl₃) δ (ppm): 9,97 (1H, s.), 7,02 (1H, s.), 3,74 (2H, t), 3,55 (2H, t), 1,93 (3H, s), 2,02-1,75 (4H, m).

### 5.2.2 1-[2-tert-Butyl-6-(trifluormethyl)pyrimidin-4-yl]-4-[4-(4-hydroxy-5-methyl-2-oxopyrimidin-1 (2H)-yl)butyl]piperazin-4-iumchlorid

Man rührte 1,5 g (7,0 mmol) 1-(4-Chlorbutyl)-4-hydroxy-5-methylpyrimidin-2(1*H*)-on aus Beispiel 5.2.1, 2,0 g (7,0 mmol) 2-*tert*-Butyl-4-piperazin-1-yl-6-(trifluormethyl)pyrimidin (DE 197 35 410) und 1,4 g (14,0 mmol) NEt₃ in 100 ml N,N-Dimethylformamid (DMF) 24 Stunden bei 110 °C. Danach gab man Essigsäureethylester zu und wusch das Gemisch zweimal mit Wasser. Man trocknete die vereinten organischen Phasen über Na₂SO₄, filtrierte das Trockenmittel ab und engte im Vakuum ein. Man reinigte den öligen Rückstand durch Chromatographie an Kieselgel (Eluierungsmittel: Dichlormethan:Methanol 95:5 v/v), rührte ihn mit Pentan aus und saugte ab. Man nahm den Feststoff in wenig Methylenchlorid auf und gab eine Lösung von HCl in Diethylether zu, wobei das Wertprodukt als Hydrochlorid ausfiel. Man saugte das Hydrochlorid ab, wusch mit Diethylether und trocknete die Titelverbindung im Vakuum bei 40 °C. Ausbeute: 1,1 g.

ESI-MS: 470,5, [M+H⁺] = 469,5, 235,3;
¹H-NMR (500 MHz, DMSO-d₆) δ (ppm): 11,57 (1H, s. br.), 11,23 (1H, s.), 7,62 (1H, s.), 7,24 (1H, s.), 4,68 (2H, s. br.), 3,65 (2H, t), 3,55 (4H, d br.), 3,14-3,00 (4H, m), 1,82-1,71 (3+2H, s+m), 1,68-1,59 (2H, m), 1,31 (9H, s).

### Beispiel 6:

### 1-(4-{4-[2-tert-Butyl-6-(trifluormethyl)pyrimidin-4-yl]piperazin-1-yl}butyl)-5-methylpyrimidin-2(1H)-on

### 6.1 5-Methylpyrimidin-2(1H)-on (Chem. Ztg. 1977, 6, 305-7)

Man erhitzte 11,4 g (0,1 mol) (2Z)-3-Ethoxy-2-methylprop-2-enal (3-Ethoxymethacrolein), 6,0 g (0,1 mol) Carbamid (Harnstoff) und 10 ml konz. HCl-Lösung in 20 ml Ethanol 3,5 Stunden am Rückfluss. Anschließend ließ man das Reaktionsgemisch abkühlen und kühlte mit Eiswasser, wobei sich ein Niederschlag bildete. Man saugte die ausgefallenen Kristalle ab, wusch mit Ethanol und trocknete bei 40 °C im Vakuum. Ausbeute: 11,0 g.

ESI-MS: [2M+Na⁺] = 243,1, [M+H⁺] =111,1;
¹H-NMR (400 MHz, DMSO-d₆) δ (ppm): 8,68 (2H, s.), 2,12 (3H, s.).

### 6.2 1-(4-Chlorbutyl)-5-methylpyrimidin-2(1H)-on

Man rührte 10,5 g (95,0 mmol) 5-Methylpyrimidin-2(1*H*)-on aus Beispiel 6.1,16,3 g (95,0 mmol) 1-Brom-4-chlorbutan und 39,4 g (285,0 mmol) K₂CO₃ in 200 ml Dimethylsulfoxid 12 Stunden bei Raumtemperatur. Man gab das Reaktionsgemisch auf Eiswasser und extrahierte das wässrige Gemisch zweimal mit Diethylether. Die wässrige Phase wurde zweimal mit Methylenchlorid extrahiert. Man trocknete die Methylenchloridphase über Na₂SO₄, filtrierte das Trockenmittel ab und engte das Lösungsmittel bis zur Trockne im Vakuum ein. Man verrührte den erhaltenen festen Rückstand mit Diethylether, saugte den Niederschlag ab, wusch mit Diethylether und trocknete. Ausbeute: 5,0 g.

¹H-NMR (400 MHz, CDCl₃) δ (ppm): 8,46 (1H, d), 7,46 (1H, d), 3,90 (2H, t), 3,57 (2H, t), 2,11 (3H, s.), 1,95 (2H, quint.), 1,88-1,78 (2H, quint).

### 6.3 1-(4-{4-[2-tert-Butyl-6-(trifluormethyl)pyrimidin-4-yl]piperazin-1-yl}butyl)-5-methylpyrimidin-2(1H)-on

Analog zu Beispiel 5.2.2 erhielt man ausgehend von 1,4 g (5,0 mmol) 1-(4-Chlorbutyl)-5-methylpyrimidin-2(1*H*)-on aus Beispiel 6.2 die Titelverbindung; Ausbeute: 0,5 g.

ESI-MS: 454,2, [M+H⁺] = 453,3, 227,1;
¹H-NMR (500 MHz, DMSO-d₆) δ (ppm): 8,41 (1H, s.), 7,97 (1H, s.), 6,93 (1H, s.), 3,82 (2H, t), 3,69 (4H, s. br.), 2,42 (4H, m sym.), 2,35 (2H, t), 2,05 (3H, s), 1,70 (2H, quint.), 1,48 (2H, quint.), 1,28 (9H, s).

### Beispiel 7:

### 1-(4-{4-[2-tert-Butyl-6-(trifluormethyl)pyrimidin-4-yl]piperazin-1-yl}butyl)-5-methyl-4-phenoxypyrimidin-2(1H)-on

### 7.1 1-(4-{4-[2-tert-Butyl-6-(trifluormethyl)pyrimidin-4-yl]piperazin-1-yl}butyl)-5-methyl-2-oxo-1,2-dihydropyrimidin-4-ylthiocyanat

Zu 484,6 mg (1,0 mmol) 1-(4-{4-[2-tert-Butyl-6-(trifluormethyl)pyrimidin-4-yl]piperazin-1-yl}butyl)-4-mercapto-5-methylpyrimidin-2(1*H*)-on aus Beispiel 2 in 20 ml Methylenchlorid gab man eine Lösung von KCN (325,6 mg, 5,0 mmol) in 5%iger NaHCO₃-Lösung (7 ml). Anschließend tropfte man ein Gemisch aus 2,0 mg 18-Krone-6 und 105,9 mg (1,0 mmol) BrCN in der zum Lösen minimal erforderlichen Menge Methylenchlorid bei 0° C zu. Anschließend trennte man die organische Phase ab und wusch sie zweimal mit einer gesättigten, wässrigen Kochsalzlösung. Man trocknete die organische Phase über Na₂SO₄, filtrierte das Trockenmittel ab und engte das Lösungsmittel im Vakuum bis zur Trockne ein; Ausbeute: 420,0 mg. 1-(4-{4-[2-*tert*-Butyl-6-(trifluormethyl)pyrimidin-4-yl]piperazin-1-yl}butyl)-5-methyl-2-oxo-1,2-dihydropyrimidin-4-ylthiocyanat wurde ohne zusätzliche Reinigung im nächsten Schritt eingesetzt.

### 7.2 1-(4-{4-[2-tert-Butyl-6-(trifluormethyl)pyrimidin-4-yl]piperazin-1-yl}butyl)-5-methyl-4-phenoxypyrimidin-2(1H)-on

Zu 400,0 mg (0,78 mmol) 1-(4-{4-[2-*tert*-Butyl-6-(trifluormethyl)pyrimidin-4-yl]piperazin-1-yl}butyl)-5-methyl-2-oxo-1,2-dihydropyrimidin-4-ylthiocyanat aus Beispiel 7.1 in 40 ml Acetonitril und 500,0 mg Molekularsieb (3 A) gab man 81,3 mg (0,9 mmol) Phenol und 325,5 mg (2,4 mmol) K₂CO₃. Anschließend rührte man das Reaktionsgemisch 12 Stunden bei Raumtemperatur. Man filtrierte vom Unlöslichen ab, gab zu dem Filtrat Methylenchlorid und extrahierte anschließend mit einer wässrigen Kochsalzlösung. Nach Trocknen der organischen Phase, Abfiltrieren des Trockenmittels und Einengen des Lösungsmittels im Vakuum nahm man den Rückstand in 100 ml Diisopropylether auf und erwärmte. Der Niederschlag wurde abgesaugt und die Mutterlauge etwas eingeengt. Anschließend versetzte man die Mutterlauge mit Pentan und kühlte im Eisbad. Man saugte den ausgefallenen Feststoff ab, wusch und trocknete ihn bei 40°C im Vakuum; wobei man 120 mg der Titelverbindung erhielt.

ESI-MS: 546,3, [M+H⁺] = 545,3, 273,1;

¹H-NMR (500 MHz, CDCl₃) δ (ppm): 7,37 (2H, t), 7,32 (1H, s.), 7,20 (1H, t), 7,13 (2H, d), 6,56 (1H, s.), 3,85 (2H, t), 3,69 (4H, s br.), 2,50 (4H, m sym.), 2,41 (2H, t), 2,12 (3H, s.), 1,79 (2H, quint.), 1,57 (2H, quint.), 1,32 (9H, s.).

### Beispiel 8:

### 1-(4-{4-[2-tert-Butyl-6-(trifluormethyl)pyrimidin-4-yl]piperazin-1-yl}butyl)-4-methylpyrimidin-2(1H)-on

### 8.1 4-Methylpyrimidin-2(1H)-on (Analog zu Aust. J. Chem. 1968, 21, 243-55)

Zu 26,4 g (0,2 mol) 4,4-Dimethoxybutan-2-on in 40 ml Ethanol und 12,0 g (0,2 mol) Harnstoff tropfte man 20,0 ml konz. HCl. Nach kurzer Zeit entstand eine klare braune Lösung und nach weiteren 10 Minuten fiel ein gelber Niederschlag aus. Man erhitzte das Reaktionsgemisch 1,5 Stunden am Rückfluss erhitzt und ließ dann abkühlen (Eiswasserbad). Danach saugte man die ausgefallenen Kristalle ab, wusch mit Ethanol und trocknete die Kristalle bei 40°C im Vakuum; Ausbeute: 22,0 g.

¹H-NMR (400 MHz, DMSO-d₆) δ (ppm): 8,61 (1H, d), 6,81 (1H, d), 2,59 (3H, s.).

### 8.2 1-(4-Chlorbutyl)-4-methylpyrimidin-2(1H)-on

Analog zu Beispiel 6.2 erhielt man aus 12,1 g (0,1 mol)4-Methylpyrimidin-2(1H)-on 5,1 g 1-(4-Chlorbutyl)-4-methylpyrimidin-2(1*H*)-on.

ESI-MS: [M+H⁺] = 201,1;
¹H-NMR (400 MHz, CDCl₃) δ (ppm): 7,52 (1H, d), 6,22 (1H, d), 3,92 (2H, t), 3,57 (2H, t), 2,39 (3H, s.), 1,94 (2H, quint.), 1,86-1,74 (2H, m).

### 8.3 1-(4-{4-[2-tert-Butyl-6-(trifluormethyl)pyrimidin-4-yl]piperazin-1-yl}butyl)-4-methylpyrimidin-2(1H)-on

Analog zu Beispiel 5.2.2 stellte man aus 1,1 g (5,5 mmol) 1-(4-Chlorbutyl)-4-methylpyrimidin-2(1*H*)-on 0,25 g der Titelverbindung her.

ESI-MS: 454,2, [M+H⁺] = 453,3, 227,1;
¹H-NMR (500 MHz, CDCl₃) δ (ppm): 7,48 (1H, d), 6,56 (1H, s.), 6,19 (1H, d), 3,90 (2H, t), 3,70 (4H, s. br.), 2,50 (4H, m sym.), 2,43 (2H, t), 2,40 (3H, s), 1,83 (2H, quint.), 1,59 (2H, quint.), 1,33 (9H, s.).

### Beispiel 9:

### 1-{4-[4-(2,6-ditert-Butylpyrimidin-4-yl)piperazin-1-yl]butyl}-4-hydroxy-5-methylpyrimidin-2(1H)-on

Analog zu Beispiel 5.2.2 erhielt man aus 390,0 mg (1,8 mmol) 1-(4-Chlorbutyl)-4-hydroxy-5-methylpyrimidin-2(1*H*)-on aus Beispiel 5.2.1 und 414,6 mg (1,5 mmol) 2,4-di*tert*-Butyl-6-piperazin-1-ylpyrimidin 170,0 mg der Titelverbindung.

ESI-MS: 458,4, [M+H⁺] = 457,4, 229,1;
¹H-NMR (500 MHz, CDCl₃) δ (ppm): 9,29 (1H, s br.), 6,97 (1H, s.), 6,24 (1H, s), 3,72 (2H, t), 3,64 (4H, m sym.), 2,52 (4H, m sym.), 2,44 (2H, t), 1,93 (3H, s), 1,74 (2H, quint.), 1,58 (2H, quint.), 1,27 (9H, s.), 1.33 (9H, s.).

### Beispiel 10:

### 1-{4-[4-(2-tert-Butyl-6-propylpyrimidin-4-yl)piperazin-1-yl]butyl}-4-hydroxy-5-methylpyrimidin-2(1H)-on

Analog zu Beispiel 5.2.2 erhielt man aus 390,0 mg (1,8 mmol) 1-(4-Chlorbutyl)-4-hydroxy-5-methylpyrimidin-2(1*H*)-on aus Beispiel 5.2.1 und 393,6 mg (1,5 mmol) 2*-tert-*Butyl-4-piperazin-1-yl-6-propylpyrimidin 220,0 mg der Titelverbindung.

ESI-MS: 444,2, [M+H⁺] = 443,4, 222,1;
¹H-NMR (500 MHz, CDCl₃) δ (ppm): 9,01 (1H, s.), 6,96 (1H, s.), 6,11 (1H, s.), 3,72 (2H, t), 3,62 (4H, m sym.), 2,57-2,49 (2+4H, m), 2,41 (2H, t), 1,93 (3H, s), 1,58 (2H, quint.), 1,31 (9H, s.).

### Beispiel 11:

### 4-(Benzylamino)-1-(4-{4-[2-tert-butyl-6-(trifluormethyl)pyrimidin-4-yl]piperazin-1-yl}butyl)-5-methylpyrimidin-2(1H)-on

Analog zu Beispiel 5.2.2 erhielt man aus 2,0 g (4,0 mmol)1-(4-{4-[2-*tert*-Butyl-6-(trifluormethyl)pyrimidin-4-yl]piperazin-1-yl}butyl)-5-methyl-2-oxo-1,2-dihydropyrimidin-4-ylthiocyanat aus Beispiel 7.1 und 0,5 g (4,4 mmol) Benzylamin 0,5 g der Titelverbindung.

ESI-MS: 559,5, [M+H⁺] = 558,5, 279,8;
¹H-NMR (500 MHz, CDCl₃) δ (ppm): 7,38 (1H, s), 7,34-7,24 (5H, m), 7,03 (1H, s. br.), 6,64 (1H, s.), 4,68 (2H, m sym.), 3,83 (2H, m), 3,11 (4H, s. br.), 2,85 (4H, s. br.), 2,04 (3H, s.), 1,84 (4H, s. br.), 1,31 (9H, s.).

### Beispiel 12:

### 1-[2-tert-Butyl-6-(trifluormethyl)pyrimidin-4-yl]-4-{4-[4-(dimethylamino)-5-methyl-2-oxopyrimidin-1 (2H)-yl]butyl}piperazin-4-iumchlorid

Analog zu Beispiel 7.2 erhielt man aus 1,0 g (2,0 mmol) 1-(4-{4-[2-tert-Butyl-6-(trifluormethyl)pyrimidin-4-yl]piperazin-1-yl}butyl)-5-methyl-2-oxo-1,2-dihydropyrimidin-4-ylthiocyanat aus Beispiel 7.1 und Dimethylamin (2M in Methanol, 1,1 ml) 0,7 g der Titelverbindung.

ESI-MS: [M+H⁺] = 496,5, 248,7;
¹H-NMR (500 MHz, DMSO-d₆) δ (ppm): 11,61 (1H, s. br.), 7,96 (1H, s.), 7,21 (1H, s), 3,76 (2H, t), 3,54 (2H, d br.), 3,27 (6H, s), 3,12-3,00 (2+2H, m), 2,20 (3H, s), 1,75-1,67 (2+2H, m), 1,30 (9H, s).

### Beispiel 13:

### 1-(4-{4-[2-tert-Butyl-6-(trifluormethyl)pyrimidin-4-yl]piperazin-1-yl}butyl)-4-hydroxypyrimidin-2(1H)-on

Analog zu Beispiel 7.2 erhielt man aus 1,0 g (4,0 mmol) 1-(4-Brombutyl)pyrimidin-2,4(1*H*,3*H*)-dion *(*J. Am. Chem. Soc. 1993, 115, 7636) 0,6 g der Titelverbindung.

ESI-MS: 456,2, [M+H⁺] = 455,3, 228,1;
¹H-NMR (500 MHz, CDCl₃) δ (ppm): 10,04 (1H, s. br.), 7,15 (1H, d), 6,58 (1H, s.), 5,68 (1H, d), 3,75 (2H, t), 3,70 (4H, s. br.), 2,51 (4H, t), 2,43 (2H, t), 1,74 (2H, quint.), 1,57 (2H, quint.), 1,33 (9H, s).

### Beispiel 14:

### 4-tert-Butyl-1-(4-{4-[2-tert-butyl-6-(trifluormethyl)pyrimidin-4-yl]piperazin-1-yl}butyl)pyrimidin-2(1H)-on

### 14.1 4-tert-Butyl-1-(4-chlorbutyl)pyrimidin-2(1H)-on

Analog zu Beispiel 6.2 erhielt man aus 16,7 g (0,1 mol) 4-tert-Butylpyrimidin-2(1H)-on 13,4 g 4-tert-Butyl-1-(4-chlorbutyl)pyrimidin-2(1H)-on.

¹H-NMR (500 MHz, CDCl₃) δ (ppm): 7,54 (1H, d), 6,38 (1H, d), 3,92 (2H, t), 3,59 (2H, t), 1,97 (2H, quint.), 1,87 (2H, quint.), 1,30 (9H, s).

### 14.2 4-tert-Butyl-1-(4-{4-[2-tert-butyt-6-(trifluoromethyl)pyrimidin-4-yl]piperazin-1-yl}butyl)pyrimidin-2(1H)-on

Analog zu Beispiel 5.2.2 erhielt man aus 1,21 g (5,0 mmol) 4-*tert*-Butyl-1-(4-chlorbutyl)pyrimidin-2(1H)-on aus Beispiel 14.1 0,8 g der Titelverbindung.

ESI-MS: 496,4, [M+H⁺] = 495,4, 248,1;
¹H-NMR (500 MHz, CDCl₃) δ (ppm): 7,52 (1H, d), 6,58 (1H, s), 6,37 (1H, d), 3,91 (2H, t), 3,70 (4H, s br.), 2,48 (4H, t), 2,41 (2H, t), 1,86 (2H, quint.), 1,59 (2H, quint.), 1,35 (9H, s), 1,30 (9H, s).

### Beispiel 15:

### 1-[2-tert-Butyl-6-(trifluormethyl)pyrimidin-4-yl]-4-{4-[2-oxo-4-(trifluormethyl)pyrimidin-1(2H)-yl]butyl}piperazin-4-iumchlorid

### 15.1 1-(4-Chlorbutyl)-4-(trifluormethyl)pyrimidin-2(1H)-on

Man rührte 4,6 g (28,3 mmol) 2-Hydroxy-4-trifluormethylpyrimidin in 60 ml N,N-Dimethylformamid (DMF) und 3,9 g (28,3 mmol) K₂CO₃ 1 Stunde bei Raumtemperatur. Danach tropfte man 4,9 g (28,3 mmol) 1-Brom-4-chlorbutan zu und rührte das Reaktionsgemisch 6 Stunden bei Raumtemperatur. Anschließend gab man Wasser zum Reaktionsgemisch und extrahierte das wässrige Gemisch mit Diethylether. Man stellte die wässrige Phase durch Zugabe von NaOH alkalisch und extrahierte die wässrige Phase mit Methylenchlorid. Anschließend trocknete man die organische Phase, filtrierte das Trockenmittel ab und engte das Lösungsmittel im Vakuum bis zur Trockne ein; Ausbeute: 1,7 g.

ESI-MS: [M+Na⁺] = 257,0, [M+H⁺] = 255,1;

### 15.2 1-[2-tert-Butyl-6-(trifluormethyl)pyrimidin-4-yl]-4-{4-[2-oxo-4-(trifluormethyl)pyrimidin-1 (2H)-yl]butyl}piperazin-4-iumchlorid

Analog zu Beispiel 5.2.2 erhielt man aus 0,6 g (2,36 mmol) 1-(4-Chlorbutyl)-4-(trifluormethyl)pyrimidin-2(1H)-on aus Beispiel 15.1 0,53 g der Titelverbindung.

ESI-MS: [M+Na⁺] = 529,3, 508,3, [M+H⁺] = 507,2, 254,1;
¹H-NMR (500 MHz, DMSO-d₆) δ (ppm): 11,37 (1H, s br.), 8,69 (1H, d), 7,20 (1H, s), 6,89 (1H, d), 4,23 (4H, s br.), 3,98 (2H, t), 3,54 (4H, m br.), 3,14-2,98 (4H, m br.), 1,75 (4H, s br.), 1,30 (9H, s).

### Beispiel 16:

### 1-[2-tert-Butyl-6-(trifluormethyl)pyrimidin-4-yl]-4-[4-(5-fluor-2,4-dioxo-3,4-dihydropyrimidin-1 (2H)-yl)butyl]piperazin-4-lumchlorid

### 16.1 1-(4-Chlorbutyl)-5-fluorpyrimidin-2,4(1H,3H)-dion

Zu einer Lösung von 1,95 g (15,0 mmol) 2,4-Dihydroxy-5-fluorpyrimidin in 50 ml Dimethylsulfoxid und 20,0 ml N,N-Dimethylformamid (DMF) tropfte man bei 0 °C 2,6 g (15,0 mmol) 1-Brom-4-chlorbutan zu. Portionsweise gab man über 1 Stunde 2,07 g (15,0 mmol) K₂CO₃ zu und rührte das Gemisch 1 Stunde bei 20°C (dialkyliertes Produkt ist bereits erkennbar). Anschließend gab man zum Reaktionsgemisch Wasser und extrahierte das wässrige Gemisch zweimal mit Diethylether und zweimal mit Methylenchlorid. Man stellte die wässrige Phase mit Salzsäure auf pH 3-4 und extrahierte danach die wässrige Phase mit Methylenchlorid. Anschließend trocknete man die organische Phase, filtrierte das Trockenmittel ab und engte das Lösungsmittel bis zur Trockne im Vakuum ein; Ausbeute: 0,6 g. 1-(4-Chlorbutyl)-5-fluorpyrimidin-2,4(1*H*,3*H*)-dion wurde ohne weitere Reinigung im nächsten Schritt eingesetzt.

### 16.2 1-[2-tert-Butyl-6-(trifluormethyl)pyrimidin-4-yl]-4-[4-(5-fluor-2,4-dioxo-3,4-dihydropyrimidin-1 (2H)-yl)butyl]piperazin-4-iumchlorid

Analog zu Beispiel 5.2.2 erhielt man aus 0,66 g (3,00 mmol) 1-(4-Chlorbutyl)-5-fluorpyrimidin-2,4(1*H*,3*H*)-dion 0,03 g der Titelverbindung.

ESI-MS: 474,5, [M+H⁺] = 473,5, 237,3.

### Beispiel 17:

### 1-{4-[4-(2-tert-Butyl-6-cyclopropylpyrimidin-4-yl)piperazin-1-yl]butyl}-4-hydroxy-5-methylpyrimidin-2(1H)-on

Analog zu Beispiel 5.2.2 erhielt man aus 0,43 g (2,00 mmol) 1-(4-Chlorbutyl)-5-methylpyrimidin-2,4(1*H*,3*H*)-dion aus Schritt 5.2.1 und 0,52 g (2,00 mmol) 2-*tert*-Butyl-4-cyclopropyl-6-piperazin-1-ylpyrimidin 0,18 g der Titelverbindung.

ESI-MS: 442,5, [M+H⁺] = 441,5, 221,3;
¹H-NMR (500 MHz, CDCl₃) δ (ppm): 8,80 (1H, s br.), 6,97 (1H, s), 6,15 (1H, s), 3,71 (2H, t), 3,62 (4H, t br.), 2,48 (4H, t), 2,40 (2H, t), 1,93 (3H, s), 1,81-1,66 (1+2H, m), 1,57 (2H, quint.), 1,27 (9H, s), 1,07 (2H, m sym.), 0,85 (2H, m sym.).

### Beispiel 18:

### 1-(4-{4-[2-tert-Butyl-6-(trifluormethyl)pyrimidin-4-yl]piperazin-1-yl}butyl)-2,4-dioxo-1,2,3,4-tetrahydropyrimidin-5-carbonitril

### 18.1 1-(4-Chlorbutyl)-2,4-dioxo-1,2,3,4-tetrahydropyrimidin-5-carbonitril

Zu einer Lösung von 2,06 g (15,0 mmol) 5-Cyanouracil in 50 ml Dimethylsulfoxid (DMSO) und 20,0 ml N,N-Dimethylformamid (DMF) tropfte man bei 0 °C 2,6 g (15,0 mmol) 1-Brom-4-chlorbutan und gab 2,07 g (15,0 mmol) K₂CO₃ portionsweise über 1 Stunde zu. Danach rührte man das Gemisch 10 Minuten bei 20°C (dialkyliertes Produkt schon erkennbar). Zu dem Reaktionsgemisch gab man Wasser und extrahierte danach die wässrige Mischung zweimal mit Diethylether und zweimal mit Methylenchlorid. Man stellte die wässrige Phase auf pH 3-4 und extrahierte anschließend mit Methylenchlorid. Danach trocknete man die Methylenchlorid-Phase, filtrierte das Trockenmittel ab und engte das Lösungsmittel bis zur Trockne im Vakuum; Ausbeute: 0,6 g. 1-(4-Chlorbutyl)-2,4-dioxo-1,2,3,4-tetrahydropyrimidin-5-carbonitril wurde ohne weitere Reinigung im nächsten Schritt eingesetzt.

ESI-MS: [M+H⁺]= 228,05.

### 18.2 1-(4-{4-[2-tert-Butyl-6-(trifluormethyl)pyrimidin-4-yl]piperazin-1-yl}butyl)-2,4-dioxo-1,2,3,4-tetrahydropyrimidin-5-carbonitril

Analog zu Beispiel 5.2.2 erhielt man aus 0,68 g (3,00 mmol) 1-(4-Chlorbutyl)-2,4-dioxo-1,2,3,4-tetrahydropyrimidin-5-carbonitril aus Beispiel 18.1 0,07 g der Titelverbindung.

ESI-MS: 481,5, [M+H⁺]= 480,5, 240,7;
¹H-NMR (500 MHz, CDCl₃) δ (ppm): 8,02 (1H,s), 6,59 (1H,s), 3,84 (2H, t), 3,71 (4H,s br.), 2,50 (4H, t br.), 2,41 (2H, t), 1,79 (2H, quint.), 1,56 (2H, quint.), 1,32 (9H, s).

### Beispiel 19:

### 4-tert-Butyl-1-{4-[4-(2-tert-butyl-6-cyclopropylpyrimidin-4-yl)piperazin-1-yl]butyl}pyrimidin-2(1H)-on

Analog zu Beispiel 5.2.2 erhielt man aus 0,49 g (2,00 mmol) 4-*tert-*Butyl-1-(4-chlorbutyl)pyrimidin-2(1*H*)-on aus Beispiel 14.1 und 0,52 g (2,00 mmol) 2-*tert*-Butyl-4-cyclopropyl-6-piperazin-1-ylpyrimidin 0,11 g der Titelverbindung.

ESI-MS: 468,5, [M+H⁺] = 467,4, 234,2;
¹H-NMR (500 MHz, CDCl₃) δ (ppm): 7,51 (1H, d), 6,36 (1H, d), 6,14 (1H, s), 3,91 (2H, t), 3,61 (4H, t br,), 2,46 (4H, t br.), 2,38 (2H, t), 1,86-1,74 (2+1H, m), 1,57 (2H, quint.), 1,31 (9H, s), 1,27 (9H, s), 1,07 (2H, m sym.), 0,87 (2H, m sym.).

### Beispiel 20:

### 1-{4-[4-(2-tert-Butyl-6-propylpyrimidin-4-yl)piperazin-1-yl]butyl}-4-hydroxypyrimidin-2(1H)-on

Analog zu Beispiel 5.2.2 erhielt man aus 1,7 mg (8,4mmol) 1-(4-Chlorbutyl)-4-hydroxypyrimidin-2(1*H*)-on (erhältlich analog zur Herstellung von 1-(4-Brombutyl)pyrimidin-2,4(1*H*,3*H*)*-*dion, s. Beispiel 13) und 1,84 mg (7,0 mmol) *2-tert*-Butyl-4-piperazin-1-yl-6-propylpyrimidin 1,8 g der Titelverbindung.

ESI-MS: 430,4, [M+H⁺] = 429,4, 215,1;
¹H-NMR (500 MHz, CDCl₃) δ (ppm): 9,43 (1H, s br.), 7,13 (1H, d), 6,11 (1H, s), 5,68 (1H, d), 3,78 (2H, t), 3,63 (4H, s br.), 2,58-2,48 (4H, m), 2,43 (2H, t), 1,79-1,66 (2+1H, m), 1,57 (2H, quint.), 1,31 (9H, s), 0,94 (3H, t).

### Beispiel 21:

### 1-[4-(4-tert-Butyl-2-oxopyrimidin-1(2H)-yl)butyl]-4-(2-tert-butyl-6-propylpyrimidin-4-yl)piperazin-1-iumchlorid

Analog zu Beispiel 5.2.2 erhielt man aus 2,04 g (8,40 mmol) 4-*tert*-Butyl-1-(4-chlorbutyl)pyrimidin-2(1*H*)-on aus Beispiel 14.1 und 1,84 mg (7,0 mmol) 2-*tert*-Butyl-4-piperazin-1-yl-6-propylpyrimidin 1,8 g der Titelverbindung.

ESI-MS: 470,5, [M+H⁺] = 469,4, 235,3, 157,2;
¹H-NMR (500 MHz, DMSO-d₆) δ (ppm): 14,13 (1H, s br.), 11,89 (1H, s br.), 8,59 (1H, d), 6,73 (1H, d), 5,00 (1H, d br.), 4,47 (1H, d br.), 3,61 (3H, d br.), 3,14 (4H, s br.), 2,88 (2H, t), 1,84-1,66 (6H, m), 1,43 (9H, s), 1,28 (9H, s), 0,94 (3H, t).

### Beispiel 22:

### 1-{4-[4-(2-tert-Butyl-6-cyclobutylpyrimidin-4-yl)piperazin-1-yl]butyl}-4-hydroxy-5-methylpyrimidin-2(1H)-on

Analog zu Beispiel 5.2.2 erhielt man aus 0,30 g (1,10 mmol) 2-*tert*-Butyl-4-cyclobutyl-6-piperazin-1-ylpyrimidin 0,11 g der Titelverbindung.

ESI-MS: 456,4, [M+H⁺] = 455,4, 228,2;
¹H-NMR (500 MHz, CDCl₃) δ (ppm): 8,88 (1H, s br.), 6,97 (1H, s), 6,11 (1H, s), 3,71 (2H, t), 3,63 (4H, s br.), 3,42 (1H, t), 2,49 (4H, t), 2,41 (2H, t), 2,27 (4H, m), 2,00 (1H, sext.), 1,92 (3H, s), 1,92-1,84 (1H, m), 1,72 (2H, quint.), 1,56 (2H, quint.), 1,32 (9H, s).

### Beispiel 23:

### 1-{4-[4-(2-tert-Butyl-6-cyclobutylpyrimidin-4-yl)piperazin-1-yl]butyl}pyrimidin-2,4(1H,3H)-dion

Analog zu Beispiel 5.2.2 erhielt man aus 0,28 g (1,40 mmol) 1-(4-Chlorbutyl)-4-hydroxypyrimidin-2(1*H*)-on (erhältlich analog zur Herstellung von 1-(4-Brombutyl)pyrimidin-2,4(1*H*,3*H*)-dion, s. Beispiel 13) und 0,30 g (1,10 mmol) 2-*tert*-Butyl-4-cyclobutyl-6-piperazin-1-ylpyrimidin 0,14 g der Titelverbindung.

ESI-MS: 442,5, [M+H⁺] = 441,5;
¹H-NMR (500 MHz, CDCl₃) δ (ppm): 7,15 (1H, d), 6,11 (1H, s), 5,69 (1H, d), 3,77 (2H, t), 3,63 (4H, s br.), 3,42 (1H, quint.), 2,51 (4H, t), 2,42 (2H, t), 2,32-2,20 (2+2H, m), 2,05-1,84 (1+1H, m), 1,75 (2H, quint.), 1,56 (2H, quint.), 1,31 (9H, s).

### Beispiel 24:

### 1-(4-{4-[2-tert-Butyl-6-(trifluormethyl)pyrimidin-4-yl]piperazin-1-yl}butyl)-4-phenylpyrimidin-2(1H)-on

### 24.1 2-Chlor-4-phenylpyrimidin

Zu 1,00 g (6,71 mmol) 2,4-Dichlorpyrimidin und 0,82 g (6,71 mmol) Benzolboronsäure in 29 ml Toluol und 7 ml Methanol gab man 2,78 g (20,14 mmol) K₂CO₃, 0,21g (0,18 mmol) Tetrakis(triphenylphosphin)Pd(0) und rührte das Reaktionsgemisch 3 Stunden bei Raumtemperatur. Nach Einengen des Reaktionsgemisches nahm man den Rückstand in Wasser/Methyl-tert.-butylether auf. Anschließend extrahierte man die wässrige Phase zweimal mit Methyl-tert.-butylether. Danach wusch man die vereinte organische Phase mit Wasser und mit einer gesättigten, wässrigen NaCl-Lösung, trocknete die organische Phase, filtrierte das Trockenmittel ab und engte ein. Man reinigte den festen, braunen Rückstand durch Flash-Chromatographie an Kieselgel (Laufmittel: Essigsäureethylester/Cyclohexan: 10:90); Ausbeute: 0,90 g.

¹H-NMR (400 MHz, CDCl₃) δ (ppm): 8,64 (1H, d), 8,10 (2H, d), 7,650 (1H, d), 7,58-7,48 (3H, m).

### 24.2 4-Phenylpyrimidin-2-ol

Man erhitzte 0,80 g (4,20 mmol) 2-Chlor-4-phenylpyrimidin aus Beispiel 24.1 in 3,20 ml konz. HCl 1 Stunde auf 100 °C. Anschließend engte man den Ansatz ein, suspendierte ihn in Methylenchlorid und engte erneut ein. Ausbeute: 0,83 g.

ESI-MS: 174,3, [M+H⁺] = 173,2;

### 24.3 1-(4-Chlorbutyl)-4-phenylpyrimidin-2(1H)-on

Man rührte 0,84 g (4,20 mmol) 4-Phenylpyrimidin-2-ol aus Beispiel 24.2 in 8,4 ml N,N-Dimethylformamid (DMF) und 0,58 g (4,20 mmol) K₂CO₃ 1 Stunde bei Raumtemperatur. Anschließend tropfte man 0,72 g (4,20 mmol) 1-Brom-4-chlorbutan zu, rührte das Reaktionsgemisch 12 Stunden bei Raumtemperatur, filtrierte das Reaktionsgemisch und engte ein. Danach nahm man den Rückstand in Toluol auf, engte ein, nahm erneut den Rückstand in Toluol auf und engte ein. Den erhaltenen Rückstand verrührte man mit Pentan und filtrierte. Ausbeute: 0,74 g.

¹H-NMR (400 MHz, CDCl₃) δ (ppm): 8,10 (2H, d), 7,71 (1H, d), 7,63-7,40 (3H, m), 6,82 (1H, d), 3,98 (2H, t), 3,58 (2H, t), 2,00 (2H, quint.), 1,90 (2H, quint.).

### 24.4 1-(4-{4-[2-tert-Butyl-6-(trifluormethyl)pyrimidin-4-yl]piperazin-1-yl}butyl)-4-phenylpyrimidin-2(1H)-on

Analog zu Beispiel 5.2.2 erhielt man aus 0,74 g (2,82 mmol) 1-(4-Chlorbutyl)-4-phenylpyrimidin-2(1H)-on aus Beispiel 24.3 0,32 g der Titelverbindung.

ESI-MS: [M+H⁺] = 515,2, 258,1;
¹H-NMR (500 MHz, CDCl₃) δ (ppm): 8,08 (2H, d), 7,86 (1H, d), 7,52 (1H, t), 7,46 (1H, t), 6,79 (1H, d), 6,57 (1H, s), 3,99 (2H, t), 3,69 (4H, s br.), 2,49 (4H, t), 2,42 (2H, t), 1,89 (2H, quint.), 1,61 (2H, quint.), 1,33 (9H, s).

### Beispiel 25:

### 1-(4-(4-[2-tert-Butyl-6-(trifluormethyl)pyrimidin-4-yl]piperazin-1-yl)butyl)-3,5-dimethylpyrimidin-2,4(1H,3H)-dion

### 25.1 1-(4-Chlorbutyl)-3,5-dimethylpyrimidin-2,4(1H,3H)-dion

2,31 mmol (0,50 g) 1-(4-Chlorbutyl)-5-methylpyrimidin-2,4(1*H*,3*H*)-dion, 0,65 g (11,54 mmol) KOH und 1,64 g (11,54 mmol) Methylrodid wurden in 20,5 ml Dimethylsulfoxid (DMSO) 5 Stunden bei Raumtemperatur gerührt. Man versetzte das Reaktionsgemisch mit Wasser und extrahierte die wässrige Mischung dreimal mit Methyl-tert.-butylether. Danach wusch man die vereinte organische Phase dreimal mit einer gesättigten, wässrigen Kochsalzlösung, trocknete die organische Phase, filtrierte das Trockenmittel ab und engte ein. Man erhielt 0,53 g eines hellen, trüben Öls.

ESI-MS: [M+H⁺] = 231,15;

### 25.2 1-(4-{4-[2-tert-Butyl-6-(trifluormethyl)pyrimidin-4-yl]piperazin-1-yl}butyl)-3,5-dimethylpyrimidin-2,4(1H,3H)-dion

Analog zu Beispiel 5.2.2 erhielt man aus 0,53 g (2,30 mmol) 1-(4-Chlorbutyl)-3,5-dimethylpyrimidin-2,4(1H,3H)-dion aus Beispiel 25.1 0,54 g der Titelverbindung.

¹H-NMR (500 MHz, CDCl₃) δ (ppm): 6,98 (1H, s), 6,59 (1H, s), 3,76 (2H, t), 3,70 (4H, s br.), 3,37 (3H, s), 2,50 (3H, t), 2,41 (2H, t), 1,94 (3H, s), 1,74 (2H, quint.), 1,56 (2H, quint.), 1,33 (9H, s).

### Beispiel 26:

### 1-(4-{4-[2-tert-Butyl-6-(trifluormethyl)pyrimidin-4-yl]piperazin-1-yl}butyl)-5-methyl-4-phenylpyrimidin-2(1H)-on

### 26.1 2-Chlor-5-methyl-4-phenylpyrimidin

Analog zu Beispiel 24.1 erhielt man aus 1,00 g (6,13 mmol) 2,4-Dichlor-5-methyl-pyrimidin 0,57 g 2-Chlor-5-methyl-4-phenylpyrimidin.

¹H-NMR (400 MHz, CDCl₃) δ (ppm): 8,47 (1H, s), 7,57 (2H, m), 7,47 (3H, m).

### 26.2 5-Methyl-4-phenylpyrimidin-2-ol

Analog zu Beispiel 24.2 erhielt man aus 0,57 g (2,79 mmol) 2-Chlor-5-methyl-4-phenylpyrimidin aus Beispiel 26.1 in 100%iger Ausbeute 5-Methyl-4-phenylpyrimidin-2-ol.

ESI-MS: [M+H⁺] = 187,15;

### 26.3 1-(4-Chlorbutyl)-5-methyl-4-phenylpyrimidin-2(1H)-on

Analog zu Beispiel 24.3 erhielt man aus 0,52 g (2,79 mmol) 5-Methyl-4-phenylpyrimidin-2-ol aus Beispiel 26.2 0,29 g 1-(4-Chlorbutyl)-5-methyl-4-phenylpyrimidin-2(1*H*)-on.

¹H-NMR (500 MHz, CDCl₃) δ (ppm): 7,60 (2H, d), 7,46 (1H, s), 7,41 (3H, m), 3,96 (2H, t), 3,60 (2H, t), 2,14 (2H, t), 2,01 (2H, quint.), 1,89 (2H, quint.).

### 26.4 1-(4-{4-[2-tert-Butyl-6-(trifluormethyl)pyrimidin-4-yl]piperazin-1-yl}butyl)-5-methyl-4-phenylpyrimidin-2(1H)-on

Analog zu Beispiel 5.2.2 erhielt man aus 0,29 g (1,05 mmol) 1-(4-Chlorbutyl)-5-methyl-4-phenylpyrimidin-2(1*H*)-on aus Beispiel 26.3 0,14 g der Titelverbindung.

¹H-NMR (500 MHz, CDCl₃) δ (ppm): 7,61 (2H, d), 7,49-7,41 (3H, m), 6,58 (1H, s), 3,98 (2H, t), 3,70 (4H, s br.), 2,52 (4H, t), 2,43 (2H, t), 2,14 (3H, s), 1,89 (2H, quint.), 1,63 (2H, quint.), 1,37 (9H, s).

### Beispiel 27:

### 1-(4-{4-[2-tert-Butyl-6-(trifluormethyl)pyrimidin-4-yl]piperazin-1-yl}butyl)-3-methyl-pyrimidin-2,4(1H,3H)-dion

### 27.1 1-(4-Chlorbutyl)-3-methylpyrimidin-2,4(1H,3H)-dion

Analog zu Beispiel 25.1 erhielt man aus 0,50 g (2,47 mmol) 1-(4-Chlorbutyl)-4-hydroxypyrimidin-2(1H)-on (erhältlich analog zur Herstellung von 1-(4-Brombutyl)pyrimidin-2,4(1*H*,3*H*)-dion (s. Beispiel 13) 0,31 g 1-(4-Chlorbutyl)-3-methylpyrimidin-2,4(1*H*,3*H*)-dion als farbloses Öl.

ESI-MS: [M+H⁺] = 217,15.

### 27.2 1-(4-{4-[2-tert-Butyl-6-(trifluormethyl)pyrimidin-4-yl]piperazin-1-yl}butyl)-3-methylpyrimidin-2,4(1H,3H)-dion

Analog zu Beispiel 5.2.2 erhielt man aus 0,31g (1,43 mmol) 1-(4-Chlorbutyl)-3-methylpyrimidin-2,4(1H,3H)-dion aus Beispiel 27.1 0,14 g der Titelverbindung.

¹H-NMR (500 MHz, CDCl₃) δ (ppm): 7,13 (1H, d), 6,56 (1H, s), 5,76 (1H, d), 3,79 (2H, t), 3,70 (4H, s br.), 3,33 (3H, s), 2,51 (4H, t), 2,43 (2H, t), 1,78 (2H, quint.), 1,58 (2H, quint.), 1,34 (9H, s).

### Beispiel 28:

### 1-(4-{4-[2-tert-Butyl-6-(trifluormethyl)pyrimidin-4-yl]piperazin-1-yl}butyl)pyrimidin-2(1H)-on

Die Herstellung erfolgte auf die in Beispiel 14 beschrieben Weise.

### Beispiel 29:

### 1-{4-[4-(2-tert-Butyl-6-methylpyrimidin-4-yl)piperazin-1-yl]butyl}-4-hydroxy-5-methylpyrimidin-2(1H)-on

Analog zu Beispiel 5.2.2 erhielt man durch Umsetzung von 1-(4-Chlorobutyl)-4-hydroxy-5-methylpyrimidin-2(1*H*)-on (3,80 mmol, 0,82 g) mit 2-tert.-Butyl-4-piperazin-1-yl-6-trifluormethylpyrimidin die Titelverbindung; Ausbeute 0,40 g.

ESI-MS: 416.5, [M+H⁺] = 415.5;

¹H-NMR (500 MHz, CDCl₃) δ (ppm): 9,19 (1H, s br.), 6,97 (1H, s), 6,13 (1H, s), 3,73 (2H, t), 3,63 (4H, m br.), 2,51 (4H, m), 2,42 (2H, t), 2,35 (3H, s), 1,92 (3H, s), 1,74 (2H, quint.), 1,56 (2H, quint.), 1,33 (9H, s).

### Beispiel 30:

### 4-Hydroxy-5-methyl-1-{4-[4-(5,6,7,8-tetrahydronaphthalen-1-yl)piperazin-1-yl]butyl}pyrimidin-2(1H)-on

Analog zu Beispiel 5.2.2 erhielt man durch Umsetzung von 1-(4-Chlorobutyl)-4-hydroxy-5-methylpyrimidin-2(1*H*)-on (5,00 mmol, 1,08 g) mit4-(Piperazin-1-yl)-5,6,7,8-tetrahydronaphthalen die Titelverbindung; Ausbeute 0,34 g.

ESI-MS: 398,4, [M+H⁺] = 397,4, 199,3;
¹H-NMR (500 MHz, CDCl₃) δ (ppm): 9,07 (1H, s br.), 7,08 (1H, t), 7,00 (1H, s), 6,87 (1H, d), 6,82 (1H, d), 3,75 (2H, t), 2,95 (4H, s), 2,79 (2H, m), 2,71 (2H, m), 2,63 (4H, s br.), 2,47 (2H, t), 1,94 (3H, s), 1,76 (2H, quint.), 1,59 (2H, quint.).

### Beispiel 31:

### 1-(4-{4-[2-tert-Butyl-6-(trifluoromethyl)pyrimidin-4-yl]piperazin-1-yl}butyl)-4,6-dimethylpyrimidin-2(1H)-on

Analog zu Beispiel 5.2.2 erhielt man durch Umsetzung von 1-(4-Brombutyl)-4,6-dimethylpyrimidin-2(1*H*)-one (1,54 mmol, 0,40 g, hergestellt aus 4,6-Dimethyl-2-hydroxypyrimidin analog zu J. Am. Chem. Soc. 1993, 115, 7643 via 4,6-Dimethyl-2-[(trimethylsilyl)oxy]pyrimidine) mit 2-tert.-Butyl-4-piperazin-1-yl-6-trifluormethylpyrimidin die Titelverbindung.

ESI-MS: 290,3, [M+H⁺] = 289,2, 165,6;

### Beispiel 32:

### 1-[4-(4-tert-Butyl-2-oxopyrimidin-1(2H)-yl)butyl]-4-(2,6-di-tert-butylpyrimidin-4-yl)piperazin-1-iumchlorid

Analog zu Beispiel 5.2.2 erhielt man durch Umsetzung von 4-*tert-*Butyl-1-(4-chlorobutyl)pyrimidin-2(1*H*)-on (2,50 mmol, 0,61 g, siehe Beispiel 14.1) mit 2,6-Di-*tert-*butyl-4-(piperazin-1-yl)pyrimidin die Titelverbindung; Ausbeute 0,29 g.

ESI-MS: 484,4, [M+H⁺] = 483,4, 242,3;
¹H-NMR (400 MHz, CDCl₃) δ (ppm): 7,52 (1H, d), 6,37 (1H, d), 6,26 (1H, s), 3,93 (2H, t), 3,64 (4H, m), 2,50 (4H, m), 2,40 (2H, t), 1,83 (2H, quint.), 1,56 (2H, quint.), 1,39-1,17 (27H, m).

### Beispiel 33:

### 1-{4-[4-(2,6-di-tert-Butylpyrimidin-4-yl)piperazin-1-yl]butyl}pyrimidine-2,4(1H,3H)-dion

Analog zu Beispiel 5.2.2 erhielt man durch Umsetzung von 1-(4-Bromobutyl)pyrimidine-2,4(1H,3H)-dione (2,50 mmol, 0,51 g) mit mit 2,6-Di-*tert*-butyl-4-(piperazin-1-yl)pyrimidin die Titelverbindung; Ausbeute 0,45 g.

ESI-MS: 444,4, [M+H⁺] = 443,2, 222,1;
¹H-NMR (400 MHz, CDCl₃) δ (ppm): 8,86 (1H, s br.), 7,15 (1H, d), 6,25 (1H, s), 5,68 (1H, d), 3,74 (2H, t), 3,63 (4H, m), 2,51 (4H, m), 2,40 (2H, t), 1,75 (2H, quint.), 1,57 (2H, quint.), 1,33 (9H, s), 1,27 (9H, s).

### Beispiel 34:

### 4-(2,6-di-tert-Butylpyrimidin-4-yl)-1-[4-(4-methyl-2-oxopyrimidin-1 (2H)-yl)butyl]piperazin-1-iumchlorid

Analog zu Beispiel 5.2.2 erhielt man durch Umsetzung von 1-(4-Chlorobutyl)-4-methylpyrimidin-2(1H)-on (2,50 mmol, 0,50 g) mit 2,6-Di-tert-butyl-4-(piperazin-1 - yl)pyrimidin die Titelverbindung; Ausbeute 0,25 g.

ESI-MS: 442,3, [M+H⁺] = 441,2, 221,1;

### Beispiel 35:

### 4-(2-tert-Butyl-6-propylpyrimidin-4-yl)-1-[4-(4-methyl-2-oxopyrimidin-1(2H)-yl)butyl]piperazin-1-ium chlorid

Analog zu Beispiel 5.2.2 erhielt man durch Umsetzung von 1-(4-Chlorobutyl)-4-methylpyrimidin-2(1*H*)-on (2,50 mmol, 0,50 g) mit 2-*tert*-Butyl-4-(piperazin-1-yl)-6-propylpyrimidin die Titelverbindung; Ausbeute 0,22 g.

¹H-NMR (400 MHz, CDCl₃) δ (ppm): 7,48 (1H, d), 6,19 (1H, d), 6,12 (1H, s), 3,88 (2H, t), 3,60 (4H, m), 2,50 (2H, t), 2,47-2,42 (4H, m), 2,33 (5H, m), 1,88-1,62 (4H, m), 1,55 (2H, quint.), 1,30 (9H, s), 0,94 (3H, t).

### Beispiel 36:

### 1-((2E)-4-{4-[2-tert-Butyl-6-(trifluoromethyl)pyrimidin-4-yl]piperazin-1-yl}-2-methylbut-2-en-1-yl)-5-methylpyrimidin-2,4(1H,3H)-dion

Analog zu Beispiel 5.2.2 erhielt man durch Umsetzung von Thymin (3,50 mmol, 0,44 g) mit mit 2-tert-Butyl-4-{4-[(2E)-4-chlor-3-methylbut-2-en-1-yl]piperazin-1-yl}-6-trifluormethylpyrimidin (3,50 mmol, 1,50 g, hergestellt wie in DE 19735410) die Titelverbindung; Ausbeute 0,78 g.

ESI-MS: 482,2, [M+H⁺] = 481,2, 241,1;

### Beispiel 37:

### 1-(4-{4-[2-tert-Butyl-6-(trifluoromethyl)pyrimidin-4-yl]piperazin-1-yl}-2-methylbutyl)-5-methylpyrimidine-2,4(1H,3H)-dion

1-((2*E*)-4-{4-[2-*tert*-Butyl-6-(trifluoromethyl)pyrimidin-4-yl]piperazin-1-yl}-2-methylbut-2-en-1-yl)-5-methylpyrimidin-2,4(1H,3H)-dione (Beispiel 36, 1,04 mmol, 0,50 g) in Methanol (5mL) wurde über Pd (10% auf Aktivkohle) mit Wasserstoff 12 h bei Raumtemperatur, und anschließend weitere 6 h bei 40°C hydriert. Der Ansatz wurde abfiltriert und der Rückstand mit Methanol nachgewaschen. Das Filtrat wurde eingeengt und an Kieselgel gereinigt (Laufmittel: Dichlormethan/Methanol: 97/3 v/v); Ausbeute 0,20 g.

ESI-MS: 484,2, [M+H⁺] = 483,3, 242,1;

### Beispiel 38:

### 4-(2-tert-Butyl-6-propylpyrimidin-4-yl)-1-[1-methyl-4-(5-methyl-2,4-dioxo-3,4-dihydropyrimidin-1 (2H)-yl)butyl]piperazin-1-iumchlorid

Analog zu Beispiel 5.2.2. erhielt man durch Umsetzung von 1-(4-Brompentyl)-5-methylpyrimidin-2,4(1*H*,3*H*)-dion (0,47 mmol, 0,13 g, hergestellt analog zu Beispiel 6.2 aus Thymin und 1,4-Dibrompentan) mit 2-tert.-Butyl-4-piperazin-1-yl-6-propylpyrimidin die Titelverbindung; Ausbeute 0,03 g.

ESI-MS: 458,5, [M+H⁺] = 457,5, 229,3;

### Beispiel 39:

### 4-(2-tert-Butyl-6-isopropylpyrimidin-4-yl)-1 -[4-(4-methyl-2-oxopyrimidin-1 (2H)-yl)butyl]piperazin-1-iumchlorid

Analog zu Beispiel 5.2.2 erhielt man durch Umsetzung von 1-(4-Chlorobutyl)-4-methylpyrimidin-2(1*H*)-on (1,25 mmol, 0,25 g) mit 2-*tert*-Butyl-4-(piperazin-1-yl)-6-isopropylpyrimidin die Titelverbindung; Ausbeute 0,04 g.

ESI-MS: [M+H⁺] = 427,5, 214,2, 143,2

### Beispiel 40:

### 4-(2-tert-Butyl-6-isopropyl-pyrimidin-4-yl)-1-[4-(5-methyl-2,4-dioxo-3,4-dihydro-2H-pyrimidin-1-yl)-butyl]-piperazin-1-iumchlorid

Analog zu Beispiel 5.2.2 erhielt man durch Umsetzung von 1-(4-Chlorbutyl)-5-methyl-1H-pyrimidin-2,4-dion (1,00 mmol, 0,22 g) mit 2-*tert*-Butyl-4-isopropyl-6-piperazin-1-yl-pyrimidin (0,95 mmol, 0,25 g; hergestellt wie in DE 19735410 beschrieben) 0,22 g der Titelverbindung.

ESI-MS: [M+H⁺] = 443,5, 222,4.

### Beispiel 41:

### 1-{4-[4-(2-tert-Butyl-6-propyl-pyrimidin-4-yl)-piperazin-1-yl]-butyl}-4-trifluormethyl-1H-pyrimidin-2-on

Analog zu Beispiel 5.2.2 erhielt man durch Umsetzung von 1-(4-Chlorbutyl)-4-trifluormethyl-1H-pyrimidin-2-on aus Beispiel 15.1 (0,98 mmol, 0,25 g) mit 2-*tert*-Butyl-4-piperazin-1-yl-6-propyl-pyrimidin (0,98 mmol, 0,26 g; hergestellt wie in DE 19735410 beschrieben) 0,21 g der Titelverbindung.

ESI-MS: [M+H⁺] = 481,4, 241,1.

### Beispiel 42:

### 1-{4-[4-(2-tert-Butyl-6-propyl-pyrimidin-4-yl)-piperazin-1-yl]-butyl}-3-methyl-1H-pyrimidin-2,4-dion (BSF 4105765, 1044-138)

Analog zu Beispiel 5.2.2 erhielt man durch Umsetzung von 1-(4-Chlorbutyl)-3-methyl-1H-pyrimidin-2,4-dion aus Beispiel 27.1 (0,92 mmol, 0,20 g) mit 2*-tert* Butyl-4-piperazin-1-yl-6-propyl-pyrimidin (0,92 mmol, 0,24 g; hergestellt wie in DE 19735410 beschrieben) 0,19 g der Titelverbindung.

ESI-MS: [M+H⁺] = 443,4, 222,2.

### Beispiel 43:

### 4-(4-Fluorphenyl)-1-[4-(4-methylpiperazin-1-yl)-butyl]-1H-pyrimidin-2-on

### 43.1 2-Chlor-4-(4-fluorphenyl)-pyrimidin

Analog zu Beispiel 24.1 erhielt man aus 2,4-Dichlorpyrimidin (40,00 mmol, 5,96 g) und 4-Fluorphenylboronsäure (40,00 mmol, 5,60 g) 6,73 g 2-Chlor-4-(4-fluorphenyl)-pyrimidin.

### 43.2 4-(4-Fluorphenyl)-pyrimidin-2-ol

Analog zu Beispiel 24.2 erhielt man aus 2-Chlor-4-(4-fluorphenyl)-pyrimidin aus Beispiel 43.1 (32,26 mmol, 6,73 g) 6,27 g 4-(4-Fluorphenyl)-pyrimidin-2-ol.

ESI-MS:[M+K⁺] = 228,9, [M+H⁺] = 191,1.

### 43.3 1-(4-Chlorbutyl)-4-(4-fluorphenyl)-1H-pyrimidin-2-on

Analog zu Beispiel 24.3 erhielt man durch Umsetzung von 4-(4-Fluorphenyl)-pyrimidin-2-ol aus Beispiel 43.2 (15,77 mmol, 3,00 g) mit 1-Brom-4-chlorbutan 1,00 g 1-(4-Chlorbutyl)-4-(4-fluorphenyl)-1H-pyrimidin-2-on.

ESI-MS: 321,0, [M+K⁺] = 319,0, 283,0, [M+H⁺] = 281,0.

### 43.4 4-(4-Fluorphenyl)-1-[4-(4-methyl-piperazin-1-yl)-butyl]-1H-pyrimidin-2-on

Analog zu Beispiel 5.2.2 erhielt man durch Umsetzung von 1-(4-Chlorbutyl)-4-(4-fluorphenyl)-1H-pyrimidin-2-on aus Beispiel 43.3 (0,61 mmol, 0,17 g) mit 1-Methylpiperazin (0,57 mmol, 0,06 g) 0,08 g der Titelverbindung.

ESI-MS: 346,1, [M+H⁺] = 345,2, 173,1.

### Beispiel 44:

### 1-{4-[4-(2-tert-Butyl-6-propyl-pyrimidin-4-yl)-piperazin-1-yl]-butyl}-4-(4-fluorphenyl)-1H-pyrimidin-2-on

Analog zu Beispiel 5.2.2 erhielt man durch Umsetzung von 1-(4-Chlorbutyl)-4-(4-fluorphenyl)-1H-pyrimidin-2-on aus Beispiel 43.3 (0,61 mmol, 0,17 g) mit 2-*tert*-Butyl-4-piperazin-1-yl-6-propyl-pyrimidin (0,74 mmol, 0,20 g; hergestellt wie in DE 19735410 beschrieben) 0,29 g der Titelverbindung.

ESI-MS: [M+H⁺] = 507,4, 254,1.

### Beispiel 45:

### 4-(2-tert-Butyl-6-trifluormethyl-pyrimidin-4-yl)-1-{4-[4-(4-fluorphenyl)-2-oxo-2H-pyrimidin-1-yl]-butyl}-piperazin-1-iumchlorid

Analog zu Beispiel 5.2.2 erhielt man durch Umsetzung von 1-(4-Chlorbutyl)-4-(4-fluorphenyl)-1H-pyrimidin-2-on aus Beispiel 43.3 (0,40 mmol, 0,11 g) mit 2-*tert*-Butyl-4-piperazin-1-yl-6-trifluormethyl-pyrimidin (0,38 mmol, 0,11 g; hergestellt wie in DE 19735410 beschrieben) 0,13 g der Titelverbindung.

ESI-MS: [M+H⁺] = 533,3, 267,1.

### Beispiel 46:

### 1-(2E)-{4-[4-(2-tert-Butyl-6-trifluormethyl-pyrimidin-4-yl)-piperazin-1-yl]-2-methyl-but-2-enyl}-1H-pyrimidin-2,4-dion

Analog zu Beispiel 5.2.2 erhielt man durch Umsetzung von Uracil (0,60 mmol, 0,07 g) mit 4-(2-*tert*-Butyl-6-trifluormethyl-pyrimidin-4-yl)-1-(4-chlor-3-methyl-but-2-enyl)-piperazin-1-ium-chlorid (0,60 mmol, 0,26 g, hergestellt wie in DE 19735410 beschrieben) 0,14 g der Titelverbindung.

ESI-MS: [M+H⁺] = 467,3, 234,1;

### Beispiel 47:

### 1-{4-[4-(2-tert-Butyl-6-propyl-pyrimidin-4-yl)-piperazin-1-yl]-butyl}-2,4-dioxo-1,2,3,4-tetrahydro-pyrimidin-5-carbonitril

Analog zu Beispiel 5.2.2 erhielt man durch Umsetzung von 1-(4-Chlorbutyl)-2,4-dioxo-1,2,3,4-tetrahydropyrimidin-5-carbonitril aus Beispiel 18.1 (0,31 mmol, 0,07 g) mit 2-*tert-*Butyt-4-piperazin-1-yl-6-propyl-pyrimidin (0,31 mmol, 0,08 g; hergestellt wie in DE 19735410 beschrieben) 27,0 mg der Titelverbindung.

ESI-MS: 455,3, [M+H⁺] = 454,2, 227,6.

### Beispiel 48:

### 4-Azetidin-1-yl-1-{4-[4-(2-tert-butyl-6-trifluormethyl-pyrimidin-4-yl)-piperazin-1-yl]-butyl}-5-methyl-1H-pyrimidin-2-on

Man rührte ein Gemisch aus 1-{4-[4-(2-tert-Butyl-6-trifluormethyl-pyrimidin-4-yl)-piperazin-1-yl]-butyl}-5-methyl-4-thioxo-3,4-dihydro-1H-pyrimidin-2-on aus Beispiel 2 (0,24 mmol, 0,12 g) und Azetidin (5,81 mmol, 0,33 g) in Ethanol (1,9 ml) 1 Stunde bei 60 °C in der Mikrowelle (Milestone Ethos 1600). Danach engte man das Reaktionsgemisch ein und reinigte den erhaltenen Rückstand durch Säulenchromatographie an Kieselgel (Eluierungsmittel: CH₂Cl₂/Methanol 95/5), wobei man 0,08 g der Titelverbindung erhielt.

ESI-MS: 509,2, [M+H⁺] = 508,3, 254,6.

### Beispiel 49:

### 1-{4-[4-(2-tert-Butyl-6-propyl-pyrimidin-4-yl)-piperazin-1-yl]-butyl}-4-phenyl-1H-pyrimidin-2-on

Analog zu Beispiel 5.2.2 erhielt man durch Umsetzung von 1-(4-Chlorbutyl)-4-phenyl-pyrimidin-2(1*H*)-on aus Beispiel 24.3 (1,13 mmol, 0,3 g) mit 2-*tert*-Butyl-4-piperazin-1-yl-6-propyl-pyrimidin (1,13 mmol, 0,3 g; hergestellt wie in DE 19735410 beschrieben) 0,30 g der Titelverbindung.

ESI-MS: 490,4, 489,4, 245,1.

### Beispiel 50:

### 1-{4-[4-(2-tert-Butyl-6-propyl-pyrimidin-4-yl)-piperazin-1-yl]-butyl}-4-furan-2-yl-1H-pyrimidin-2-on

### 50.1 2-Chlor-4-furan-2-yl-pyrimidin

Analog zu Beispiel 24.1 erhielt man durch Umsetzung von 2,4-Dichlorpyrimidin (33,56 mmol, 5,00 g) mit Furan-2-boronsäure (33,56 mmol, 3,76 g) 3,87 g 2-Chlor-4-furan-2-yl-pyrimidin.

ESI-MS: 183,1, [M+H⁺] = 181,1.

### 50.2 4-Furan-2-yl-pyrimidin-2-ol

Analog zu Beispiel 24.2 erhielt man aus 2-Chlor-4-furan-2-yl-pyrimidin aus Beispiel 50.1 (21,43 mmol, 3,87 g) 4,16 g 4-Furan-2-yl-pyrimidin-2-ol.

ESI-MS: [2M+Na⁺] = 347,0, [M+H⁺] = 163,1.

### 50.3 1-(4-Chlorbutyl)-4-furan-2-yl-1H-pyrimidin-2-on

Analog zu Beispiel 24.3 erhielt man aus 4-Furan-2-yl-pyrimidin-2-ol aus Beispiel 50.2 (12,83 mmol, 2,08 g) 0,61 g 1-(4-Chlorbutyl)-4-furan-2-yl-1H-pyrimidin-2-on.

ESI-MS: 255,1, [M+H⁺] = 253,1.

### 50.4 1-{4-[4-(2-tert Butyl-6-propyl-pyrimidin-4-yl)-piperazin-1-yl]-butyl}-4-furan-2-yl-1H-pyrimidin-2-on

Analog zu Beispiel 5.2.2 erhielt man durch Umsetzung von 1-(4-Chlorbutyl)-4-furan-2-yl-1H-pyrimidin-2-on aus Beispiel 50.3 (1,19 mmol, 0,30 g) mit 2-*tert*-Butyl-4-piperazin-1-yl-6-propyl-pyrimidin (1,19 mmol, 0,31 g; hergestellt wie in DE 19735410 beschrieben) 0,14 g der Titelverbindung.

ESI-MS: [M+H⁺] = 479,4, 240,1.

### Beispiel 51:

### 1-{4-[4-(2-tert-Butyl-6-trifluormethyl-pyrimidin-4-yl)-piperazin-1-yl]-butyl}-4-furan-2-yl-1H-pyrimidin-2-on

Analog zu Beispiel 5.2.2 erhielt man durch Umsetzung von 1-(4-Chlorbutyl)-4-furan-2-yl-1H-pyrimidin-2-on aus Beispiel 50.3 (1,19 mmol, 0,30 g) mit 2-*tert*-Butyl-4-piperazin-1-yl-6-trifluormethyl-pyrimidin (1,18 mmol, 0,34 g; hergestellt wie in DE 19735410 beschrieben) 0,29 g der Titelverbindung.

ESI-MS: 506,2, [M+H⁺] = 505,2, 253,1.

### Beispiel 52:

### 1-{4-[4-(2-tert-Butyl-6-trifluormethyl-pyrimidin-4-yl)-piperazin-1-yl]-butyl}-5-chlor-1H-pyrimidin-2,4-dion

### 52.1 5-Chlor-1-(4-chlorbutyl)-1H-pyrimidin-2,4-dion

Analog zu Beispiel 18.1 erhielt man aus 5-Chloruracil (34,12 mmol, 5,00 g) und 1-Brom-4-chlorbutan 1,75 g der Titelverbindung.

ESI-MS: 239,1, [M+H⁺] = 237,1.

### 52.2 1-{4-[4-(2-tert-Butyl-6-trifluormethyl-pyrimidin-4-yl)-piperazin-1-yl]-butyl}-5-chlor-1H-pyrimidin-2,4-dion

Analog zu Beispiel 5.2.2 erhielt man durch Umsetzung von 5-Chlor-1-(4-chlorbutyl)-1H-pyrimidin-2,4-dion aus Beispiel 52.1 (0,84 mmol, 0,20 g) mit 2-*tert*-Butyl-4-piperazin-1-yl-6-trifluormethyl-pyrimidin (0,85 mmol, 0,25 g; hergestellt wie in DE 19735410 beschrieben) 0,10 g der Titelverbindung.

ESI-MS: 492,3, [M+H⁺] = 489,2, 245,1.

### Beispiel 53:

### 1-{4-[4-(2-tert-Butyl-6-propyl-pyrimidin-4-yl)-piperazin-1-yl]-butyl}-5-fluor-1H-pyrimidin-2,4-dion

Analog zu Beispiel 5.2.2 erhielt man durch Umsetzung von 1-(4-Chlorbutyl)-5-fluor-1H-pyrimidin-2,4-dion aus Beispiel 16.1 (2,27 mmol, 0,50 g) mit 2-*tert*-Butyl-4-piperazin-1-yl-6-propyl-pyrimidin (2,27 mmol, 0,60 g; hergestellt wie in DE 19735410 beschrieben) 0,50 g der Titelverbindung.

¹H-NMR (400 MHz, DMSO-d₆) δ (ppm): 8,08 (1H, d), 6,41 (1H, s), 3,63 (2H, t), 3,56 (4H, s br.), 2,46 (2H, t), 2,38 (4H, s br.), 2,30 (2H, t), 1,60 (4H, m), 1,43 (2H, m), 1,24 (9H, s), 0,89 (3H, t).

### Beispiel 54:

### 1-{4-[4-(2-tert-Butyl-6-trifluormethyl-pyrimidin-4-yl)-piperazin-1-yl]-pentyl}-5-methyl-1H-pyrimidin-2,4-dion

Analog zu Beispiel 5.2.2 erhielt man durch Umsetzung von 1-(4-Brompentyl)-5-methyl-1H-pyrimidin-2,4-dion (1,82 mmol, 0,50 g; hergestellt analog zu Beispiel 6.2 aus Thymin und 1,4-Dibrompentan) mit 2-*tert*-Butyl-4-piperazin-1-yl-6-trifluormethyl-pyrimidin (1,73 mmol, 0,50 g; hergestellt wie in DE 19735410 beschrieben) 0,29 g der Titelverbindung.

ESI-MS: 484,2, [M+H⁺] = 483,3, 242,1.

### Beispiel 55:

### 1-{4-[4-(2-tert-Butyl-6-trifluormethyl-pyrimidin-4-yl)-piperazin-1-yl]-butyl}-5-trifluormethyl-1H-pyrimidin-2,4-dion

### 55.1 1-(4-Chlorbutyl)-5-trifluormethyl-1H-pyrimidin-2,4-dion

Analog zu Beispiel 18.1 erhielt man durch Umsetzung von 5-Trifluormethyl-1H-pyrimidin-2,4-dion (5,28 mmol, 0,98 g) mit 1-Brom-4-chlorbutan 0,42 g 1-(4-Chlorbutyl)-5-trifluormethyl-1H-pyrimidin-2,4-dion.

ESI-MS: 311,0, [M+K⁺] = 309,0, 273,0, [M+H⁺] = 271,0.

### 55.2 1-{4-[4-(2-tert-Butyl-6-trifluormethyl-pyrimidin-4-yl)-piperazin-1-yl]-butyl}-5-trifluormethyl-1H-pyrimidin-2,4-dion

Analog zu Beispiel 5.2.2 erhielt man durch Umsetzung von 1-(4-Chlorbutyl)-5-trifluormethyl-1H-pyrimidin-2,4-dion aus Beispiel 55.1 (0,55 mmol, 0,15 g) mit 2-*tert-*Butyl-4-piperazin-1-yl-6-trifluormethyl-pyrimidin (0,53 mmol, 0,15 g; hergestellt wie in DE 19735410 beschrieben) 75,0 mg der Titelverbindung.

ESI-MS: 524,3, [M+H⁺] = 523,2, 262,1.

### Beispiel 56:

### 1-{4-[4-(2-tert-Butyl-6-propyl-pyrimidin-4-yl)-piperazin-1-yl]-butyl}-5-trifluormethyl-1H-pyrimidin-2,4-dion

Analog zu Beispiel 5.2.2 erhielt man durch Umsetzung von 1-(4-Chlorbutyl)-5-trifluormethyl-1H-pyrimidin-2,4-dion aus Beispiel 55.1 (0,55 mmol, 0,15 g) mit 2-*tert-*Butyl-4-piperazin-1-yl-6-propyl-pyrimidin (0,53 mmol, 0,14 g; hergestellt wie in DE 19735410 beschrieben) 40,0 mg der Titelverbindung.

ESI-MS: 498,2, [M+H⁺] = 497,2, 249,1.

### Beispiel 57:

### 4-(2-tert-Butyl-6-propyl-pyrimidin-4-yl)-1-[4-(2-oxo-4-(o-tolyl)-2H-pyrimidin-1-yl)-butyl]-piperazin-1-ium-Fumarat

### 57.1 2-Chlor-4-(o-tolyl)-pyrimidin

Analog zu Beispiel 24.1 erhielt man aus 2,4-Dichlorpyrimidin (13,42 mmol, 2,00 g) und o-Toluolboronsäure (13,42 mmol, 1,83 g) 3,10 g 2-Chlor-4-(o-tolyl)-pyrimidin.

### 57.2 4-(o-Tolyl)-1H-pyrimidin-2-on

Analog zu Beispiel 24.2 erhielt man unter Verwendung von 2-Chlor-4-(o-tolyl)-pyrimidin aus Beispiel 57.1 (0,49 mmol, 0,10 g) 50,0 mg 4-(o-tolyl)-1H-pyrimidin-2-on.

ESI-MS: [M+H⁺] = 187,1.

### 57.3 1-(4-Chlorbutyl)-4-(o-tolyl)-1H-pyrimidin-2-on

Analog zu Beispiel 24.3 erhielt man durch Umsetzung von 4-(o-Totyl)-1H-pyrimidin-2-on (7,30 mmol, 1,36 g) mit 1-Brom-4-chlorbutan 1,54 g 1-(4-Chlorbutyl)-4-(o-tolyl)-1H-pyrimidin-2-on.

ESI-MS: 279,0, [M+H⁺] = 277,0.

### 57.4 4-(2-tert-Butyl-6-propyl-pyrimidin-4-yl)-1-[4-(2-oxo-4-(o-tolyl)-2H-pyrimidin-1-yl)-butyl]-piperazin-1-ium-Fumarat

Analog zu Beispiel 5.2.2 erhielt man durch Umsetzung von 1-(4-Chlorbutyl)-4-(o-tolyl)-1H-pyrimidin-2-on aus Beispiel 57.3 (0,72 mmol, 0,20 g) mit 2*-tert* Butyl-4-piperazin-1-yl-6-propyl-pyrimidin (0,69 mmol, 0,18 g; hergestellt wie in DE 19735410 beschrieben) 0,15 g der Titelverbindung.

ESI-MS: [M+H⁺] = 503,4, 252,1.

### Beispiel 58:

### 1-{4-[4-(2-tert-Butyl-6-trifluormethyl-pyrimidin-4-yl)-piperazin-1-yl]-butyl}-4-(m-tolyl)-1H-pyrimidin-2-on

### 58.1 2-Chlor-4-(m-tolyl)-pyrimidin

Analog zu Beispiel 24.1 erhielt man durch Umsetzung von 2,4-Dichlorpyrimidin (20,14 mmol, 3,00 g) mit 4-m-Tolylboronsäure (20,14 mmol, 2,74 g) 4,70 g 2-Chlor-4-(m-tolyl)-pyrimidin.

### 58.2 4-(m-Tolyl)-1H-pyrimidin-2-on

Analog zu Beispiel 24.2 erhielt man ausgehend von 2-Chlor-4-(m-tolyl)-pyrimidin aus Beispiel 58.1 (14,66 mmol, 3,00 g) 1,34 g 4-(m-Tolyl)-1H-pyrimidin-2-on.

ESI-MS: [M+H⁺] = 187,1.

### 58.3 1-(4-Chlorbutyl)-4-(m-tolyl)-1H-pyrimidin-2-on

Analog zu Beispiel 24.3 erhielt man durch Umsetzung von 4-(m-Tolyl)-1H-pyrimidin-2-on (6,98 mmol, 1,30 g) mit 1-Brom-4-chlorbutan 1,17 g der Titelverbindung.

ESI-MS: 279,0, [M+H⁺] = 277,0;

### 58.4 1-{4-[4-(2-tert-Butyl-6-trifluormethyl-pyrimidin-4-yl)-piperazin-1-yl]-butyl}-4-(m-tolyl)-1H-pyrimidin-2-on

Analog zu Beispiel 5.2.2 erhielt man durch Umsetzung von 1-(4-Chlorbutyl)-4-(m-tolyl)-1H-pyrimidin-2-on aus Beispiel 58.3 (0,54 mmol, 0,15 g) mit 2-*tert*-Butyl-4-piperazin-1-yl-6-trifluormethyl-pyrimidin (0,40 mmol, 0,14 g; hergestellt wie in DE 19735410 beschrieben) 0,12 g der Titelverbindung.

ESI-MS: 530,3, [M+H⁺] = 529,3, 265,1.

### Beispiel 59:

### 1-{4-[4-(2-tert-Butyl-6-propyl-pyrimidin-4-yl)-piperazin-1-yl]-butyl}-4-(m-tolyl)-1H-pyrimidin-2-on

Analog zu Beispiel 5.2.2 erhielt man durch Umsetzung von 1-(4-Chlorbutyl)-4-(m-tolyl)-1H-pyrimidin-2-on aus Beispiel 58.3 (0,54 mmol, 0,15 g) mit 2-*tert*-Butyl-4-piperazin-1-yl-6-propyl-pyrimidin (0,49 mmol, 0,13 g; hergestellt wie in DE 19735410 beschrieben) 0,15 g 1-{4-[4-(2-*tert*-Butyl-6-propyl-pyrimidin-4-yl)-piperazin-1-yl]-butyl}-4-(m-tolyl)-1H-pyrimidin-2-on.

ESI-MS: [M+H⁺] = 503,4, 252,2.

### Beispiel 60:

### 4-(2-tert-Butyl-6-trifluormethyl-pyrimidin-4-yl)-1-[4-(2-oxo-4-(o-tolyl)-2H-pyrimidin-1-yl)-butyl]-piperazin-1-ium-Fumarat

Analog zu Beispiel 5.2.2 erhielt man durch Umsetzung von 1-(4-Chlorbutyl)-4-(o-tolyl)-1H-pyrimidin-2-on aus Beispiel 57.3 (0,72 mmol, 0,20 g) mit 2-*tert-*Butyl-4-piperazin-1-yl-6-trifluormethyl-pyrimidin (0,69 mmol, 0,20 g; hergestellt wie in DE 19735410 beschrieben) 0,21 g der Titelverbindung.

ESI-MS: [M+H⁺] = 529,3, 265,1.

### Beispiel 61:

### 4-(2-tert-Butyl-6-propyl-pyrimidin-4-yl)1-[4-(2,4-dioxo-3,4-dihydro-2H-pyrimidin-1-yl)-1-methyl-butyl]-piperazin-1-ium-Fumarat

### 61.1 1-(4-Brompentyl)-1H-pyrimidin-2,4-dion

Analog zu Beispiel 6.2 erhielt man durch Umsetzung von Uracil (28,55 mmol, 3,20 g) mit 1,4-Dibrompentan (29,35 mmol, 6,75 g) 1,07 g der Titelverbindung.

ESI-MS: 263,0, [M+H⁺] = 261,0.

### 61.2 4-(2-tert-Butyl-6-propyl-pyrimidin-4-yl)-1-[4-(2,4-dioxo-3,4-dihydro-2H-pyrimidin-1-yl)-1-methyl-butyl]-piperazin-1-ium-Fumarat

Analog zu Beispiel 5.2.2 erhielt man durch Umsetzung von 1-(4-Brompentyl)-1H-pyrimidin-2,4-dion aus Beispiel 61.1 (1,15 mmol, 0,30 g) mit *2-tert*-Butyl-4-piperazin-1-yl-6-propyl-pyrimidin (1,09 mmol, 0,29 g; hergestellt wie in DE 19735410 beschrieben) 0,10 g der Titelverbindung.

ESI-MS: 444,4, [M+H⁺] = 443,4, 222,1.

### Beispiel 62:

### 1-{4-[4-(2-tert-Butyl-6-trifluormethyl-pyrimidin-4-yl)-piperazin-1-yl]-pentyl}-1H-pyrimidin-2,4-dion

Analog zu Beispiel 5.2.2 erhielt man durch Umsetzung von 1-(4-Brompentyl)-1H-pyrimidin-2,4-dion aus Beispiel 61.1 (1,15 mmol, 0,30 g) mit 2-*tert*-Butyl-4-piperazin-1-yl-6-trifluormethyl-pyrimidin (1,09 mmol, 0,31 g; hergestellt wie in DE 19735410 beschrieben) 0,07 g der Titelverbindung.

ESI-MS: 470,2, [M+H]⁺ = 469,2, 235,1.

### Beispiel 63:

### (+)-1-{4-[4-(2-tert-Butyl-6-propyl-pyrimidin-4-yl)-piperazin-1-yl]-pentyl}-5-methyl-1H-pyrimidin-2,4-dion

Zur Racematspaltung gab man eine Lösung von 4-(2-*tert*-Butyl-6-propylpyrimidin-4-yl)-1-[1-methyl-4-(5-methyl-2,4-dioxo-3,4-dihydropyrimidin-1(2*H*)-yl)butyl]piperazin-1-iumchlorid aus Beispiel 38 (200 mg) in 3 ml Hexan/lsopropanol (9:1 (V/V)) auf eine chirale Säule (DAICEL ChiralPAK AD, Länge: 50 cm, innerer Durchmesser: 5 cm, Partikelgröße: 20 µ); Eluierungsmittel: Hexan/Ethanol/Triethylamin (85:15:0,1 (V/V)). Nach dem Einengen des erhaltenen Filtrats erhielt man 0,08 g der Titelverbindung.

ESI-MS: 458,4, [M+H⁺] = 457,4, 229,1;
α (20 °C, c = 2 mg/ml, CHCl₃, 1 = 1 dm): +10°.

### Beispiel 64:

### (-)-1-{4-[4-(2-tert-Butyl-6-trifluormethyl-pyrimidin-4-yl)-piperazin-1-yl]-2-methyl-butyl}-5-methyl-1H-pyrimidin-2,4-dion

Zur Racematspaltung gab man eine Lösung von 1-(4-{4-[2-*tert*-Butyl-6-(trifluormethyl)pyrimidin-4-yl]piperazin-1-yl}-2-methylbutyl)-5-methylpyrimidin-2,4(1*H*,3*H*)-dion (200 mg) aus Beispiel 37 in 3 ml Hexan/lsopropanol (9:1 (V/V)) auf eine optische Säule (DAICEL ChiralPAK AD, Länge: 50 cm, innerer Durchmesser: 5 cm, Partikelgröße: 20 µ, Eluierungsmittel: Hexan/Ethanol/Triethylamin (85:15:0,1 (V/V)). Nach dem Einengen des erhaltenen Filtrats erhielt man 0,08 g der Titelverbindung.

ESI-MS: [M+H⁺] = 483,3, 242,1;
α (20 °C, c = 2 mg/ml, CHCl₃, I = 1 dm): -25,5°.

### Beispiel 65:

### 1-(2E)-{4-[4-(2-tert-Butyl-6-trifluormethyl-pyrimidin-4-yl)piperazin-1-yl]-2-methyl-but-2-enyl}-5-methyl-1H-pyrimidin-2,4-dion

Analog zu Beispiel 5.2.2 erhielt man durch Umsetzung von 5-Methyl-1H-pyrimidin-2,4-dion (0,60 mmol, 0,08 g) und 4-(2-*tert*-Butyl-6-trifluormethyl-pyrimidin-4-yl)-1-(4-chlor-3-methyl-but-2-enyl)-piperazin-1-iumchlorid (0,60 mmol, 0,22 g; hergestellt wie in DE 19735410 beschrieben) 0,16 g der Titelverbindung.

ESI-MS: 456,4, [M+H⁺] = 455,4, 228,1.

### Beispiel 66:

### 4-(2-tert-Butyl-6-trifluormethyl-pyrimidin-4-y1)-1-[4-(2-oxo-4-(p-tolyl)-2H-pyrimidin-1-yl)-butyl]-piperazin-1-ium-Fumarat

### 66.1 2-Chlor-4-(p-tolyl)-pyrimidin

Analog zu Beispiel 24.1 erhielt man durch Umsetzung von 2,4-Dichlorpyrimidin (13,42 mmol, 2,00 g) mit p-Tolylboronsäure (13,42 mmol, 1,83 g) 3,19 g 2-Chlor-4-(p-tolyl)-pyrimidin.

### 66.2 4-(p-Tolyl)-1H-pyrimidin-2-on

Analog zu Beispiel 24.2 erhielt man aus 2-Chlor-4-(p-tolyl)-pyrimidin (15,59 mmol, 3,19 g) aus Beispiel 66.1 1,95 g der Titelverbindung.

ESI-MS: [M+H⁺] = 187,1.

### 66.3 1-(4-Chlorbutyl)-4-(p-tolyl)-1H-pyrimidin-2-on

Analog zu Beispiel 24.3 erhielt man durch Umsetzung von 4-(p-Tolyl)-1H-pyrimidin-2-on aus Beispiel 66.2 (10,47 mmol, 1,95 g) mit 1-Brom-4-chlorbutan 2,47 g 1-(4-Chlorbutyl)-4-(p-tolyl)-1H-pyrimidin-2-on.

ESI-MS: 279,1, [M+H⁺] = 277,0.

### 66.4 4-(2-tert-Butyl-6-trifluormethyl-pyrimidin-4-yl)-1-[4-(2-oxo-4-(p-tolyl)-2H-pyrimidin-1-yl)-butyl]-piperazin-1-ium-Fumarat

Analog zu Beispiel 5.2.2 erhielt man durch Umsetzung von 1-(4-Chlorbutyl)-4-(p-tolyl)-1H-pyrimidin-2-on aus Beispiel 66.3 (0,90 mmol, 0,25 g) mit 2-*tert*-Butyl-4-piperazin-1-yl-6-trifluormethyl-pyrimidin (0,81 mmol, 0,23 g; hergestellt wie in DE 19735410 beschrieben) 0,27 g der Titelverbindung.

ESI-MS: [M+H⁺] = 529,3, 265,1.

### Beispiel 67:

### 4-(2-tert-Butyl-6-trifluormethyl-pyrimidin-4-yl)-1-{4-[4-(2-fluorphenyl)-2-oxo-2H-pyrimidin-1-yl]-butyl}-piperazin-1-ium-Fumarat

### 67.1 2-Chlor-4-(2-fluorphenyl)-pyrimidin

Analog zu Beispiel 24.1 erhielt man durch Umsetzung von 2,4-Dichlorpyrimidin (13,42 mmol, 2,00 g) mit 2-Fluorphenylboronsäure (13,42 mmol, 1,88 g) 1,10 g 2-Chlor-4-(2-fluorphenyl)-pyrimidin.

ESI-MS: 211,1, [M+H⁺] = 209,1.

### 67.2 4-(2-Fluorphenyl)-pyrimidin-2-ol

Analog zu Beispiel 24.2 erhielt man aus 2-Chlor-4-(2-fluorphenyl)-pyrimidin aus Beispiel 67.1 (5,27 mmol, 1,10 g) 1,10 g 4-(2-Fluorphenyl)-pyrimidin-2-ol.

ESI-MS: [M+H⁺] = 191,1.

### 67.3 1-(4-Chlorbutyl)-4-(2-fluorphenyl)-1H-pyrimidin-2-on

Analog zu Beispiel 24.3 erhielt man durch Umsetzung von 4-(2-Fluorphenyl)-pyrimidin-2-ol (5,52 mmol, 1,05 g) aus Beispiel 67.2 mit 1-Brom-4-chlorbutan 0,48 g der Titelverbindung.

### 67.4 4-(2-tert-Butyl-6-trifluormethyl-pyrimidin-4-yl)-1-{4-[4-(2-fluorphenyl)-2-oxo-2H-pyrimidin-1-yl]-butyl}-piperazin-1-ium-Fumarat

Analog zu Beispiel 5.2.2 erhielt man durch Umsetzung von 1-(4-Chlorbutyl)-4-(2-fluorphenyl)-1H-pyrimidin-2-on aus Beispiel 67.3 (0,61 mmol, 0,20 g) mit 2-*tert*-Butyl-4-piperazin-1-yl-6-trifluormethyl-pyrimidin (0,64 mmol, 0,18 g; hergestellt wie in DE 19735410 beschrieben) 0,33 g der Titelverbindung.

ESI-MS: 534,2, [M+H⁺] = 533,3, 267,1.

### Beispiel 68:

### (+)-1-{4-[4-(2-tert-Butyl-6-trifluormethyl-pyrimidin-4-yl)-piperazin-1-yl]-2-methyl-butyl}-5-methyl-1H-pyrimidin-2,4-dion

Zur Racematspaltung gab man eine Lösung von 1-(4-{4-[2-*tert*-Butyl-6-(trifluormethyl)-pyrimidin-4-yl]piperazin-1-yl}-2-methylbutyl)-5-methylpyrimidin-2,4(1*H*,3*H*)-dion aus Beispiel 37 (200 mg) in 1,5 ml Isopropanol, 1,5 ml Hexan und 80 µl Methanol auf eine chirale Säule (DAICEL ChiralPAK AD, Länge: 50 cm, innerer Durchmesser: 5 cm, Partikelgröße:20 µ); Eluierungsmittel: Hexan/Ethanol/Triethylamin (85:15:0,1 (V/V)). Nach dem Einengen des erhaltenen Filtrats erhielt man 0,08 g der Titelverbindung.

ESI-MS: [M+H⁺] = 483,3, 242,1;
α (20 °C, c = 2 mg/ml, CHCl₃, I = 1 dm): +17°.

### Beispiel 69:

### 1-{4-[4-(2-tert-Butyl-6-propyl-pyrimidin-4-yl)-piperazin-1-yl]-butyl}-4-methyl-1H-pyrimidin-2-on

Analog zu Beispiel 5.2.2 erhielt man durch Umsetzung von 1-(4-Chlorbutyl)-4-(2-fluorphenyl)-1H-pyrimidin-2-on aus Beispiel 67.3 (0,61 mmol, 0,20 g) mit 2-*tert-*Butyl-4-piperazin-1-yl-6-propyl-pyrimidin (0,64 mmol, 0,17 g; hergestellt wie in DE 19735410 beschrieben) 0,23 g der Titelverbindung.

ESI-MS: [M+H⁺] = 507,4, 254,1.

### Beispiel 70:

### 1-{4-[4-(2-tert-Butyl-6-propyl-pyrimidin-4-yl)-piperazin-1-yl]-2-methyl-butyl}-5-methyl-1H-pyrimidin-2,4-dion

Analog zu Beispiel 37 erhielt man aus 1-{4-[4-(2-*tert*-Butyl-6-propyl-pyrimidin-4-yl)-piperazin-1-yl]-2-methyl-but-2-enyl}-5-methyl-1H-pyrimidin-2,4-dion aus Beispiel 65 (0,06 mmol, 25,0 mg) 7,5 mg der Titelverbindung.

ESI-MS: 458,4, [M+H⁺] = 457,4, 229,1.

### Beispiel 71:

### 1-{4-[4-(2-tert-Butyl-6-trifluormethyl-pyrimidin-4-yl)-piperazin-1-yl]-butyl}-4-cyclobutyl-1H-pyrimidin-2-on

### 71.1 1-Cyclobutyl-3,3-dimethoxy-propan-1-on

Analog zu Helv. Chim. Acta 2002, 85, 2926-29 (Beispiel 6, S. 2928) erhielt man durch Umsetzung von 1-Cyclobutylethanon (152,83 mmol, 15,00 g) mit Ameisensäuremethylester (308,16 mmol, 18,51 g) 11,00 g der Titelverbindung.

### 71.2 4-Cyclobutyl-1H-pyrimidin-2-on

Analog zu Beispiel 8.1 erhielt man durch Umsetzung von 1-Cyclobutyl-3,3-dimethoxypropan-1-on (63,87 mmol, 11,00 g) aus Beispiel 71.1 mit Harnstoff 0,75 g 4-Cyclobutyl-1H-pyrimidin-2-on.

### 71.3 1-(4-Chlorbutyl)-4-cyclobutyl-1H-pyrimidin-2-on

Analog zu Beispiel 5.2.1 erhielt man durch Umsetzung von 4-Cyclobutyl-1H-pyrimidin-2-on aus Beispiel 71.2 (4,99 mmol, 0,75 g) mit 1-Brom-4-chlorbutan 0,38 g der Titelverbindung.

ESI-MS: 243,1[M+H⁺] = 241,1;

### 71.4 1-{4-[4-(2-terf Butyl-6-trifluormethyl-pyrimidin-4-yl)-piperazin-1-yl]-butyl}-4-cyclobutyl-1H-pyrimidin-2-on

Analog zu Beispiel 5.2.2 erhielt man durch Umsetzung von 1-(4-Chlorbutyl)-4-cyclobutyl-1H-pyrimidin-2-on aus Beispiel 71.3 (0,75 mmol, 0,18 g) mit 2-*tert*-Butyl-4-piperazin-1-yl-6-trifluormethyl-pyrimidin (0,67 mmol, 0,19 g; hergestellt wie in DE 19735410 beschrieben) 0,19 g der Titelverbindung.

ESI-MS: [M+H⁺] = 493,4, 247,1.

### Beispiel 72:

### 4-(2-tert-Butyl-6-propyl-pyrimidin-4-yl)-1-{4-[4-(3-fluorphenyl)-2-oxo-2H-pyrimidin-1-yl]-butyl}-piperazin-1-ium-Fumarat

### 72.1 2-Chlor-4-(3-fluorphenyl)-pyrimidin

Analog zu Beispiel 24.1 erhielt man durch Umsetzung von 2,4-Dichlorpyrimidin (13,42 mmol, 2,00 g) mit 3-Fluorphenylboronsäure (13,42 mmol, 1,88 g) 1,00 g 2-Chlor-4-(3-fluorphenyl)-pyrimidin

ESI-MS: 210,9, [M+H⁺] = 208,9.

### 72.2 4-(3-Fluorphenyl)-pyrimidin-2-ol

Analog zu Beispiel 24.2 erhielt man aus 2-Chlor-4-(3-fluorphenyl)-pyrimidin aus Beispiel 72.1 (4,79 mmol, 1,00 g) 1,06 g 4-(3-Fluorphenyl)-pyrimidin-2-ol.

ESI-MS: [M+H⁺] = 191,1.

### 72.3 1-(4-Chlorbutyl)-4-(3-fluorphenyl)-1H-pyrimidin-2-on

Analog zu Beispiel 24.3 erhielt man durch Umsetzung von 4-(3-Fluorphenyl)-pyrimidin-2-ol aus Beispiel 72.2 (5,26 mmol, 1,00 g) mit 1-Brom-4-chlorbutan 0,34 g der Titelverbindung.

ESI-MS: 283,0, [M+H⁺] = 281,0.

### 72.4 4-(2-tert-Butyl-6-propyl-pyrimidin-4-yl)-1-{4-[4-(3-fluorphenyl)-2-oxo-2H-pyrimidin-1-yl]-butyl}-piperazin-1-ium-Fumarat

Analog zu Beispiel 5.2.2 erhielt man durch Umsetzung von 1-(4-Chlorbutyl)-4-(3-fluorphenyl)-1H-pyrimidin-2-on aus Beispiel 72.3 (0,61 mmol, 0,17 g) mit 2-*tert*-Butyl-4-piperazin-1-yl-6-propyl-pyrimidin (0,55 mmol, 0,14 g; hergestellt wie in DE 19735410 beschrieben) 0,11 g der Titelverbindung.

ESI-MS: [M+H⁺] = 507,4, 254,2.

### Beispiel 73:

### 4-(2-tert-Butyl-6-trifluormethyl-pyrimidin-4-yl)-1-(4-[4-(3-fluorphenyl)-2-oxo-2H-pyrimidin-1-yl]-butyl}-piperazin-1-ium-Fumarat

Analog zu Beispiel 5.2.2 erhielt man durch Umsetzung von 1-(4-Chlorbutyl)-4-(3-fluorphenyl)-1H-pyrimidin-2-on aus Beispiel 72.3 (0,61 mmol, 0,17 g) mit 2-*tert*-Butyl-4-piperazin-1-yl-6-trifluormethyl-pyrimidin (0,55 mmol, 0,16 g; hergestellt wie in DE 19735410 beschrieben) 0,16 g der Titelverbindung.

ESI-MS: [M+Na⁺] = 555,3, 534,3, [M+H⁺] = 533,3, 267,3.

### Beispiel 74:

### 1-{4-[4-(2-tert-Butyl-6-propyl-pyrimidin-4-yl)-piperazin-1-yl]-butyl}-4-cyclobutyl-1H-pyrimidin-2-on

Analog zu Beispiel 5.2.2 erhielt man durch Umsetzung von 1-(4-Chlorbutyl)-4-cyclobutyl-1H-pyrimidin-2-on aus Beispiel 71.3 (0,75 mmol, 0,18 g) mit 2-*tert-*Butyl-4-piperazin-1-yl-6-propyl-pyrimidin (0,67 mmol, 0,18 g; hergestellt wie in DE 19735410 beschrieben) 0,12 g der Titelverbindung.

ESI-MS: [M+H⁺] = 467,4, 234,1.

### Beispiel 75:

### 4-(2-tert-Butyl-6-propyl-pyrimidin-4-yl)-1-[4-(2-oxo-4-(p-tolyl)-2H-pyrimidin-1-yl)-butyl]-piperazin-1-Fumarat

Analog zu Beispiel 5.2.2 erhielt man durch Umsetzung von 1-(4-Chlorbutyl)-4-(p-tolyl)-1H-pyrimidin-2-on aus Beispiel 66.3 (0,90 mmol, 0,25 g) mit 2-*tert*-Butyl-4-piperazin-1-yl-6-propyl-pyrimidin (0,81 mmol, 0,21 g; hergestellt wie in DE 19735410 beschrieben) 0,18 g der Titelverbindung.

ESI-MS: [M+H⁺] = 503,4, 252,1.

### Beispiel 76:

### (-)-1-{4-[4-(2-tert-Butyl-6-propyl-pyrimidin-4-yl)-piperazin-1-yl]-pentyl}-5-methyl-1H-pyrimidin-2,4-dion

Zur Racematspaltung gab man eine Lösung von 4-(2-*tert*-Butyl-6-propylpyrimidin-4-yl)-1-[1-methyl-4-(5-methyl-2,4-dioxo-3,4-dihydropyrimidin-1(2*H*)-yl)butyl]piperazin-1-ium chlorid aus Beispiel 38 (200 mg) in 3 ml Hexan/Isopropanol (9:1 (V/V)) auf eine chirale Säule (DAICEL ChiralPAK AD, Länge: 50 cm, innerer Durchmesser: 5 cm, Partikelgröße:20 µ); Eluierungsmittel: Hexan/Ethanol/Triethylamin (85:15:0,1 (V/V)). Nach dem Einengen des erhaltenen Filtrats erhielt man 0,08 g der Titelverbindung.

ESI-MS: 458,4, [M+H⁺] = 457,4, 229,1;
α (20 °C, c = 2 mg/ml, CHCl₃, I = 1 dm): -22°.

### Beispiel 77:

### 4-(2-tert-Butyl-6-propyl-pyrimidin-4-yl)-1-[4-(5-chlor-2,4-dioxo-3,4-dihydro-2H-pyrimidin-1-yl)-butyl]-piperazin-1-ium-Acetat

Analog zu Beispiel 5.2.2 erhielt man durch Umsetzung von 5-Chlor-1-(4-chlor-butyl)-1H-pyrimidin-2,4-dion aus Beispiel 52.1 (0,83 mmol, 0,20 g) mit 2-*tert-*Butyl-4-piperazin-1-yl-6-propyl-pyrimidin (0,86 mmol, 0,23 g; hergestellt wie in DE 19735410 beschrieben) 65,0 mg der Titelverbindung.

¹H-NMR (400 MHz, DMSO-d₆) δ (ppm): 8,16 (1H, s), 6,43 (1H, s), 3,69 (2H, t), 3,57 (4H, s br.), 2,46 (2H, t), 2,39 (4H, s br.), 2,30 (2H, t), 1,64 (4H, m), 1,44 (2H, quint.), 1,27 (9H, s), 0,88 (3H, t).

### Beispiel 78:

### 1-{4-[4-(2,3-Dichlorphenyl)-piperazin-1-yl]-butyl}-1H-pyrimidin-2,4-dion

Analog zu Beispiel 5.2.2 erhielt man durch Umsetzung,von 1-(4-Chlorbutyl)-1H-pyrimidin-2,4-dion (hergestellt wie in J. Am. Chem. Soc. 1993, 115, 7636 beschrieben) (0,74 mmol, 0,15 g) mit 1-(2,3-Dichlor-phenyl)-piperazin (0,66 mmol, 0,15 g) 0,21 g der Titelverbindung.

ESI-MS: 399,0, [M+H⁺] = 397,0.

### Beispiel 79:

### 1-{4-[4-(2,3-Dichlorphenyl)-piperazin-1-yl]-butyl}-5-fluor-1H-pyrimidin-2,4-dion

Analog zu Beispiel 5.2.2 erhielt man durch Umsetzung von 1-(4-Chlorbutyl)-5-fluor-1H-pyrimidin-2,4-dion aus Beispiel 16.1 (0,91 mmol, 0,20 g) mit 1-(2,3-Dichlorphenyl)-piperazin (0,82 mmol, 0,19 g) 0,21 g der Titelverbindung.

ESI-MS: 417,3, [M+H⁺] = 415,3.

### Beispiel 80:

### 1-{4-[4-(2,3-Dichlorphenyl)-piperazin-1-yl]-butyl}-4-methyl-1H-pyrimidin-2-on

Analog zu Beispiel 5.2.2 erhielt man durch Umsetzung von 1-(4-Chlorbutyl)-4-methyl-1H-pyrimidin-2-on aus Beispiel 8.2 (0,37 mmol, 75,0 mg) mit 1-(2,3-Dichlorphenyl)-piperazin (0,34 mmol, 77,73 mg) 70,0 mg der Titelverbindung.

ESI-MS: 397,1, 395,1.

### Beispiel 81:

### 4-(2,3-Dichlorphenyl)-1-[4-(2-oxo-4-phenyl-2H-pyrimidin-1-yl)-butyl]-piperazin-1-ium-Fumarat

Analog zu Beispiel 5.2.2 erhielt man durch Umsetzung von 1-(4-Chlorbutyl)-4-phenyl-1H-pyrimidin-2-on aus Beispiel 24.3 (0,76 mmol, 0,20 g) mit 1-(2,3-Dichlorphenyl)-piperazin (0,69 mmol, 0,16 g) 0,16 g der Titelverbindung.

ESI-MS: 459,0, [M+H⁺] = 458,1, 457,1, 229,1.

### Beispiel 82:

### 1-{4-[4-(2,3-Dichlorphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-5-methyl-1H-pyrimidin-2,4-dion aus Beispiel 5.2.1 (0,69 mmol, 0,15 g) mit 1-(2,3-Dichlorphenyl)-piperazin (0,62 mmol, 0,14 g) 0,17 g der Titelverbindung.

ESI-MS: 427,15,425,15.

### Beispiel 83:

### 4-(2-tert-Butyl-6-trifluormethyl-pyrimidin-4-yl)-1-[4-(5-ethyl-2,4-dioxo-3,4-dihydro-2H-pyrimidin-1-yl)-butyl]-piperazin-1-Fumarat

### 83.1 1-(4-Chlorbutyl)-5-ethyl-1H-pyrimidin-2,4-dion

Analog zu Beispiel 18.1 erhielt man durch Umsetzung von 5-Ethyl-1H-pyrimidin-2,4-dion (6,42 mmol, 0,90 g) mit 1-Brom-4-chlorbutan 0,21 g 1-(4-Chlorbutyl)-5-ethyl-1H-pyrimidin-2,4-dion.

ESI-MS: 233,1, [M+H⁺] = 231,1.

### 83.2 4-(2-tert-Butyl-6-trifluormethyl-pyrimidin-4-yl)-1-[4-(5-ethyl-2,4-dioxo-3,4-dihydro-2H-pyrimidin-1-yl)-butyl]-piperazin-1-Fumarat

Analog zu Beispiel 5.2.2 erhielt man durch Umsetzung von 1-(4-Chlorbutyl)-5-ethyl-1H-pyrimidin-2,4-dion aus Beispiel 83.1 (0,35 mmol, 0,08 g) mit 2-*tert*-Butyl-4-piperazin-1-yl-6-trifluormethyl-pyrimidin (0,36 mmol, 0,11 g; hergestellt wie in DE 19735410 beschrieben) 0,14 g der Titelverbindung.

ESI-MS: 484,2, [M+H⁺] = 483,3, 242,1.

### Beispiel 84:

### 4-(2-tert-Butyl-6-propyl-pyrimidln-4-yl)-1-[4-(5-ethyl-2,4-dioxo-3,4-dihydro-2H-pyrimidin-1-yl)-butyl]-piperazin-1-ium-Fumarat

Analog zu Beispiel 5.2.2 erhielt man durch Umsetzung von 1-(4-Chlorbutyl)-5-ethyl-1H-pyrimidin-2,4-dion aus Beispiel 83.1 (0,35 mmol, 0,08 g) mit 2-*tert*-Butyl-4-piperazin-1-yl-6-propyl-pyrimidin (0,36 mmol, 0,10 g; hergestellt wie in DE 19735410 beschrieben) 0,07 g der Titelverbindung.

ESI-MS: 458,4, [M+H⁺] = 457,4, 229,1.

### Beispiel 85:

### 4-(2-tert-Butyl-6-trifluormethyl-pyrimidin-4-yl)-1-[4-(4-ethoxy-2-oxo-2H-pyrimidin-1-yl)-butyl]-piperazin-1-ium-Fumarat

### 85.1 1-(4-Chlorbutyl)-4-ethoxy-1H-pyrimidin-2-on

Analog zu Beispiel 5.2.1 erhielt man durch Umsetzung von 4-Ethoxy-1H-pyrimidin-2-on (4,17 mmol, 0,59 g) mit 1-Brom-4-chlorbutan 0,21 g 1-(4-Chlorbutyl)-4-ethoxy-1H-pyrimidin-2-on.

ESI-MS: [M+K⁺] = 269,0, 233,1, 232,1, [M+H⁺] = 231,1.

### 85.2 4-(2-tert-Butyl-6-trifluormethyl-pyrimidin-4-yl)-1-[4-(4-ethoxy-2-oxo-2H-pyrimidin-1-yl)-butyl]-piperazin-1-ium-Fumarat

Analog zu Beispiel 5.2.2 erhielt man durch Umsetzung von 1-(4-Chlorbutyl)-4-ethoxy-1H-pyrimidin-2-on aus Beispiel 85.1 (0,56 mmol, 0,13 g) mit 2-*tert*-Butyl-4-piperazin-1-yl-6-trifluormethyl-pyrimidin (0,57 mmol, 0,17 g; hergestellt wie in DE 19735410 beschrieben) 0,13 g der Titelverbindung.

ESI-MS: [M+K⁺] = 521,3, [M+H⁺] = 484,2, 483,3.

### Beispiel 86:

### 4-(2-tert-Butyl-6-propyl-pyrimidin-4-yl)-1-[4-(4-ethoxy-2-oxo-2H-pyrimidin-1-yl)-butyl]-piperazin-1-ium-Fumarat

Analog zu Beispiel 5.2.2 erhielt man durch Umsetzung von 1-(4-Chlorbutyl)-4-ethoxy-1H-pyrimidin-2-on aus Beispiel 85.1 (0,56 mmol, 0,13 g) mit 2-*tert*-Butyl-4-piperafin-1-yl-6-propyl-pyrimidin (0,57 mmol, 0,15 g; hergestellt wie in DE 19735410 beschrieben) 0,14 g der Titelverbindung.

ESI-MS: [M+Na⁺] = 495,2, 459,4, 458,4, [M+H⁺] = 457,4.

### Beispiel 87:

### 1-{4-[4-(2-tert-Butyl-6-propyl-pyrimidin-4-yl)-piperazin-1-yl]-butyl}-4-thiophen-2-yl-1H-pyrimidin-2-on

### 87.1 2-Chlor-4-thiophen-2-yl-pyrimidin

Analog zu Beispiel 24.1 erhielt man durch Umsetzung von 2,4-Dichlorpyrimidin (33,56 mmol, 5,00 g) mit Thiophen-2-boronsäure (33,56 mmol, 4,29 g) 5,16 g 2-Chlor-4-thiophen-2-yl-pyrimidin.

ESI-MS: 198,9, [M+H⁺] = 196,9.

### 87.2 4-Thiophen-2-yl-pyrimidin-2-ol

Analog zu Beispiel 24.2 erhielt man aus 2-Chlor-4-thiophen-2-yl-pyrimidin aus Beispiel 87.1 (26,24 mmol, 5,16 g) 5,40 g 4-Thiophen-2-yl-pyrimidin-2-ol.

ESI-MS: [M+H⁺] = 179,1.

### 87.3 1-(4-Chlorbutyl)-4-thiophen-2-yl-1H-pyrimidin-2-on

Analog zu Beispiel 24.3 erhielt man durch Umsetzung von 4-Thiophen-2-yl-pyrimidin-2-ol aus Beispiel 87.2 (16,83 mmol, 3,00 g) mit 1-Brom-4-chlorbutan 4,48 g 1-(4-Chlorbutyl)-4-thiophen-2-yl-1H-pyrimidin-2-on.

ESI-MS: 271,0, [M+H⁺] = 269,0.

### 87.4 1-{4-[4-(2-tert-Butyl-6-propyl-pyrimidin-4-yl)piperazin-1-yl]-butyl}-4-thiophen-2-yl-1H-pyrimidin-2-on

Analog zu Beispiel 5.2.2 erhielt man durch Umsetzung von 1-(4-Chlorbutyl)-4-thiophen-2-yl-1H-pyrimidin-2-on aus Beispiel 87.3 (1,86 mmol, 0,50 g) mit 2-*tert-*Butyl-4-piperazin-1-yl-6-propyl-pyrimidin (1,87 mmol, 0,49 g; hergestellt wie in DE 19735410 beschrieben) 0,21 g der Titelverbindung.

¹H-NMR (400 MHz, DMSO-d₆) δ (ppm): 8,22 (1H, d), 8,04 (1H, d), 7,88 (1H, d), 7,24 (1H, t), 7,01 (1H, d), 6,41 (1H, s), 3,87 (2H, t), 3,57 (4H, s br.), 2,45 (2H, t), 2,39 (4H, s br.), 2,34 (2H, t), 1,76-1,55 (4H, m), 1,46 (2H, quint.), 1,25 (9H, s), 0,89 (3H, t).

### Beispiel 88:

### 1-{4-[4-(2-tert-Butyl-6-trifluormethyl-pyrimidin-4-yl)-piperazin-1-yl]-butyl}-4-thiophen-2-yl-1H-pyrimidin-2-on

Analog zu Beispiel 5.2.2 erhielt man durch Umsetzung von 1-(4-Chlorbutyl)-4-thiophen-2-yl-1H-pyrimidin-2-on aus Beispiel 87.3 (1,86 mmol, 0,50 g) mit 2-*tert*-Butyl-4-piperazin-1-yl-6-trifluormethyl-pyrimidin (1,87 mmol, 0,54 g; hergestellt wie in DE 19735410 beschrieben) 0,21 g der Titelverbindung.

¹H-NMR (400 MHz, DMSO-d₆) δ (ppm): 8,23 (1H, d), 8,03 (1H, s), 7,88 (1H, d), 7,23 (1H, s), 7,02 (2H, s), 3,87 (2H, t), 3,70 (4H, s br.), 2,41 (4H, s br.), 2,33 (2H, t), 1,74 (2H, quint.), 1,44 (2H, quint.), 1,28 (9H, s).

### Beispiel 89:

### 1-[4-(4-Azetidin-1-yl-2-oxo-2H-pyrimidin-1-yl)-butyl]-4-(2-tert-butyl-6-trifluormethyl-pyrimidin-4-yl)-piperazin-l-ium-Fumarat

### 89.1 1-{4-[4-(2-tert-Butyl-6-trifluormethyl-pyrimidin-4-yl)-piperazin-1-yl]-butyl}-4-thioxo-3,4-dihydro-1H-pyrimidin-2-on

Ausgehend von 1-(4-{4-[2-*tert*-Butyl-6-(trifluormethyl)pyrimidin-4-yl]piperazin-1-yl}butyl)-4-hydroxypyrimidin-2(1*H*)-on aus Beispiel 13 erhielt man auf die in J. Med. Chem. 1984, 27, 1470-80, S. 1478, Beispiel 8b beschriebene Weise die Titelverbindung.

### 89.2 1-[4-(4-Azetidin-1-yl-2-oxo-2H-pyrimidin-1-yl)-butyl]-4-(2-tert-butyl-6-trifluormethyl-pyrimidin-4-yl)-piperazin-1-ium-Fumarat

Analog zu Beispiel 48 erhielt man durch Umsetzung von 1-{4-[4-(2-*tert*-Butyl-6-trifluormethyl-pyrimidin-4-yl)-piperazin-1-yl]-butyl}-4-thioxo-3,4-dihydro-1H-pyrimidin-2-on (0,21 mmol, 0,12 g) mit Azetidin (5,25 mmol, 0,30 g) 0,07 g der Titelverbindung.

ESI-MS: [M+K⁺] = 532,3, 495,2, [M+H⁺] = 494,2;

### Beispiel 90:

### 4-(2-tert-Butyl-6-trifluormethyl-pyrimidin-4-yl)-1-[4-(2-oxo-4-pyrrolidin-1-yl-2H-pyrimidin-1-yl)-butyl]-piperazin-1-ium-Fumarat

Analog zu 48 erhielt man durch Umsetzung von 1-{4-[4-(2-*tert*-Butyl-6-trifluormethyl-pyrimidin-4-yl)-piperazin-1-yl]-butyl}-4-thioxo-3,4-dihydro-1H-pyrimidin-2-on aus Beispiel 89.1 (0,21 mmol, 0,12 g) mit Pyrrolidin (5,20 mmol, 0,37 g) 0,05 g der Titelverbindung.

ESI-MS: [M+H⁺] = 508,3, 254,6.

### Beispiel 91:

### 4-(2-tert-Butyl-6-trifluorrnethyl-pyrimidin-4-yl)-1-[4-(2-oxo-4-piperidin-1-yl-2H-pyrimidin-1-yl)-butyl]-piperazin-1-lum-Fumarat

Analog zu Beispiel 48 erhielt man durch Umsetzung von 1-{4-[4-(2-*tert*-Butyl-6-trifluormethyl-pyrimidin-4-yl)-piperazin-1-yl]-butyl}-4-thioxo-3,4-dihydro-1H-pyrimidin-2-on (0,21 mmol, 0,12 g) mit Piperidin (5,05 mmol, 0,43 g) 0,06 g der Titelverbindung.

ESI-MS: [M+Na⁺] = 544,3, 523,3, [M+H⁺] = 522,3, 261,6;

### Beispiel 92:

### 4-(2-tert-Butyl-6-trifluormethyl-pyrimidin-4-yl)-1-[4-(2-oxo-4-thiophen-3-yl-2H-pyrimidin-1-yl)-butyl]-piperazin-1-ium-Fumarat

### 92.1 2-Chlor-4-thiophen-3-yl-pyrimidin

Analog zu Beispiel 24.1 erhielt man durch Umsetzung von 2,4-Dichlorpyrimidin (7,82 mmol, 1,16 g) mit Thiophen-3-boronsäure (7,82 mmol, 1,00 g) 1,25 g 2-Chlor-4-thiophen-3-yl-pyrimidin.

ESI-MS: [M+H⁺] = 196,9;

### 92.2 4-Thiophen-3-yl-1H-pyrimidin-2-on

Analog zu Beispiel 24.2 erhielt man aus 2-Chlor-4-thiophen-3-yl-pyrimidin aus Beispiel 92.1 (5,26 mmol, 1,15 g) 1,30 g 4-Thiophen-3-yl-1H-pyrimidin-2-on.

ESI-MS: [M+H⁺] = 179,1;

### 92.3 1-(4-Chlorbutyl)-4-thiophen-3-yl-1H-pyrimidin-2-on

Analog zu Beispiel 24.3 erhielt man durch Umsetzung von 4-Thiophen-3-yl-1H-pyrimidin-2-on aus Beispiel 92.2 (5,81 mmol, 1,15 g) mit 1-Brom-4-chlorbutan 0,25 g 1-(4-Chlorbutyl)-4-thiophen-3-yl-1H-pyrimidin-2-on.

ESI-MS: [M+H⁺] = 269,0.

### 92.4 4-(2-tert-Butyl-6-trifluormethyl-pyrimidin-4-yl)-1-[4-(2-oxo-4-thiophen-3-yl-2H-pyrimidin-1-yl)-butyl]-piperazin-1-ium-Fumarat

Analog zu Beispiel 5.2.2 erhielt man durch Umsetzung von 1-(4-Chlorbutyl)-4-thiophen-3-yl-1H-pyrimidin-2-on aus Beispiel 92.3 (0,37 mmol, 0,11 g) mit 2-*tert*-Butyl-4-piperazin-1-y1-6-trifluormethyl-pyrimidin (0,38 mmol, 0,11 g; hergestellt wie in DE 19735410 beschrieben) 0,12 g der Titelverbindung.

ESI-MS: 522,2, [M+H⁺] = 521,3, 261,1.

### Beispiel 93:

### 1-{4-[4-(2-tert-Butyl-6-propyl-pyrimidin-4-yl)-piperazin-1-yl]-butyl}-4-thiophen-3-yl-1H-pyrimidin-2-on

Analog zu Beispiel 5.2.2 erhielt man durch Umsetzung von 1-(4-Chlorbutyl)-4-thiophen-3-yl-1H-pyrimidin-2-on aus Beispiel 92.3 (0,40 mmol, 0,12 g) mit 2-*tert*-Butyl-4-piperazin-1-yl-6-propyl-pyrimidin (0,42 mmol, 0,11 g; hergestellt wie in DE 19735410 beschrieben) 0,08 g der Titelverbindung.

ESI-MS: [M+H⁺] = 495,4, 248,1.

### Beispiel 94:

### 4-(2-tert-Butyl-6-trifluormethyl-pyrimidin-4-yl)-1-[4-(4-cyclopropyl-2-oxo-2H-pyrimidin-1 - yl)-butyl]-piperazin-1-ium-Acetat

### 94.1 1-Cyclopropyl-3,3-dimethoxy-propan-1-on

Analog zu Helv. Chim. Acta 2002, 85, 2926-29 (Beispiel 6, S. 2928) erhielt man durch Umsetzung von 1-Cyclopropyl-methylketon (149,79 mmol, 12,60 g) mit Ameisensäuremethylester (300,0 mmol, 18,02 g) 6,50 g 1-Cyclopropyl-3,3-dimethoxy-propan-1-on.

### 94.2 4-Cyclopropyl-1H-pyrimidin-2-on .

Analog zu Beispiel 8.1 erhielt man aus 1-Cyclopropyl-3,3-dimethoxy-propan-1-on aus Beispiel 94.1 (12,64 mmol, 2,00 g) mit Harnstoff 0,11 g 4-Cyclopropyl-1H-pyrimidin-2-on.

### 94.3 1-(4-Chlorbutyl)-4-cychloropropyl-1H-pyrimidin-2-on

Analog zu Beispiel 5.2.1 erhielt man durch Umsetzung von 4-Cyclopropyl-1H-pyrimidin-2-on aus Beispiel 94.2 (0,81 mmol, 0,11 g) mit 1-Brom-4-chlorbutan 0,05 g 1-(4-Chlorbutyl)-4-cyclopropyl-1H-pyrimidin-2-on.

### 94.4 4-(2-tert-Butyl-6-trifluormethyl-pyrimidin-4-yl)-1-[4-(4-cyclopropyl-2-oxo-2H-pyrimidin-1-yl)-butyl]-piperazin-1-ium-Acetat

Analog zu Beispiel 5.2.2 erhielt man durch Umsetzung von 1-(4-Chlorbutyl)-4-cyclopropyl-1H-pyrimidin-2-on (0,22 mmol, 50,0 mg) mit 2-*tert*-Butyl-4-piperazin-1-yl-6-trifluormethyl-pyrimidin (0,21 mmol, 0,06 g; hergestellt wie in DE 19735410 beschrieben) 0,02 g der Titelverbindung.

ESI-MS: [M+Na⁺] = 501,2, 480,2, [M+H⁺] = 479,2, 240,1.

### Beispiel 95:

### 4-(2-tert-Butyl-6-trifluormethyl-pyrimidin-4-yl)-1-[4-(4-ethyl-2-oxo-2H-pyrimidin-1-yl)-butyl]-piperazin-1-ium-Fumarat

### 95.1 1,1-Dimethoxy-pentan-3-on

Analog zu Helv. Chim. Acta 2002, 85, 2926-29 (Beispiel 6, S. 2928) erhielt man durch Umsetzung von Ethylmethylketon (161,98 mmol, 11,68 g) mit Ameisensäuremethylester (324,66 mmol, 19,50 g) 6,60 g 1,1-Dimethoxy-pentan-3-on.

### 95.2 4-Ethyl-1H-pyrimidin-2-on

Analog zu Beispiel 8.1 erhielt man durch Umsetzung von 1,1-Dimethoxy-pentan-3-on aus Beispiel 95.1 (29,55 mmol, 4,80 g) mit Harnstoff 2,90 g 4-Ethyl-1H-pyrimidin-2-on.

ESI-MS: [M+H⁺] = 125,1.

### 95.3 1-(4-Chlorbutyl)-4-ethyl-1H-pyrimidin-2-on

Analog zu Beispiel 5.2.1 erhielt man durch Umsetzung von 4-Ethyl-1H-pyrimidin-2-on aus Beispiel 95.2 (23,36 mmol, 2,90 g) mit 1-Brom-4-chlorbutan 0,22 g 1-(4-Chlorbutyl)-4-ethyl-1H-pyrimidin-2-on.

ESI-MS: [M+H⁺] = 215,1.

### 95.4 4-(2-tert-Butyl-6-trifluormethyl-pyrimidin-4-yl)-1-[4-(4-ethyl-2-oxo-2H-pyrimidin-1-yl)-butyl]-piperazin-1-ium-Fumarat

Analog zu Beispiel 5.2.2 erhielt man durch Umsetzung von 1-(4-Chlorbutyl)-4-ethyl-1H-pyrimidin-2-on aus Beispiel 95.3 (0,47 mmol, 0,10 g) mit 2-*tert*-Butyl-4-piperazin-1-yl-6-trifluormethyl-pyrimidin (0,47 mmol, 0,14 g; hergestellt wie in DE 19735410 beschrieben) 0,09 g der Titelverbindung.

ESI-MS: [M+H⁺] = 467,3, 234,1.

### Beispiel 96:

### 4-(2-tert-Butyl-6-propyl-pyrimidin-4-yl)-1-[4-(4-ethyl-2-oxo-2H-pyrimidin-1-yl)-butyl]-piperazin-1-ium-Fumarat

Analog zu Beispiel 5.2.2 erhielt man durch Umsetzung von 1-(4-Chlorbutyl)-4-ethyl-1H-pyrimidin-2-on aus Beispiel 95.3 (0,33 mmol, 0,07 g) mit 2-*tert*-Butyl-4-piperazin-1-yl-6-propyl-pyrimidin (0,34 mmol, 0,09 g; hergestellt wie in DE 19735410 beschrieben) 12,0 mg der Titelverbindung.

ESI-MS: [M+H⁺] = 441,4, 221,1.

### Beispiel 97:

### 4-(2-tert-Butyl-6-trifluormethyl-pyrimidin-4-yl)-1-[4-(4-Isopropyl-2-oxo-2H-pyrimidin-1-yl)-butyl]-piperazin-1-ium-Fumarat

### 97.1 1,1-Dimethoxy-4-methyl-pentan-3-on

Analog zu Helv. Chim. Acta 2002, 85, 2926-29 (Beispiel 6, S. 2928) erhielt man durch Umsetzung von 3-Methyl-butan-2-on (186,68 mmol, 16,08 g) mit Ameisensäuremethylester (373,04 mmol, 22,40 g) 7,80 g der Titelverbindung.

### 97.2 4-Isopropyl-1H-pyrimidin-2-on

Analog zu Beispiel 8.1 erhielt man aus 1,1-Dimethoxy-4-methyl-pentan-3-on aus Beispiel 97.1 (43,69 mmol, 7,00 g) mit Harnstoff 3,30 g 4-Isopropyl-1H-pyrimidin-2-on.

ESI-MS: [M+H⁺] = 139,1;

### 97.3 1-(4-Chlorbutyl)-4-isopropyl-1H-pyrimidin-2-on

Analog zu Beispiel 5.2.1 erhielt man durch Umsetzung von 4-Isopropyl-1H-pyrimidin-2-on aus Beispiel 97.2 (14,47 mmol, 2,00 g) mit 1-Brom-4-chlorbutan 0,90 g 1-(4-Chlorbutyl)-4-isopropyl-1H-pyrimidin-2-on.

ESI-MS: 231,1, [M+H⁺] = 229,1.

### 97.4 4-(2-tert-Butyl-6-trifluormethyl-pyrimidin-4-yl)-1-[4-(4-isopropyl-2-oxo-2H-pyrimidin-1-yl)-butyl]-piperazin-1-ium-Fumarat

Analog zu Beispiel 5.2.2 erhielt man durch Umsetzung von 1-(4-Chlorbutyl)-4-isopropyl-1H-pyrimidin-2-on aus Beispiel 97.3 (1,09 mmol, 0,25 g) mit 2-*tert*-Butyl-4-piperazin-1-yl-6-trifluormethyl-pyrimidin (1,09 mmol, 0,32 g; hergestellt wie in DE 19735410 beschrieben) 0,27 g der Titelverbindung.

ESI-MS: [M+H⁺] = 481,2, 241,1;

### Beispiel 98:

### 4-(2-tert-Butyl-6-propyl-pyrimidin-4-yl)-1-[4-(4-isopropyl-2-oxo-2H-pyrimidin-1-yl)-butyl]-piperazin-1-Fumarat

Analog zu Beispiel 5.2.2 erhielt man durch Umsetzung von 1-(4-Chlorbutyl)-4-isopropyl-1H-pyrimidin-2-on aus Beispiel 97.3 (1,09 mmol, 0,25 g) mit 2-*tert*-Butyl-4-piperazin-1-yl-6-propyl-pyrimidin (1,11 mmol, 0,29 g; hergestellt wie in DE 19735410 beschrieben) 0,35 g der Titelverbindung.

ESI-MS: [M+H⁺] = 455,4, 228,1.

### Beispiel 99:

### 4-(2-tert-Butyl-6-trifluormethyl-pyrimidin-4-yl)-1-[4-(4-cyclohexyl-2-oxo-2H-pyrimidin-1-yl)-butyl]-piperazin-1-ium-Fumarat

### 99.1 1-Cyclohexyl-3,3-dimethoxy-propan-1-on

Analog zu Helv. Chim. Acta 2002, 85, 2926-29 (Beispiel 6, S. 2928) erhielt man durch Umsetzung von 1-Cyclohexyl-ethanon (150,55 mmol, 19,00 g) mit Ameisensäuremethylester (300,05 mmol, 18,02 g) 1,50 g der Titelverbindung.

### 99.2 4-Cyclohexyl-1H-pyrimidin-2-on

Analog zu Beispiel 8.1 erhielt man durch Umsetzung von 1-Cyclohexyl-3,3-dimethoxypropan-1-on aus Beispiel 99.1 (6,99 mmol, 1,40 g) mit Harnstoff 0,62 g der Titelverbindung.

ESI-MS: [M+H⁺] = 179,1.

### 99.3 1-(4-Chlorbutyl)-4-cyclohexyl-1H-pyrimidin-2-on

Analog zu Beispiel 5.2.1 erhielt man durch Umsetzung von 4-Cyclohexyl-1H-pyrimidin-2-on aus Beispiel 99.2 (2,55 mmol, 0,46 g) mit 1-Brom-4-chlorbutan 0,65 g 1-(4-Chlorbutyl)-4-cyclohexyl-1H-pyrimidin-2-on.

ESI-MS: 271,1, [M+H⁺] = 269,1.

### 99.4 4-(2-tert-Butyl-6-trifluormethyl-pyrimidin-4-yl)-1-[4-(4-cyclohexyl-2-oxo-2H-pyrimidin-1-yl)-butyl]-piperazin-1-ium-Fumarat

Analog zu Beispiel 5.2.2 erhielt man durch Umsetzung von 1-(4-Chlorbutyl)-4-cyclohexyl-1H-pyrimidin-2-on aus Beispiel 99.3 (1,12 mmol, 0,30 g) mit 2-*tert*-Butyl-4-piperazin-1-yl-6-trifluormethyl-pyrimidin (1,14 mmol, 0,33 g; hergestellt wie in DE 19735410 beschrieben) 0,40 g der Titelverbindung.

ESI-MS: [M+H⁺] = 521,3, 261,1.

### Beispiel 100:

### 4-(2-tert-Butyl-6-propyl-pyrimidin-4-yl)-1-[4-(4-cyclohexyl-2-oxo-2H-pyrimidin-1-yl)-butyl]-piperazin-1-ium-Fumarat

Analog zu Beispiel 5.2.2 erhielt man durch Umsetzung von 1-(4-Chlorbutyl)-4-cyclohexyl-1H-pyrimidin-2-on aus Beispiel 99.3 (0,82 mmol, 0,22 g) mit 2-*tert*-Butyl-4-piperazin-1-yl-6-propyl-pyrimidin (0,82 mmol, 0,22 g; hergestellt wie in DE 19735410 beschrieben) 0,23 g der Titelverbindung.

ESI-MS: [M+H⁺] = 495,4, 248,3.

### Beispiel 101:

### 1-{4-[4-(2-tert-Butyl-6-trifluormethyl-pyrimidin-4-yl)-piperazin-1-yl]-butyl}-5-phenyl-1H-pyrimidin-2-on

### 101.15-Phenyl-1H-pyrimidin-2-on

Analog zu Vorschrift in Tetrahedron 1997, 53, 14437-50 erhielt man aus 5-Brom-1H-pyrimidin-2-on (2,29 mmol, 0,40 g) 40,0 mg 5-Phenyl-1H-pyrimidin-2-on.

ESI-MS: [M+H⁺] = 173,1.

### 101.21-(4-Chlorbutyl)-5-phenyl-1H-pyrimidin-2-on

Analog zu Beispiel 5.2.1 erhielt man durch Umsetzung von 5-Phenyl-1H-pyrimidin-2-on aus Beispiel 101.1 (0,23 mmol, 0,04 g) mit 1-Brom-4-chlorbutan 0,03 g 1-(4-Chlorbutyl)-5-phenyl-1H-pyrimidin-2-on.

ESI-MS: [M+H⁺] = 263,1.

### 101.31-{4-[4-(2-tert-Butyl-6-trifluormethyl-pyrimidin-4-yl)-piperazin-1-yl]-butyl}-5-phenyl-1H-pyrimidin-2-on

Analog zu Beispiel 5.2.2 erhielt man durch Umsetzung von 1-(4-Chlorbutyl)-5-phenyl-1H-pyrimidin-2-on aus Beispiel 101.3 (0,10 mmol, 0,03 g) mit 2-*tert*-Butyl-4-piperazin-1-yl-6-trifluormethyl-pyrimidin (0,09 mmol, 0,03 g; hergestellt wie in DE 19735410 beschrieben) 8,0 mg der Titelverbindung.

ESI-MS: [M+H⁺] = 515,3, 258,2.

### Beispiel 102:

### 1-{4-[4-(3,4-Dimethoxyphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(3,4-Dimethoxyphenyl)-piperazin die Titelverbindung.

ESI-MS: [M+H⁺] = 403,2.

### Beispiel 103:

### 1-{4-[4-(2-Fluorphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(2-Fluorphenyl)-piperazin die Titelverbindung.

ESI-MS: [M+H⁺] = 361,1.

### Beispiel 104:

### 1-{4-[4-(4-Fluorphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(4-Fluorphenyl)-piperazin die Titelverbindung.

ESI-MS: [M+H⁺] = 361,0.

### Beispiel 105:

### 1-{4-[4-(2-Methoxyphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(2-Methoxyphenyl)-piperazin die Titelverbindung.

ESI-MS: [M+H⁺] = 373,0.

### Beispiel 106:

### 1-{4-[4-(4-Methoxyphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(4-Methoxyphenyl)-piperazin die Titelverbindung.

ESI-MS: [M+H⁺] = 373,1.

### Beispiel 107:

### 1-{4-[4-(2-Ethoxyphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(2-Ethoxyphenyt)-piperazin die Titelverbindung.

ESI-MS: [M+H⁺] = 387,0, 234,9.

### Beispiel 108:

### 2-{4-[4-(5-Methyl-2,4-dioxo-3,4-dihydro-2H-pyrimidin-1-yl)-butyl]-piperazin-1-yl}-nicotinonitril

Analog zu Beispiel 5.2.2 erhielt man durch Umsetzung von 1-(4-Chlorbutyl)-4-hydroxy-5-methylpyrimidin-2(1*H*)-on mit 2-Piperazin-1-yl-nicotinonitril die Titelverbindung.

ESI-MS: [2M+H⁺] = 737,2, [M+H⁺] = 369,1.

### Beispiel 109:

1-{4-[4-(2,4-Dimethoxyphenyl)-piperazin-1-yl]-butyl}-5-methyl-1H-pyrimidin-2,4-dion Analog zu Beispiel 5.2.2 erhielt man durch Umsetzung von 1-(4-Chlorbutyl)-4-hydroxy-5-methylpyrimidin-2(1*H*)-on mit 1-(2,4-Dimethoxyphenyl)-piperazin die Titelverbindung.

ESI-MS: [M+H⁺] = 403,2.

### Beispiel 110:

### 1-{4-[4-(3,4-Difluorphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(3,4-Dffluorphenyl)-piperafin die Titelverbindung.

ESI-MS: [2M+H⁺] = 757,1, [M+H⁺] = 379,0.

### Beispiel 111:

### 5-Methyl-1-[4-(4-(o-tolyl)-piperazin-1-yl)-butyl]-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(o-Tolyl)-piperazin (1-(2-Methylphenyl)-piperazin) die Titelverbindung.

ESI-MS: [M+H⁺] = 357,1.

### Beispiel 112:

### 5-Methyl-1-{4-[4-(6-methyl-pyridin-2-yl)-piperazin-1-yl]-butyl}-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(6-Methyl-pyridin-2-yl)-piperazin die Titelverbindung.

ESI-MS: [2M+H⁺] = 715,2, [M+H⁺] = 358,0, 134,9.

### Beispiel 113:

1-{4-[4-(2,5-Dimethoxyphenyl)-piperazin-1-yl]-butyl}-5-methyl-1H-pyrimidin-2,4-dion Analog zu Beispiel 5.2.2 erhielt man durch Umsetzung von 1-(4-Chlorbutyl)-4-hydroxy-5-methylpyrimidin-2(1*H*)-on mit 1-(2,5-Dimethoxyphenyl)-piperazin die Titelverbindung.

ESI-MS: [M+H⁺] = 417,2, 264,9, 151,2.

### Beispiel 114:

### 5-Methyl-1-{4-[4-(3-methyl-pyridin-2-yl)-piperazin-1-yl]-butyl}-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(3-Methyl-pyridin-2-yl)-piperazin die Titelverbindung.

ESI-MS: [M+H⁺] = 358,1.

### Beispiel 115:

### 5-Methyl-1-{4-[4-(4-methyl-pyridin-2-yl)-piperazin-1-yl]-butyl}-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(4-Methyt-pyridin-2-yl)-piperazin die Titelverbindung.

ESI-MS: [M+H⁺] = 358,0, 134,9.

### Beispiel 116:

### 4-{4-[4-(5-Methyl-2,4-dioxo-3,4-dihydro-2H-pyrimidin-1-yl)-butyl]-piperazin-1-yl}-benzonitril

Analog zu Beispiel 5.2.2 erhielt man durch Umsetzung von 1-(4-Chlorbutyl)-4-hydroxy-5-methylpyrimidin-2(1*H*)-on mit 4-Piperazin-1-yl-benzonitril die Titelverbindung.

ESI-MS: [2M+H⁺] = 735,1 , [M+H⁺] = 368,1.

### Beispiel 117:

### 1-{4-[4-(5-Chlor-2-methoxyphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(5-Chlor-2-methoxyphenyl)-piperazin die Titelverbindung.

ESI-MS: [M+H⁺] = 407,0, 226,8.

### Beispiel 118:

### 1-{4-[4-(4-tert-Butyl-phenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(4-*tert*-Butyl-phenyl)-piperazin die Titelverbindung.

ESI-MS: [M+H⁺] = 399,3, 218,9.

### Beispiel 119:

### 1-{4-[4-(3,5-Dichlorphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(3,5-Dichlorphenyl)-piperazin die Titelverbindung.

ESI-MS: [M+H⁺] = 411,2, 230,8.

### Beispiel 120:

### 2-{4-[4-(5-Methyl-2,4-dioxo-3,4-dihydro-2H-pyrimidin-1-yl)-butyl]-piperazin-1-yl}-benzonitril

Analog zu Beispiel 5.2.2 erhielt man durch Umsetzung von 1-(4-Chlorbutyl)-4-hydroxy-5-methylpyrimidin-2(1*H*)-on mit 2-(Piperazin-1-yl)-benzonitril die Titelverbindung.

ESI-MS: [M+H⁺] = 368,1, 187,9.

### Beispiel 121:

### 1-{4-[4-(4-Chlor-3-trifluormethyl-phenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(4-Chlor-3-trifluormethyl-phenyl)-piperazin die Titelverbindung.

ESI-MS: [M+H⁺] = 445,0, 403,0, 292,8, 264,8.

### Beispiel 122:

### 1-{4-[4-(2,6-Dimethylphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(2,6-Dimethylphenyl)-piperazin die Titelverbindung.

ESI-MS: [M+H⁺] = 371,0, 190,9, 147,9.

### Beispiel 123:

### 1-{4-[4-(2,4-Dichlorphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(2,4-Dichlorphenyl)-piperazin die Titelverbindung.

ESI-MS: [M+H⁺] = 411,0, 230,8, 187,7.

### Beispiel 124:

### 5-Methyl-1-{4-[4-(3-trifluormethyl-pyridin-2-yl)-piperazin-1-yl]-butyl}-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(3-Trifluormethyl-pyridin-2-yl)-piperazin die Titelverbindung.

ESI-MS: [M+H⁺] = 412,2, 259,9, 231,8, 188,8.

### Beispiel 125:

### 1-{4-[4-(3,5-Bis-trifluormethyl-phenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(3,5-Bis-trifluormethyl-phenyl)-piperazin die Titelverbindung.

ESI-MS: [M+H⁺] = 479,0, 340,2, 298,8.

### Beispiel 126:

### 5-Methyl-1-[4-(4-(p-tolyl)-piperazin-1-yl)-butyl]-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(p-Tolyl)-piperazin (1-(4-Methylphenyl)-piperazin) die Titelverbindung.

ESI-MS: [M+H⁺] = 357,0, 204,9, 176,9.

### Beispiel 127:

### 1-{4-[4-(2-Chlorphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(2-Chlorphenyl)-piperazin die Titelverbindung.

ESI-MS: [M+H⁺] = 376,9, 196,8.

### Beispiel 128:

### 1-{4-[4-(2,3-Dimethyl-phenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(2,3-Dimethyl-phenyl)-piperazin die Titelverbindung.

ESI-MS: [M+H⁺] = 371,0, 190,9.

### Beispiel 129:

### 5-Methyl-1-{4-[4-(3-trifluormethyl-phenyl)-piperazin-1-yl]-butyl}-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(3-Trifluormethyl-phenyl)-piperazin die Titelverbindung.

ESI-MS: [M+H⁺] = 411,1, 271,9, 230,8.

### Beispiel 130:

### 1-{4-[4-(4-Chlorphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(4-Chlorphenyl)-piperazin die Titelverbindung.

ESI-MS: [M+H⁺] = 376,9, 196,8, 153,8.

### Beispiel 131:

### 1-{4-[4-(2,4-Difluorphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(2,4-Difluorphenyl)-piperazin die Titelverbindung.

ESI-MS: [M+H⁺] = 379,0, 198,9.

### Beispiel 132:

### 1-{4-[4-(3,5-Dimethyl-phenyl)-piperazin-1-yl]-butyl}-5-methyl-1H-pyrimidin-2,4-dion Analog zu Beispiel 5.2.2 erhielt man durch Umsetzung von 1-(4-Chlorbutyl)-4-hydroxy-5-methylpyrimidin-2(1H)-on mit 1-(3,5-Dimethyl-phenyl)-piperazin die Titelverbindung.

ESI-MS: [2M+H⁺] = 741,2, [M+H⁺] = 371,0, 190,9, 147,9.

### Beispiel 133:

### 1-{4-[4-(4-Chlor 2-fluorphenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(4-Chlor-2-fluorphenyl)-piperazin die Titelverbindung.

ESI-MS: [M+H⁺] = 395,0, 214,8.

### Beispiel 134:

1-{4-[4-(3,5-Dimethoxyphenyl)-piperazin-1-yl]-butyl}-5-methyl-1H-pyrimidin-2,4-dion Analog zu Beispiel 5.2.2 erhielt man durch Umsetzung von 1-(4-Chlorbutyl)-4-hydroxy-5-methylpyrimidin-2(1*H*)-on mit 1-(3,5-Dimethoxyphenyl)-piperazin die Titelverbindung.

ESI-MS: [M+H⁺] = 403,2, 222,9.

### Beispiel 135:

### 1-{4-[4-(2,3-Dichlor-phenyl)-piperazin-1-yl]-butyl}-5-methyl-1H-pyrimidin-2,4-dion

Analog zu Beispiel 5.2.2 erhielt man durch Umsetzung von 1-(4-Chlorbutyl)-4-hydroxy-5-methylpyrimidin-2(1*H*)-on mit 1-(2,3-Dichlorphenyl)-piperazin die Titelverbindung.

ESI-MS: [M+H⁺] = 410,9, 230,8.

### Beispiele für galenische Applikationsformen

### A) Tabletten

Auf einer Tablettenpresse werden in üblicher Weise Tabletten folgender Zusammensetzung gepresst:

| | |
|---|---|
| 40 mg | Substanz des Beispiels 2 |
| 120 mg | Maisstärke |
| 13,5 mg | Gelatine |
| 45 mg | Milchzucker |
| 2,25 mg | Aerosil^{®} (chemisch reine Kieselsäure in submikroskopisch feiner |
| | Verteilung) |
| 6,75 mg | Kartoffelstärke (als 6%iger Kleister) |

### B) Dragees

| | |
|---|---|
| 20 mg | Substanz des Beispiels 2 |
| 60 mg | Kernmasse |
| 70 mg | Verzuckerungsmasse |

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Biologische Untersuchungen - Rezeptorbindungsstudien:

Die zu testende Substanz wurde entweder in Methanol/Chremophor^{®} (BASF-AG) oder in Dimethylsulfoxid gelost und anschließend mit Wasser auf die gewünschte Konzentration verdünnt.

### Dopamin-D₃-Rezeptor

Der Ansatz (0,250 ml) setzte sich zusammen aus Membranen von ∼10⁶ HEK-293 Zellen mit stabil exprimierten humanen Dopamin-D₃-Rezeptoren, 0,1 nM [¹²⁵I]-Jodosulprid und Inkubationspuffer (totale Bindung) oder zusätzlich Testsubstanz (Hemmkurve) oder 1µM Spiperon (unspezifische Bindung). Dreifach-Ansätze wurden durchgeführt.

Der Inkubationspuffer enthielt 50 mM Tris, 120 mM NaCl, 5 mM KCI, 2 mM CaCl₂, 2 mM MgCl₂ und 0,1% Rinderserumalbumin, 10 µM Quinolone, 0.1 % Ascorbinsäure (täglich frisch hergestellt). Der Puffer wurde mit HCl auf pH 7.4 eingestellt.

### Dopamin-D_{2L}-Rezeptor

Der Ansatz (1 ml) setzte sich zusammen aus Membranen von ~10⁶ HEK-293 Zellen mit stabil exprimierten humanen Dopamin-D_{2L} Rezeptoren (lange Isoform) sowie 0,01 nM [¹²⁵I]-Jodospiperon und Inkubationspuffer (totale Bindung) oder zusätzlich Testsubstanz (Hemmkurve) oder 1µM Haloperidol (unspezifische Bindung). Dreifach-Ansätze wurden durchgeführt.

Der Inkubationspuffer enthielt 50 mM Tris, 120 mM NaCl, 5 mM KCI, 2 mM CaCl₂, 2 mM MgCl₂ und 0,1% Rinderserumalbumin. Der Puffer wurde mit HCl auf pH 7.4 eingestellt.

### Messung und Auswertung:

Nach Inkubation für 60 Minuten bei 25 °C wurden die Ansätze mit einem Zellsammelgerät über Whatman GF/B Glasfaserfilter unter Vakuum filtriert. Die Filter wurden mit einem Filter-Transfer-System in Szintillationsgläser überführt. Nach Zugabe von 4 ml Ultima Gold^{®} (Packard) wurden die Proben eine Stunde geschüttelt und anschließend die Radioaktivität im Beta-Counter (Packard, Tricarb 2000 oder 2200CA) gezählt. Die cp-Werte wurden anhand einer Standard-Quenchreihe mit Hilfe des geräteeigenen Programms in dpm umgerechnet.

Die Auswertung der Hemmkurven erfolgte durch iterative nichtlineare Regressionsanalyse mit dem Statistical Analysis System (SAS), ähnlich dem von Munson und Rodbard beschreibenen Programm "LIGAND".

Die erfindungsgemäßen Verbindungen zeigen in diesen Tests sehr gute Affinitäten am D₃-Rezeptor (< 100 nM häufig < 50 nM) und binden selektiv an den D₃-Rezeptor. Die Ergebnisse der Bindungstests sind in Tabelle 2 angegeben.

**Tabelle 2:**

| Beispiel | Kᵢ (D₃) [nM] | Selektivität D₃ vs. D₂L^{*} |
|---|---|---|
| 2 | 3,23 | 88 |
| 5 | 1,30 | 246 |
| 6 | 31,2 | 35 |
| 8 | 4,4 | 71 |
| 9 | 0,74 | 53 |
| 10 | 1,75 | 97 |
| 11 | 3,14 | 65 |
| 12 | 3,44 | 63 |
| 13 | 1,88 | 104 |
| 14 | 2,62 | 82 |
| 15 | 14,5 | 64 |
| 16 | 2,99 | 201 |
| 17 | 0,71 | 134 |
| 18 | 4,78 | 99 |
| 19 | 1,44 | 96 |
| 20 | 1,87 | 108 |
| 21 | 1,94 | 96 |
| 22 | 1,16 | 77 |
| 23 | 1,51 | 63 |
| 24 | 1,38 | 122 |
| 25 | 10,40 | 34 |
| 26 | 8,10 | 55 |
| 27 | 11,8 | 52 |
| 29 | 7,64 | 67 |
| 33 | 0,70 | 42 |
| 35 | 4,96 | 116 |
| 36 | 3, 82 | 209 |
| 37 | 3,24 | 126 |
| 38 | 5, 33 | 103 |
| 39 | 6,09 | 66 |
| 40 | 2,12 | 67 |
| 41 | 5,78 | 62 |
| 44 | 0,63 | 158 |
| 45 | 4,99 | 37 |
| 46 | 5,41 | 43 |
| 47 | 4,87 | 51 |
| 49 | 0,54 | 86 |
| 50 | 0,72 | 77 |
| 52 | 2,28 | 97 |
| 53 | 2,32 | 56 |
| 54 | 9,25 | 52 |
| 55 | 5,44 | 119 |
| 56 | 1,02 | 134 |
| 57 | 0,96 | 111 |
| 59 | 1,80 | 44 |
| 60 | 4,33 | 91 |
| 61 | 5,84 | 28 |
| 62 | 11,0 | 49 |
| 63 | 2,97 | 65 |
| 64 | 2, 57 | 57 |
| 65 | 3,31 | 62 |
| 66 | 13,5 | 37 |
| 67 | 10,1 | 39 |
| 69 | 1,80 | 54 |
| 70 | 10,0 | 30 |
| 71 | 7,44 | 37 |
| 72 | 1,49 | 54 |
| 73 | 9,30 | 31 |
| 74 | 1,52 | 53 |
| 75 | 2,30 | 80 |
| 76 | 3,03 | 103 |
| 77 | 0,74 | 150 |
| 78 | 0,32 | 48 |
| 79 | 0,86 | 30 |
| 81 | 2,56 | 94 |
| 82 | 0,31 | 54 |
| 83 | 1,66 | 89 |
| 84 | 0,75 | 63 |
| 85 | 7,36 | 35 |
| 86 | 2,44 | 43 |
| 87 | 0,97 | 31 |
| 92 | 4,56 | 31 |
| 93 | 0,65 | 40 |
| 94 | 4,28 | 36 |
| 95 | 6,53 | 45 |
| 97 | 4,26 | 58 |
| 98 | 1,62 | 59 |
| 99 | 4,82 | 57 |
| 100 | 1,38 | 51 |
| 111 | 6,80 | 30 |
| 119 | 0,46 | 65 |
| 125 | 6,8 | 63 |
| 127 | 1,5 | 45 |
| 128 | 2,4 | 55 |
| 129 | 1,3 | 48 |
| 131 | 25,7 | 30 |
| 132 | 5,5 | 34 |
| 134 | 13,1 | 55 |
| 135 | 0,27 | 87 |

| | | |
|---|---|---|
| * Kᵢ(D₃)/Kᵢ(D_{2L}) | | |

### Hemmung der mitochondrialen Atmung:

Die Hemmung der mitochondrialen Atmung durch die erfindungsgemäßen Verbindungen wurde über die Bestimmung der Sauerstoffverbrauchs von Mitochondrien bei der Produktion von ATP in an sich bekannter Weise ermittelt (siehe Donelly et al. Arch. Toxicol. 1994, 68. S.110, Wong et al. Toxicol. Lett. 2000, 116(3), S. 171-81, Devi et al. Life Sci. 1997, 60(11), S. 849-55).

Die Untersuchung erfolgte mittels eines Strathkelvin-Messgerätes zur Bestimmung von gelöstem O₂ unter Verwendung der zugehörigen Aufnahme und Auswertungssoftware.

Hierzu wurden zunächst die O₂ Elektroden des Messgeräts in Hudson's Puffer (140 mM KCl/ 5mM KH₂PO₄/ 20 mM MOPS/ pH=7.2) und danach mit wässriger Natriumsulfit-Lösung (20 g/l) equilibriert. Nach Equilibrierung und vor jeder Messung wurden die Elektroden mit Wasser (bidest) gespült.

In einem Reaktionsgefäß mit 6 separaten Kammern, jeweils ausgerüstet mit Magnetrührstäbchen und thermostatisiert auf 37°C, gab man 1,4 ml Hudson's Puffer und anschließend 7,5 µl einer Lösung des Wirkstoffs in Dimethylsulfoxid (üblicherweise in einem Konzentrationsbereich von 0,8 bis 40 mM) in die Kammern 2 bis 6 des Untersuchungsgefäß und in Kammer 1 7,5 µl DMSO ohne Wirkstoff. In jede Kammer gab man eine Suspension von frisch isolierten funktionalen Mitochondrien in einer Menge von 1,5 mg Gesamtprotein (pro Kammer). Anschließend führte man die Elektrode in die jeweilige Kammer ein, ließ die Elektroden 30-60 s equilibrieren, gab dann 25 µL Bernsteinsäure-Lösung (300 mM) zu und equilibrierte 60-120 s. Hierzu gab man 2 µL Adenosin-Diphosphat-Lösung (200 mM), equiliebrierte einige Minuten und gab dann 1 µL 2,4-Dinitrophenol-Lösung (300 mM) zu und wartete wenigstens eine weitere Minute. Anschließend wurde die Sauerstoffkonzentration aufgezeichnet. Hieraus ermittelte man die zur Hemmung der Sauerstoffaufnahme erforderliche Konzentration (IC₅₀-Wert).

Die erfindungsgemäßen Verbindungen hemmen die mitochondriale Atmung erst bei hoher Konzentration von in der Regel oberhalb 50 µM, insbesondere > 100 µM (lC₅₀-Werte) und speziell > 200 µM.

### Untersuchung der unselektiven Proteinbindung

Es werden 2 Konzentrationen von der zu testenden Substanz in Plasma untersucht. Für die Konzentration von 20000 ng/ml werden 100 µl einer Stammlösung (1mg/ml) in 4,9 ml Plasma gespikt, für die Konzentration von 5000 ng/ml 25 µl der Stammlösung in 4,975 ml Plasma. Von jeder Konzentration werden für eine Dreifachbestimmung jeweils 1 ml des gespikten Plasmas in Ultrazentrifugationsröhrchen eingewogen und 18 h bei 80000 x g und 15°C abzentrifugiert. Von jeden Röhrchen wird direkt von der Oberfläche 5 Proben ä 100 µl des jeweiligen Überstandes abgenommen, in Eppendorf-Röhrchen pipettiert, mit 100 µl Acetonitril/ Wassergemisch (1:1) versetzt und bis zur Analytik bei -20°C eingefroren. Die verbliebenen 500 µl Plasma mit dem Pellett werden mit 500 µl Acetonitril/ Wassergemisch (1:1) aufgenommen und bis zur Analytik eingefroren.

Die Analytik erfolgt mittels LC/MS/MS. Für die Auswertung werden die Konzentrationen der ersten 2 x 100 µl des Plasmaüberstandes in Relation zu der wiedergefundenen Gesamtkonzentration gesetzt.

Die erfindungsgemäßen Verbindungen zeichnen sich durch eine vergleichsweise geringe Proteinbindung aus. Sie weisen daher eine höhere freie Konzentration im Plasma auf und sollten aufgrund eines gleichmäßigeren Plasmaspiegels (geringere Freisetzung aus der Plasmaproteinbindung, z. B. durch körperliche Aktivität oder Arzneimittelwechselwirkung) eine bessere Verträglichkeit zeigen.

## Patentansprüche

1. Pyrimidin-2-onverbindungen der allgemeinen Formel I 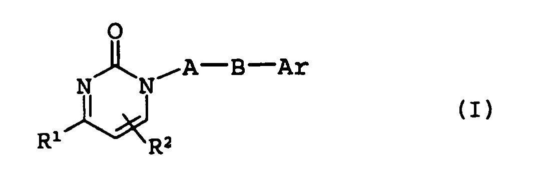 worin
A für (CH₂)ₙ, worin n für 4, 5 oder 6 steht, oder für *trans*-CH₂-CH=CH-CH₂-, trans-CH₂-C(CH₃)=CH-CH_{T}, -CH₂-CH(CH₃)-CH₂-CH₂- oder -CH₂-CH₂-CH₂-CH(CH₃)- steht;
B für einen Rest der Formel: 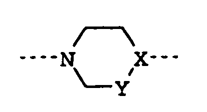 steht, worin X CH oder N bedeutet und Y für CH₂ oder CH₂CH₂ steht oder X-Y auch gemeinsam für C=CH, C=CH-CH₂ oder CH-CH=CH stehen können,
R¹ ausgewählt ist unter OR^{3a}, NR⁴R⁵, SR⁶, C₃-C₆-Cycloalkyl, C₁-C₄-Alkyl, das gegebenenfalls durch OH, C₁-C₄-Alkoxy, Halogen oder Phenyl substituiert ist,
5- oder 6-gliedrigem aromatischem Heterocyclyl mit 1, 2 oder 3 Heteroatomen, ausgewählt unter O, S und N, das durch ein oder zwei Reste substituiert sein kann, die unabhängig voneinander ausgewählt sind unter C₁-C₄-Alkyl, C₁-C₄-Alkoxy, NR⁴R⁵, CN, OH, C₁-C₂-Fluoralkyl oder Halogen, und
Phenyl, das durch ein oder zwei Reste substituiert sein kann, die unabhängig voneinander ausgewählt sind unter C₁-C₄-Alkyl, C₁-C₄-Alkoxy, NR⁴R⁵, OH, CN, C₁-C₂-Fluoralkyl oder Halogen,
R² ausgewählt ist unter H, C₁-C₄-Alkyl, C₁-C₂-Fluoralkyl, Halogen und Cyano
Ar für einen aromatischen Rest steht, der ausgewählt ist unter Phenyl, Pyridyl, Pyrimidinyl und Triazinyl, wobei der aromatische Rest 1, 2 oder 3 Substituenten aufweisen kann, die unabhängig voneinander ausgewählt sind unter C₁-C₆-Alkyl, das gegebenenfalls durch OH, C₁-C₄-Alkoxy, Halogen oder Phenyl substituiert ist, C₂-C₆-Alkenyl, C₂-C₆-Alkinyl, C₃-C₆-Cycloalkyl, C₅-C₁₀-Bicycloalkyl, C₆-C₁₀-Tricycloalkyl, wobei die drei letztgenannten Gruppen gegebenenfalls durch Halogen oder C₁-C₄-Alkyl substituiert sein können, Halogen, CN, OR^{3c}, NR⁴R⁵, NO₂, SR⁶, SO₂R⁶, SO₂NR⁴R⁵, COOR⁷, COR⁸, 5- oder 6-gliedriges Heterocyclyl mit 1, 2 oder 3 Heteroatomen, ausgewählt unter O, S und N, und Phenyl, wobei Phenyl und Heterocyclyl gegebenenfalls ein oder zwei Substituenten tragen, die unabhängig voneinander ausgewählt sind unter C₁-C₄-Alkyl, C₁-C₄-Alkoxy, NR⁴R⁵, CN, C₁-C₂-Fluoralkyl und Halogen, und wobei 2 an benachbarte C-Atome des aromatischen Rests gebundene Substituenten gemeinsam für C₃-oder C₄-Alkylen stehen können oder gemeinsam mit den C-Atomen, an die sie gebunden sind, für einen annellierten, ungesättigten 5 oder 6-gliedrigen Carbocyclus oder für einen 5- oder 6-gliedrigen Heterocyclus mit 1 oder 2 Stickstoffatomen als Ringglieder stehen können,
R³, R^{3a}, R^{3b}, R^{3c}, R^{3d}, R⁴, R⁵, R⁶, R⁷ und R⁸ unabhängig voneinander für H, C₁-C₆-Alkyl, das gegebenenfalls durch OH, C₁-C₄-Alkoxy oder Phenyl substituiert ist, C₁-C₆-Halogenalkyl oder Phenyl, stehen, wobei R⁵ auch eine Gruppe COR⁹ bedeuten kann, wobei R⁹ für Wasserstoff, C₁-C₄-Alkyl oder Phenyl, das gegebenenfalls durch ein oder zwei Reste substituiert ist, die unabhängig voneinander ausgewählt sind unter C₁-C₄-Alkyl, C₁-C₄-Alkoxy, NR⁴R⁵, CN, C₁-C₂-Fluoralkyl oder Halogen, stehen, wobei
R⁴ mit R⁵ auch gemeinsam mit dem Stickstoffatom, an das sie gebunden sind, einen 4-, 5- oder 6-gliedrigen gesättigten oder ungesättigten Heterocyclus bilden können, der gegebenenfalls ein weiteres Heteroatom, ausgewählt unter O, S und NR¹⁰ als Ringglied aufweisen kann, wobei R¹⁰ für Wasserstoff oder C₁-C₄-Alkyl steht, und wobei der Heterocyclus gegebenenfalls 1, 2, 3 oder 4 C₁-C₄-Alkylgruppen tragen kann,
sowie die Derivate und Tautomere der Formeln Ia oder Ib 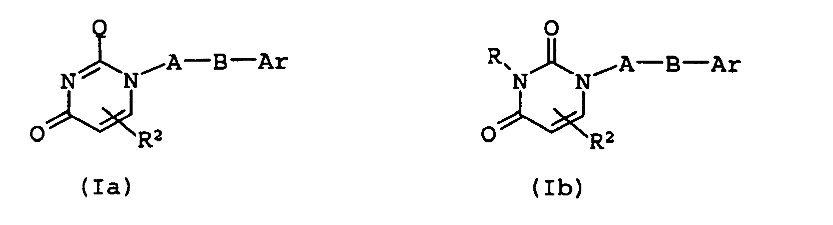 worin R für Wasserstoff oder C₁-C₄-Alkyl steht und Q Halogen oder eine Gruppe OR^{3d} bedeutet, und A, B, Ar und R² die zuvor genannten Bedeutungen aufweisen,
sowie die physiologisch akzeptablen Salze dieser Verbindungen.

2. Pyrimidinonverbindungen nach Anspruch 1, worin Ar ausgewählt ist unter einem Rest der allgemeinen Formel: 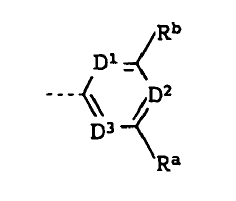 worin wenigstens eine der Variablen D¹ bis D³ für N und die übrigen Variablen D¹ bis D³ für CH stehen und R^{a} und R^{b} unabhängig voneinander die folgenden Bedeutungen aufweisen: OR^{3c}, NR⁴R⁵, CN, C₁-C₆-Alkyl, das gegebenenfalls durch OH, C₁-C₄-Alkoxy, Halogen oder Phenyl substituiert ist, C₂-C₆-Alkenyl, C₂-C₆-Alkinyl, C₃-C₆-Cycloalkyl, C₅-C₁₀-Bicycloalkyl, C₆-C₁₀-Tricycloalkyl, wobei die drei letztgenannten Gruppen gegebenenfalls durch Halogen oder C₁-C₄-Alkyl substituiert sein können, Halogen, CN, C₁-C₄-Alkoxy, 5-oder 6-gliedriges Heterocyclyl mit 1, 2 oder 3 Heteroatomen, ausgewählt unter O, S und N, und Phenyl, wobei Phenyl und Heterocyclyl gegebenenfalls ein oder zwei Substituenten tragen, die unabhängig voneinander ausgewählt sind unter C₁-C₄-Alkyl, C₁-C₄-Alkoxy, NR⁴R⁵, CN, C₁-C₂-Fluoralkyl oder Halogen.

3. Pyrimidinonverbindungen nach Anspruch 2, worin R^{a} für C₁-C₆-Alkyl steht und R^{b} ausgewählt ist unter C₁-C₆-Alkyl, C₃-C₆-Cycloalkyl und C₁-C₂-Fluoralkyl.

4. Pyrimidinonverbindungen nach einem der vorhergehenden Ansprüche, worin R¹ für OR^{3a}, insbesondere für OH steht.

5. Pyrimidinonverbindungen nach einem der vorhergehenden Ansprüche, worin A eine Gruppe der Formel -(CH₂)ₙ- bedeutet, worin n für 4 steht.

6. Pyrimidinonverbindungen nach einem der vorhergehenden Ansprüche, worin B in Formel I ausgewählt ist unter bivalenten Resten der allgemeinen Formeln:

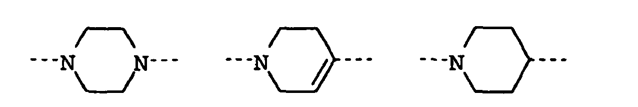

7. Pyrimidinonverbindungen nach Anspruch 1 der allgemeinen Formel I.1 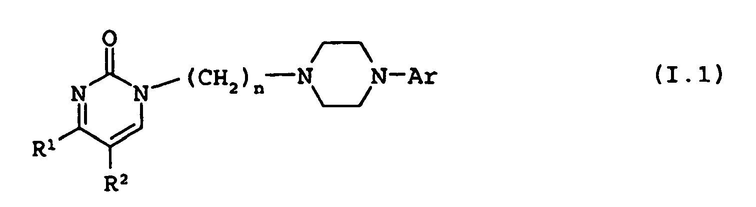 worin Ar, R¹ und R² die in Anspruch 1 genannten Bedeutungen aufweisen,
n für 4, 5 oder 6 steht,
sowie die Derivate und Tautomere der Formeln Ia.1 oder Ib.1 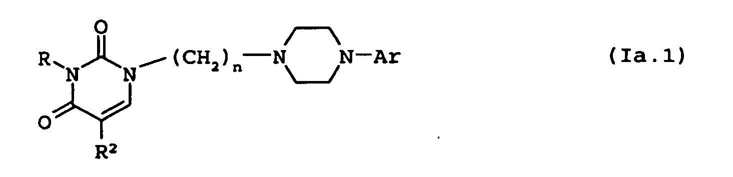 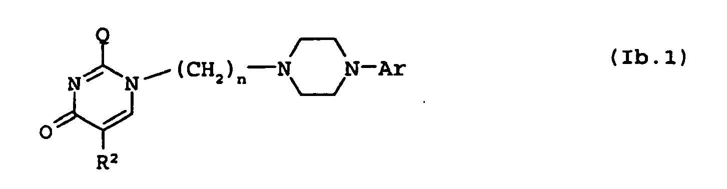 worin R für Wasserstoff oder C₁-C₄-Alkyl steht und Q Halogen oder eine Gruppe OR^{3d} bedeutet, und n, Ar, R² und R^{3d} in Anspruch 1 die für Formel I genannten Bedeutungen aufweisen, sowie die physiologisch verträglichen Säureadditionssalze dieser Verbindungen.

8. Pyrimidinonverbindungen nach Anspruch 7, worin Ar ausgewählt ist unter einem Rest der allgemeinen Formel: 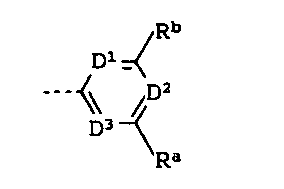 worin wenigstens einer der Variablen D¹ bis D³ für N und die übrigen Variablen D¹ bis D³ für CH stehen,
R^{b} für C₁-C₆-Alkyl steht und R^{a} ausgewählt ist unter C₁-C₆-Alkyl, C₃-C₆-Cycloalkyl und C₁-C₂-Fluoralkyl.

9. Pyrimidinonverbindungen nach Anspruch 8, worin D¹ und D² für N und D³ für CH stehen.

10. Pyrimidinonverbindungen nach einem Ansprüche 7 bis 9, worin R¹ ausgewählt ist unter C₁-C₄-Alkyl, C₃-C₆-Cycloalkyl, OR^{3a}, worin R^{3a} die zuvor genannten Bedeutungen aufweist, und
Phenyl, das durch ein oder zwei Reste substituiert sein kann, die unabhängig voneinander ausgewählt sind unter C₁-C₄-Alkyl, C₁-C₄-Alkoxy, OH, CN, C₁-C₂-Fluoralkyl oder Halogen.

11. Pyrimidinonverbindungen nach Anspruch 10, worin R¹ für OR^{3a} und insbesondere für OH steht.

12. Pyrimidinonverbindungen nach Anspruch 10, worin R¹ für C₁-C₄-Alkyl und insbesondere für Methyl steht.

13. Pyrimidinonverbindungen nach einem der Ansprüche 7 bis 12, worin n für 4 steht.

14. Pharmazeutisches Mittel, enthaltend wenigstens eine Verbindung nach einem der Ansprüche 1 bis 13 oder 18 und/oder deren Säureadditionssalz, gegebenenfalls zusammen mit physiologisch akzeptablen Trägern und/oder Hilfsstoffen.

15. Verwendung von Verbindungen nach einem der Ansprüche 1 bis 13 oder 18 und von deren pharmakologisch akzeptablen Säureadditionssalzen zur Herstellung eines pharmazeutischen Mittels zur Behandlung von Erkrankungen, die auf die Beeinflussung durch Dopamin-D₃-Rezeptorliganden ansprechen.

16. Verwendung von Verbindungen nach einem der Ansprüche 1 bis 13 oder 18 und von deren pharmakologisch akzeptablen Säureadditionssalzen zur Herstellung eines pharmazeutischen Mittels zur Behandlung von Erkrankungen des zentralen Nervensystems.

17. Verwendung nach Anspruch 16 zur Behandlung von Schizophrenie und/oder Depression.

18. Pyrimidinonverbindungen nach Anspruch 1 der allgemeinen Formel I.1a 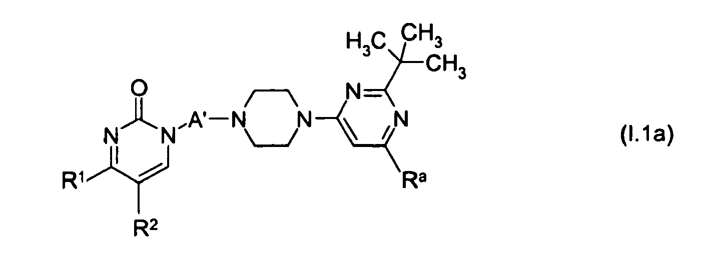 worin
R¹ für OH, R² für H, R^{a} für CHF₂ und A' für (CH₂)₄ stehen, oder
R¹ für OH, R² für H, R^{a} für CF₃ und A' für (CH₂)₄ stehen, oder
R¹ für OH, R² für CH₃, R^{a} für CF₃ und A' für (CH₂)₄ stehen, oder
R¹ für OH, R² für F, R^{a} für CF₃ und A' für (CH₂)₄ stehen, oder
R¹ für OH, R² für H, R^{a} für CH₂CH₂CH₃ und A' für (CH₂)₄ stehen, oder
R¹ für OH, R² für CH₃, R^{a} für CH₂CH₂CH₃ und A' für (CH₂)₄ stehen.

## Claims

1. Pyrimidin-2-one compounds of the general formula I 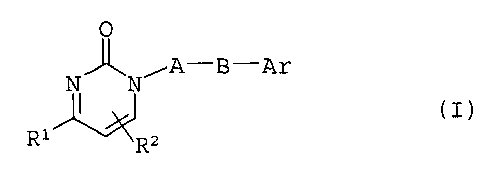 in which
A is (CH₂)ₙ, in which n is 4, 5 or 6, or is *trans-*CH₂-CH=CH-CH₂-, *trans* -CH₂-C(CH₃)=CH-CH₂-, -CH₂-CH(CH₃)-CH₂-CH₂- or -CH₂-CH₂-CH₂-CH(CH₃)-;
B is a radical of the formula: 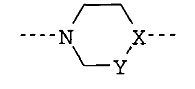 in which X is CH or N, and Y is CH₂ or CH₂CH₂, or X-Y together may also be C=CH, C=CH-CH₂ or CH-CH=CH,
R¹ is selected from OR^{3a}, NR⁴R⁵, SR⁶, C₃-C₆-cycloalkyl, C₁-C₄-alkyl which is optionally substituted by OH, C₁-C₄-alkoxy, halogen or phenyl,
5- or 6-membered aromatic heterocyclyl having 1, 2 or 3 heteroatoms selected from O, S and N, which may be substituted by one or two radicals which are selected independently of one another from C₁-C₄-alkyl, C₁-C₄-alkoxy, NR⁴R⁵, CN, OH, C₁-C₂-fluoroalkyl or halogen, and
phenyl which may be substituted by one or two radicals which are selected independently of one another from C₁-C₄-alkyl, C₁-C₄-alkoxy, NR⁴R⁵, OH, CN, C₁-C₂-fluoroalkyl or halogen,
R₂ is selected from H, C₁-C₄-alkyl, C₁-C₂-fluoroalkyl, halogen and cyano,
Ar is an aromatic radical which is selected from phenyl, pyridyl, pyrimidinyl and triazinyl, where the aromatic radical may have 1, 2 or 3 substituents which are selected independently of one another from C₁-C₆-alkyl which is optionally substituted by OH, C₁-C₄-alkoxy, halogen or phenyl, or C₂-C₆-alkenyl, C₂-C₆-alkynyl, C₃-C₆-cycloalkyl, C₅-C₁₀-bicycloalkyl, C₆-C₁₀-tricycloalkyl, where the last three groups mentioned may optionally be substituted by halogen or C₁-C₄-alkyl, or halogen, CN, OR^{3c}, NR⁴R⁵, NO₂, SR⁶, SO₂R⁶, SO₂NR⁴R⁵, COOR⁷, COR⁸, 5-or 6-membered heterocyclyl having 1, 2 or 3 heteroatoms selected from 0, S and N, and phenyl, where phenyl and heterocyclyl optionally have one or two substituents which are selected independently of one another from C₁-C₄-alkyl, C₁-C₄-alkoxy, NR⁴R⁵, CN, C₁-C₂-fluoroalkyl and halogen, and where 2 substituents bonded to adjacent C atoms of the aromatic radical may together be C₃- or C₄-alkylene, or may together with the C atoms to which they are bonded be a fused-on, unsaturated 5 or 6-membered carbocycle or a 5-or 6-membered heterocycle having 1 or 2 nitrogen atoms as ring members,
R³, R^{3a}, R^{3b}, R^{3c}, R^{3a}, R⁴, R⁵, R⁶, R⁷ and R⁸ are independently of one another H, C₁-C₆-alkyl which is optionally substituted by OH, C₁-C₄-alkoxy or phenyl, or C₁-C₆-haloalkyl or phenyl, where R⁵ may also be a group COR⁹ where R⁹ is hydrogen, C₁-C₄-alkyl or phenyl which is optionally substituted by one or two radicals which are selected independently of one another from C₁-C₄-alkyl, C₁-C₄-alkoxy, NR⁴R⁵, CN, C₁-C₂-fluoroalkyl or halogen, where
R⁴ with R⁵ may also form together with the nitrogen atom to which they are bonded a 4-, 5- or 6-membered saturated or unsaturated heterocycle which may optionally have a further heteroatom selected from O, S and NR¹⁰ as ring member, where R¹⁰ is hydrogen or C₁-C₄-alkyl, and where the heterocycle may optionally carry 1, 2, 3 or 4 C₁-C₄-alkyl groups,
and the derivatives and tautomers of the formulae Ia or Ib 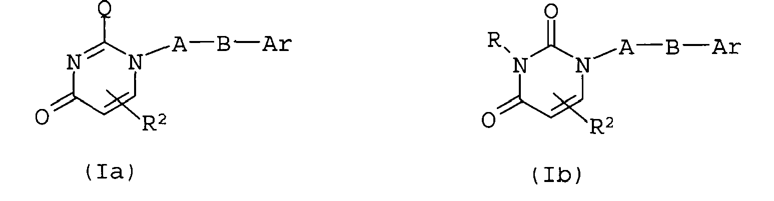 in which R is hydrogen or C₁-C₄-alkyl, and Q is halogen or a group OR^{3d}, and A, B, Ar and R² have the aforementioned meanings,
and the physiologically tolerated salts of these compounds.

2. Pyrimidinone compounds according to Claim 1, in which Ar is selected from a radical of the general formula: 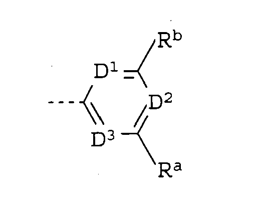 in which at least one of the variables D¹ to D³ is N and the other variables D¹ to D³ are CH, and R^{a} and R^{b} have independently of one another the following meanings: OR^{3c}, NR⁴R⁵, CN, C₁-C₆-alkyl which is optionally substituted by OH, C₁-C₄-alkoxy, halogen or phenyl, or C₂-C₆-alkenyl, C₂-C₆-alkynyl, C₃-C₆-cycloalkyl, C₅-C₁₀-bicycloalkyl, C₆-C₁₀-tricycloalkyl, where the last three groups mentioned may optionally be substituted by halogen or C₁-C₄-alkyl, or halogen, CN, C₁-C₄-alkoxy, 5- or 6-membered heterocyclyl having 1, 2 or 3 heteroatoms selected from O, S and N, and phenyl, where phenyl and heterocyclyl optionally have one or two substituents which are selected independently of one another from C₁-C₄-alkyl, C₁-C₄-alkoxy, NR⁴R⁵, CN, C₁-C₂-fluoroalkyl or halogen.

3. Pyrimidinone compounds according to Claim 2, in which R^{a} is C₁-C₆-alkyl and R^{b} is selected from C₁-C₆-alkyl, C₃-C₆-cycloalkyl and C₁-C₂-fluoroalkyl.

4. Pyrimidinone compounds according to one of the preceding claims, in which R¹ is OR^{3a}, in particular OH.

5. Pyrimidinone compounds according to one of the preceding claims, in which A is a group of the formula -(CH₂)ₙ- in which n is 4.

6. Pyrimidinone compounds according to one of the preceding claims, in which B in formula I is selected from bivalent radicals of the general formulae:

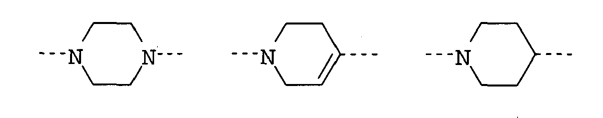

7. Pyrimidinone compounds according to Claim 1 of the general formula I.1 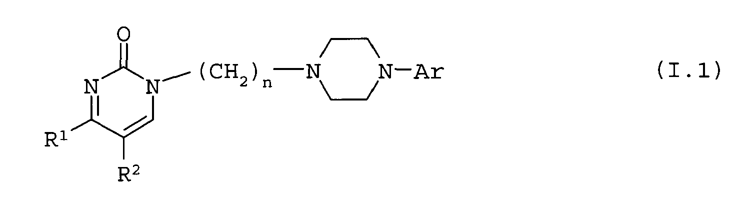 in which Ar, R¹ and R² have the meanings stated in Claim 1,
n is 4, 5 or 6,
and the derivatives and tautomers of the formulae Ia.1 or Ib.1 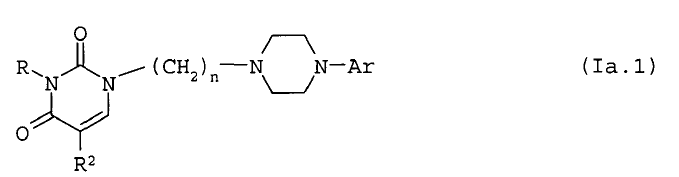 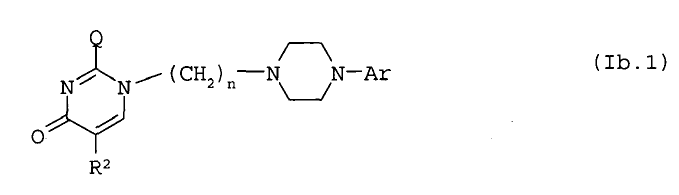 in which R is hydrogen or C₁-C₄-alkyl, and Q is halogen or a group OR^{3d}, and n, Ar, R² and R^{3d} have the meanings mentioned for formula I in Claim 1, and the physiologically tolerated acid addition salts of these compounds.

8. Pyrimidinone compounds according to Claim 7, in which Ar is selected from a radical of the general formula: 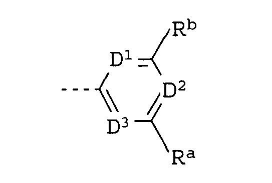 in which at least one of the variables D¹ to D³ is N and the other variables D¹ to D³ are CH,
R^{b} is C₁-C₆-alkyl, and R^{a} is selected from C₁-C₆-alkyl, C₃-C₆-cycloalkyl and C₁-C₂-fluoroalkyl.

9. Pyrimidinone compounds according to Claim 8, in which D¹ and D² are N and D³ is CH.

10. Pyrimidinone compounds according to one of Claims 7 to 9, in which R¹ is selected from C₁-C₄-alkyl, C₃-C₆-cycloalkyl, OR^{3a}, in which R^{3a} has the aforementioned meanings, and
phenyl which may be substituted by one or two radicals which are selected independently of one another from C₁-C₄-alkyl, C₁-C₄-alkoxy, OH, CN, C₁-C₂-fluoroalkyl or halogen.

11. Pyrimidinone compounds according to Claim 10, in which R¹ is OR^{3a} and in particular OH.

12. Pyrimidinone compounds according to Claim 10, in which R¹ is C₁-C₄-alkyl and in particular methyl.

13. Pyrimidinone compounds according to one of Claims 7 to 12, in which n is 4.

14. Pharmaceutical compositions comprising at least one compound according to one of Claims 1 to 13 or 18 and/or the acid addition salt thereof, if appropriate together with physiologically acceptable carriers and/or excipients.

15. Use of compounds according to one of Claims 1 to 13 or 18 and of the pharmacologically acceptable acid addition salts thereof for producing a pharmaceutical composition for the treatment of disorders which respond to influencing by dopamine D₃ receptor ligands.

16. Use of compounds according to one of Claims 1 to 13 or 18 and of the pharmacologically acceptable acid addition salts thereof for producing a pharmaceutical composition for the treatment of disorders of the central nervous system.

17. Use according to Claim 16 for the treatment of schizophrenia and/or depression.

18. Pyrimidinone compounds according to Claim 1 of the general formula I.1a, 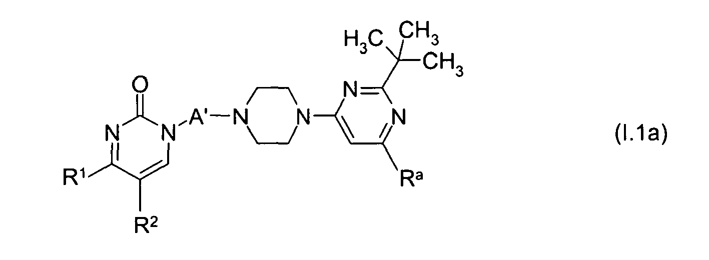 wherein
R¹ is OH, R² is H, R^{a} is CHF₂ and A' is (CH₂)₄, oder .
R¹ is OH, R² is H, R^{a} is CF₃ and A' is (CH₂) ₄, or
R¹ is OH, R² is CH₃, R^{a} is CF₃ and A' is (CH₂)₄, or
R¹ is OH, R² is F, R^{a} is CF₃ and A' is (CH₂)₄ or
R¹ is OH, R² is H, R^{a} is CH₂CH₂CH₃ and A' is (CH₂)₄ or
R¹ is OH, R² is CH₃, R^{a} is CH₂CH₂CH₃ and A' is (CH₂) ₄.

## Revendications

1. Composés de pyrimidin-2-one de formule générale I 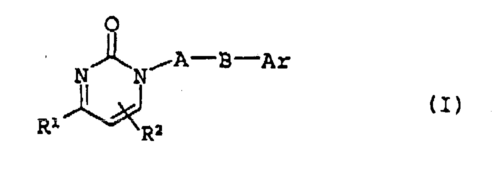 dans laquelle
A représente (CH₂)ₙ, où n est 4, 5 ou 6, ou trans-CH₂-CH=CH-CH₂-, trans-CH₂-C(CH₃)=CH-CH₂-, -CH₂-CH(CH₃)-CH₂-CH₂- ou -CH₂-CH₂-CH₂CH(CH₃)-;
B représente un reste de formule 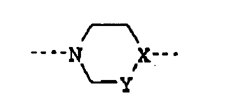 dans laquelle X représente CH ou N et Y représente CH₂ ou CH₂CH₂, ou X-Y peuvent aussi représenter ensemble C=CH, C=CH-CH₂ ou CH-CH=CH,
R¹ est choisi parmi OR^{3a}, NR⁴R⁵, SR⁶, cycloalkyle en C₃-C₆, alkyle en C₁-C₄ éventuellement substitué par OH, alcoxy en C₁-C₄, halogène ou phényle, hétérocyclyle aromatique de 5 ou 6 chaînons ayant 1, 2 ou 3 hétéroatomes choisis parmi O, S et N, qui peut être substitué par un ou deux restes choisis indépendamment l'un de l'autre parmi alkyle en C₁-C₄, alcoxy en C₁-C₄, NR⁴R⁵, CN, OH, fluoroalkyle en C₁-C₂ ou halogène, et
phényle, qui peut être substitué par un ou deux restes choisis indépendamment l'un de l'autre parmi alkyle en C₁-C₄, alcoxy en C₁-C₄, NR⁴R⁵, OH, CN, fluoroalkyle en C₁-C₂ ou halogène,
R² est choisi parmi H, alkyle en C₁-C₄, fluoroalkyle en C₁-C₂, halogène et cyano,
Ar représente un reste aromatique choisi parmi phényle, pyridyle, pyrimidinyle et triazinyle, le reste aromatique pouvant présenter 1, 2 ou 3 substituants choisis indépendamment les uns des autres parmi alkyle en C₁-C₆ éventuellement substitué par OH, alcoxy en C₁-C₄, halogène ou phényle, alcényle en C₂-C₆, alcynyle en C₂-C₆, cycloalkyle en C₃-C₆, bicycloalkyle en C₅-C₁₀, tricycloalkyle en C₆-C₁₀, les trois derniers groupes cités pouvant éventuellement être substitués par halogène ou alkyle en C₁-C₄, halogène, CN, OR^{3c}, NR⁴R⁵, NO₂, SR⁶, SO₂R⁶, SO₂NR⁴R⁵, COOR⁷, COR⁸, hétérocyclyle de 5 ou 6 chaînons ayant 1, 2 ou 3 hétéroatomes choisis parmi O, S et N, et phényle, les restes phényle et hétérocyclyle portant éventuellement un ou deux substituants choisis indépendamment l'un de l'autre parmi alkyle en C₁-C₄, alcoxy en C₁-C₄, NR⁴R⁵, CN, fluoroalkyle en C₁-C₂ et halogène, et 2 substituants liés à des atomes de carbone adjacents du reste aromatique pouvant représenter ensemble un alkylène en C₃ ou C₄ ou représenter ensemble, avec les atomes de carbone auxquels ils sont liés, un carbocycle insaturé condensé de 5 ou 6 chaînons ou un hétérocycle de 5 ou 6 chaînons avec 1 ou 2 atomes d'azote comme chaînons cycliques,
R³, R^{3a}, R^{3b}, R^{3c}, R^{3d}, R⁴, R⁵, R⁶, R⁷ et R⁸ sont indépendamment les uns des autres H, alkyle en C₁-C₆ éventuellement substitué par OH, alcoxy en C₁-C₄ ou phényle, halogénoalkyle en C₁-C₆ ou phényle, R⁵ pouvant aussi représenter un groupe COR⁹ où R⁹ est l'hydrogène, alkyle en C₁-C₄ ou phényle éventuellement substitué par un ou deux restes choisis indépendamment l'un de l'autre parmi alkyle en C₁-C₄, alcoxy en C₁-C₄, NR⁴R⁵, CN, fluoroalkyle en C₁-C₂ ou halogène, et
R⁴ et R⁵ peuvent aussi former ensemble, avec l'atome d'azote auquel ils sont liés, un hétérocycle saturé ou insaturé de 4, 5 ou 6 chaînons qui peut éventuellement contenir un autre hétéroatome choisi parmi O, S et NR¹⁰ comme chaînon cyclique, où R¹⁰ est l'hydrogène ou un alkyle en C₁-C₄, et l'hétérocycle pouvant éventuellement porter 1, 2, 3 ou 4 groupes alkyle en C₁-C₄,
et les dérivés et tautomères de formule la ou Ib 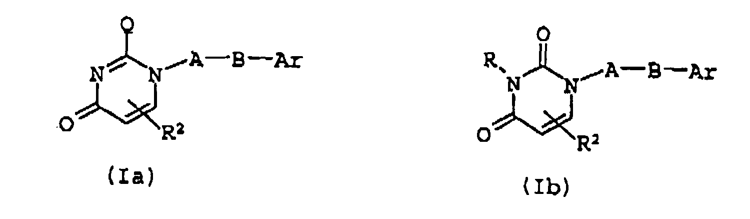 où R est l'hydrogène ou un alkyle en C₁-C₄ et Q est un halogène ou un groupe OR^{3d}, et A, B, Ar et R² ont les significations indiquées précédemment,
ainsi que les sels physiologiquement acceptables de ces composés.

2. Composés de pyrimidinone selon la revendication 1, dans lesquels Ar est choisi parmi les restes de formule générale 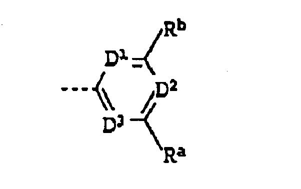 dans laquelle au moins l'une des variables D¹ à D³ représente N et les autres variables D¹ à D³ représentent CH et R^{a} et R^{b} ont indépendamment l'un de l'autre les significations suivantes: OR^{3c}, NR⁴R⁵, CN, alkyle en C₁-C₆ éventuellement substitué par OH, alcoxy en C₁-C₄, halogène ou phényle, alcényle en C₂-C₆, alcynyle en C₂-C₆, cycloalkyle en C₃-C₆, bicycloalkyle en C₅-C₁₀, tricycloalkyle en C₆-C₁₀, les trois derniers groupes cités pouvant éventuellement être substitués par halogène ou alkyle en C₁-C₄, halogène, CN, alcoxy en C₁-C₄, hétérocyclyle de 5 ou 6 chaînons avec 1, 2 ou 3 hétéroatomes choisis parmi O, S et N, et phényle, les restes phényle et hétérocyclyle portant éventuellement un ou deux substituants choisis indépendamment l'un de l'autre parmi alkyle en C₁-C₄, alcoxy en C₁-C₄, NR⁴R⁵, CN, fluoroalkyle en C₁-C₂ ou halogène.

3. Composés de pyrimidinone selon la revendication 2, dans lesquels R^{a} représente un alkyle en C₁-C₆ et R^{b} est choisi parmi alkyle en C₁-C₆, cycloalkyle en C₃-C₆ et fluoroalkyle en C₁-C₂.

4. Composés de pyrimidinone selon l'une des revendications précédentes, dans lesquels R¹ représente OR^{3a}, en particulier OH.

5. Composés de pyrimidinone selon l'une des revendications précédentes, dans lesquels A est un groupe de formule -(CH₂)ₙ- où n est 4.

6. Composés de pyrimidinone selon l'une des revendications précédentes, dans lesquels B, dans la formule I, est choisi parmi les restes bivalents de formule générale

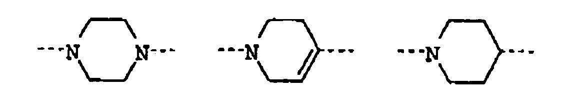

7. Composés de pyrimidinone selon la revendication 1, de formule générale I.1 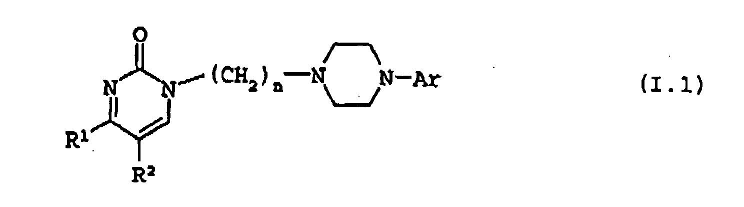 dans laquelle Ar, R¹ et R² ont les significations indiquées dans la revendication 1,
n est 4, 5 ou 6,
et les dérivés et tautomères de formule Ia.1 ou Ib.1 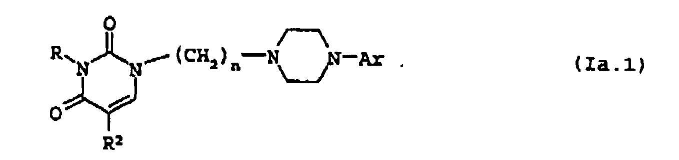 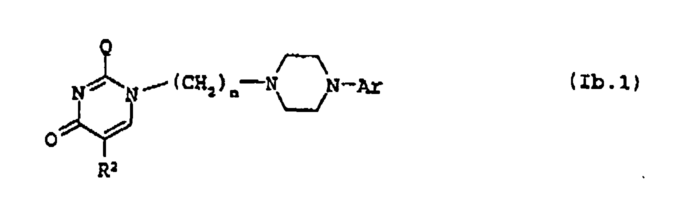 dans lesquels R représente l'hydrogène ou un alkyle en C₁-C₄ et Q représente un halogène ou un groupe OR^{3d}, et n, Ar, R² et R^{3d} ont les significations indiquées dans la revendication 1 pour la formule I, ainsi que les sels d'addition d'acides physiologiquement acceptables de ces composés.

8. Composés de pyrimidinone selon la revendication 7, dans lesquels Ar est choisi parmi les restes de formule générale: 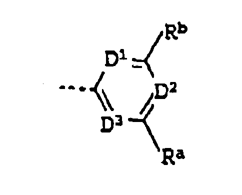 dans laquelle au moins l'une des variables D¹ à D³ représente N et les autres variables D¹ à D³ représentent CH,
R^{b} représente un alkyle en C₁-C₆ et R^{a} est choisi parmi alkyle en C₁-C₆, cycloalkyle en C₃-C₆ et fluoroalkyle en C₁-C₂.

9. Composés de pyrimidinone selon la revendication 8, dans lesquels D¹ et D² représentent N et D³ représente CH.

10. Composés de pyrimidinone selon l'une des revendications 7 à 9, dans lesquels R¹ est choisi parmi alkyle en C₁-C₄, cycloalkyle en C₃-C₆, OR^{3a}, où R^{3a} a les significations indiquées précédemment, et phényle pouvant être substitué par un ou deux restes choisis indépendamment l'un de l'autre parmi alkyle en C₁-C₄, alcoxy en C₁-C₄, OH, CN, fluoroalkyle en C₁-C₂ ou halogène.

11. Composés de pyrimidinone selon la revendication 10, dans lesquels R¹ représente OR^{3a} et en particulier OH.

12. Composés de pyrimidinone selon la revendication 10, dans lesquels R¹ représente un alkyle en C₁-C₄ et en particulier un méthyle.

13. Composés de pyrimidinone selon l'une des revendications 7 à 12, dans lesquels n est 4.

14. Composition pharmaceutique, contenant au moins un composé selon l'une des revendications 1 à 13 ou 18 et/ou un de leurs sels d'addition d'acides, éventuellement avec des supports et/ou produits auxiliaires physiologiquement acceptables.

15. Utilisation de composés selon l'une des revendications 1 à 13 ou 18 et de leurs sels d'addition d'acides pharmacologiquement acceptables pour la préparation d'une composition pharmaceutique destinée au traitement de maladies qui réagissent à l'influence exercée par des ligands de récepteurs D₃ de la dopamine.

16. Utilisation de composés selon l'une des revendications 1 à 13 ou 18 et de leurs sels d'addition d'acides pharmacologiquement acceptables pour la préparation d'une composition pharmaceutique destinée au traitement de maladies du système nerveux central.

17. Utilisation selon la revendication 16 pour le traitement de la schizophrénie et/ou de la dépression.

18. Composés de pyrimidinone selon la revendication 1, de formule générale I.1 a 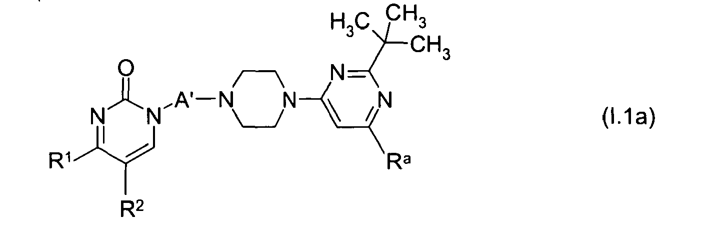 dans laquelle
R¹ est OH, R² est H, R^{a} est CHF₂ et A' est (CH₂)₄, ou
R¹ est OH, R² est H, R^{a} est CF₃ et A' est (CH₂)₄, ou
R¹ est OH, R² est CH₃, R^{a} est CF₃ et A' est (CH₂)₄, ou
R¹ est OH, R² est F, R^{a} est CF₃ et A' est (CH₂)₄ ou
R¹ est OH, R² est H, R^{a} est CH₂CH₂CH₃ et A' est (CH₂)₄ ou
R¹ est OH, R² est CH₃, R^{a} est CH₂CH₂CH₃ et A' est (CH₂)₄.
